# EUROPEAN PATENT SPECIFICATION

(11) **EP 2 764 101 B1**
(45) Date of publication and mention of the grant of the patent: **29.03.2017**
(21) Application number: 12766987.7
(22) Date of filing: 03.10.2012
(51) Int. Cl.: C12N 15/113, C12N 15/82, A01H 5/00

(54) **RNAi FOR THE CONTROL OF FUNGI AND OOMYCETES BY INHIBITING SACCHAROPINE DEHYDROGENASE GENE**
RNAI ZUR BEKÄMPFUNG VON PILZEN UND EIPILZEN DURCH HEMMUNG DES SACCHAROPIN-DEHYDROGENASE-GENS
ARNI POUR LA LUTTE CONTRE DES CHAMPIGNONS ET OOMYCÈTES PAR INHIBITION DU GÈNE DE LA SACCHAROPINE DÉSHYDROGÉNASE

(30) Priority: 04.10.2011 EP 11356013; 18.06.2012 US 201261661062 P
(43) Date of publication of application: 13.08.2014
(73) Proprietor: Bayer Intellectual Property GmbH, 40789 Monheim (DE)
(72) Inventor: DELEBARRE, Thomas, 69300 Caluire-et-Cuire (FR); DORME, Cécile, 69008 Lyon (FR); ESSIGMANN, Bernd, 69370 Saint-Didier-au-Mont-d'Or (FR); SCHMITT, Frédéric, 01600 Saint-Didier-de-Formans (FR); VILLALBA, François, 69250 Albigny-sur-Saône (FR); PAGET, Eric, 69300 Caluire-et-Cuire (FR)
(74) Representative: Guitton, Carole
(86) International application number: PCT/EP2012/069521
(87) International publication number: WO 2013/050410

(56) References cited:
- WO-A2-2006/070227
- WO-A2-2009/112270
- US-A1- 2003 180 829
- Stärkel C.: "Host Induced Gene Silencing - strategies for the improvement of resistance against Cercospora beticola in sugar beet (B. vulgaris L.) and against Fusarium graminearum in wheat (T. aestivum L.) and maize (Z. mays L.)", Ph. D. thesis, June 2011 (2011-06), XP002668931, Hamburg Retrieved from the Internet: URL:http://ediss.sub.uni-hamburg.de/vollte xte/2011/5286/pdf/Dissertation.pdf [retrieved on 2012-02-07]
- HENGYU XU ET AL: "The [alpha]-Aminoadipate Pathway for Lysine Biosynthesis in Fungi", CELL BIOCHEMISTRY AND BIOPHYSICS, vol. 46, no. 1, 1 January 2006 (2006-01-01), pages 43-64, XP055018476, ISSN: 1085-9195, DOI: 10.1385/CBB:46:1:43
- D. NOWARA ET AL: "HIGS: Host-Induced Gene Silencing in the Obligate Biotrophic Fungal Pathogen Blumeria graminis", THE PLANT CELL ONLINE, vol. 22, no. 9, 1 September 2010 (2010-09-01), pages 3130-3141, XP055018497, ISSN: 1040-4651, DOI: 10.1105/tpc.110.077040
- Sonnenberger, K. et al.: "Disruption of the homoaconitase gene of the fungal barley pathogen Pyrenophora teres results in lysin auxotrophy, disturbed conidiation and strongly reduced virulence.", Mitteilungen der DeutschenPhytomedizinischen Gesellschaft e.V., vol. 33, no. 3 September 2003 (2003-09), pages 50-51, XP002668930, Retrieved from the Internet: URL:http://dpg.phytomedizin.org/uploads/me dia/Phytomedizin_2003-3.pdf [retrieved on 2012-02-07]
- F. SCHOBEL ET AL: "Evaluation of Lysine Biosynthesis as an Antifungal Drug Target: Biochemical Characterization of Aspergillus fumigatus Homocitrate Synthase and Virulence Studies", EUKARYOTIC CELL, vol. 9, no. 6, 1 June 2010 (2010-06-01), pages 878-893, XP055018464, ISSN: 1535-9778, DOI: 10.1128/EC.00020-10
- HOUMARD NANCY M ET AL: "High-lysine corn generated by endosperm-specific suppression of lysine catabolism using RNAi", PLANT BIOTECHNOLOGY JOURNAL, BLACKWELL, OXFORD, GB, vol. 5, no. 5, 1 September 2007 (2007-09-01), pages 605-614, XP002611935, ISSN: 1467-7644, DOI: 10.1111/J.1467-7652.2007.00265.X [retrieved on 2007-06-06]
- YIN CHUNTAO ET AL: "Development of a Host-Induced RNAi System in the Wheat Stripe Rust Fungus Puccinia striiformis f. sp tritici", MOLECULAR PLANT - MICROBE INTERACTIONS, AMERICAN PHYTOPATHOLOGICAL SOCIETY, UNITED STATES, vol. 24, no. 5, 1 May 2011 (2011-05-01), pages 554-561, XP008148368, ISSN: 0894-0282, DOI: 10.1094/MPMI-10-10-0229

### SEQUENCE MISTING

<110> Bayer Intellectual Property GmbH
<120> RNAi for the control of fungi and oomycetes by inhibiting saccharopine dehydrogenase gene
<130> BCS 11-4010
<160> 58
<170> PatentIn version 3.5
<210> 1
   <211> 1143
   <212> DNA
   <213> Aspergillus clavatus
<220>
   <221> CDS
   <222> (1)..(1143)
   <223> Lyes1
<400> 1
   atg tcg agc aac aag att tgg ctg cgc gct gag acc aag ccc gct gaa      48
   Met Ser Ser Asn Lys Ile Trp Leu Arg Ala Glu Thr Lys Pro Ala Glu
   1               5                   10                  15
   gcg cgg tct gcc ttg act cct acc aca tgc aag gct ctt atg gat gcc      96
   Ala Arg Ser Ala Leu Thr Pro Thr Thr Cys Lys Ala Leu Met Asp Ala
               20                  25                  30
   ggc tac gac gtt act gtc gag cgt tcc aca caa cgc atc ttt gat ggt      144
   Gly Tyr Asp Val Thr Val Glu Arg Ser Thr Gln Arg Ile Phe Asp Gly
           35                  40                  45
   gag gat aac ata ctc tcc tac ctt atc ggc gct ccc ctg gtt gag gaa      192
   Glu Asp Asn Ile Leu Ser Tyr Leu Ile Gly Ala Pro Leu Val Glu Glu
       50                  55                  60
   ggc tca tgg gtc aag gat gcc ccc aag gac gct tac gtc ctg ggt ctc      240
   Gly Ser Trp Val Lys Asp Ala Pro Lys Asp Ala Tyr Val Leu Gly Leu
   65                  70                  75                  80
   aag gag ctt ccc gaa gat gac ttc ccc ctc gag cac gtc cac atc tct      288
   Lys Glu Leu Pro Glu Asp Asp Phe Pro Leu Glu His Val His Ile Ser
                   85                  90                  95
   ttc gca cac tgc tac aag gaa caa ggt ggc tgg gag aag gtt ctt agc      336
   Phe Ala His Cys Tyr Lys Glu Gln Gly Gly Trp Glu Lys Val Leu Ser
               100                 105                 110
   cgg tgg ccc cgt gga ggc ggc acc ctc ttg gac ttg gag ttc ctc aca      384
   Arg Trp Pro Arg Gly Gly Gly Thr Leu Leu Asp Leu Glu Phe Leu Thr
           115                 120                 125
   gat gat gtt ggt cgc agg gta gct gct ttc gga tac tct gct ggt tat      432
   Asp Asp Val Gly Arg Arg Val Ala Ala Phe Gly Tyr Ser Ala Gly Tyr
       130                 135                 140
   gca ggc tct gct ctg gcc gtc aag aac tgg gcc tgg caa ttg aca cac      480
   Ala Gly Ser Ala Leu Ala Val Lys Asn Trp Ala Trp Gln Leu Thr His
   145                 150                 155                 160
   ccc gag ggc gag cct ctg ccc ggc gag aca ccc tat gag aac caa gat        528
   Pro Glu Gly Glu Pro Leu Pro Gly Glu Thr Pro Tyr Glu Asn Gln Asp
                   165                 170                 175
   ctt ctg atc gcg tcc gtg aag gag tcg ctg gag gtt ggc aag aag cag        576
   Leu Leu Ile Ala Ser Val Lys Glu Ser Leu Glu Val Gly Lys Lys Gln
               180                 185                 190
   tct gga aag tcg ccc aag gtg ctt gtc att gga gct ctt ggc cgt tgc        624
   Ser Gly Lys Ser Pro Lys Val Leu Val Ile Gly Ala Leu Gly Arg Cys
           195                 200                 205
   ggc aag ggt gct gtg cag ctg gcc aag gat gtt ggc att ccc gag tct        672
   Gly Lys Gly Ala Val Gln Leu Ala Lys Asp Val Gly Ile Pro Glu Ser
       210                 215                 220
   gat atc atc cag tgg gat att gaa gag acc aag aag ggt gga ccc ttc        720
   Asp Ile Ile Gln Trp Asp Ile Glu Glu Thr Lys Lys Gly Gly Pro Phe
   225                 230                 235                 240
   cgt gag atc gtt gag gac gtt gat atc ttc gtc aac tgc atc tac ctc        768
   Arg Glu Ile Val Glu Asp Val Asp Ile Phe Val Asn Cys Ile Tyr Leu
                   245                 250                 255
   tcc gcc aag atc cct cct ttc gtc aac gcc gag acc ctc tct act cct        816
   Ser Ala Lys Ile Pro Pro Phe Val Asn Ala Glu Thr Leu Ser Thr Pro
               260                 265                 270
   aac cgc cgc ttg tct gtc att tgc gac gtg agc gct gac aca acc aac        864
   Asn Arg Arg Leu Ser Val Ile Cys Asp Val Ser Ala Asp Thr Thr Asn
           275                 280                 285
   ccc cac aac cct atc ccc gtc tac tcg atc acc acc acc ttt gat aag        912
   Pro His Asn Pro Ile Pro Val Tyr Ser Ile Thr Thr Thr Phe Asp Lys
       290                 295                 300
   ccc acg gtg cca gtg acc ctg tcc gcc ggc gcc cag ggt ccc cct ctg        960
   Pro Thr Val Pro Val Thr Leu Ser Ala Gly Ala Gln Gly Pro Pro Leu
   305                 310                 315                 320
   agc gtg atc agc att gat cat ctc ccc tct ctc ttg ccc cgt gag agc       1008
   Ser Val Ile Ser Ile Asp His Leu Pro Ser Leu Leu Pro Arg Glu Ser
                   325                 330                 335
   tcc gag atg ttc agc gaa gcg ctg ctg cct agc ctg ctg cag ctc aag       1056
   Ser Glu Met Phe Ser Glu Ala Leu Leu Pro Ser Leu Leu Gln Leu Lys
               340                 345                 350
   gac aga aag aac gct cgc gtc tgg aag cag gcg gag gat ctg ttt aac       1104
   Asp Arg Lys Asn Ala Arg Val Trp Lys Gln Ala Glu Asp Leu Phe Asn
           355                 360                 365
   gag aag gtc gct acg ttg ccc gag tcg atg cgc gca taa                   1143
   Glu Lys Val Ala Thr Leu Pro Glu Ser Met Arg Ala
       370                 375                 380
<210> 2
   <211> 380
   <212> PRT
   <213> Aspergillus clavatus
<400> 2
   Met Ser Ser Asn Lys Ile Trp Leu Arg Ala Glu Thr Lys Pro Ala Glu
   1               5                   10                  15
   Ala Arg Ser Ala Leu Thr Pro Thr Thr Cys Lys Ala Leu Met Asp Ala
               20                  25                  30
   Gly Tyr Asp Val Thr Val Glu Arg Ser Thr Gln Arg Ile Phe Asp Gly
           35                  40                  45
   Glu Asp Asn Ile Leu Ser Tyr Leu Ile Gly Ala Pro Leu Val Glu Glu
       50                  55                  60
   Gly Ser Trp Val Lys Asp Ala Pro Lys Asp Ala Tyr Val Leu Gly Leu
   65                  70                  75                  80
   Lys Glu Leu Pro Glu Asp Asp Phe Pro Leu Glu His Val His Ile Ser
                   85                  90                  95
   Phe Ala His Cys Tyr Lys Glu Gln Gly Gly Trp Glu Lys Val Leu Ser
               100                 105                 110
   Arg Trp Pro Arg Gly Gly Gly Thr Leu Leu Asp Leu Glu Phe Leu Thr
           115                 120                 125
   Asp Asp Val Gly Arg Arg Val Ala Ala Phe Gly Tyr Ser Ala Gly Tyr
       130                 135                 140
   Ala Gly Ser Ala Leu Ala Val Lys Asn Trp Ala Trp Gln Leu Thr His
   145                 150                 155                 160
   Pro Glu Gly Glu Pro Leu Pro Gly Glu Thr Pro Tyr Glu Asn Gln Asp
                   165                 170                 175
   Leu Leu Ile Ala Ser Val Lys Glu Ser Leu Glu Val Gly Lys Lys Gln
               180                 185                 190
   Ser Gly Lys Ser Pro Lys Val Leu Val Ile Gly Ala Leu Gly Arg Cys
           195                 200                 205
   Gly Lys Gly Ala Val Gln Leu Ala Lys Asp Val Gly Ile Pro Glu Ser
       210                 215                 220
   Asp Ile Ile Gln Trp Asp Ile Glu Glu Thr Lys Lys Gly Gly Pro Phe
   225                 230                 235                 240
   Arg Glu Ile Val Glu Asp Val Asp Ile Phe Val Asn Cys Ile Tyr Leu
                   245                 250                 255
   Ser Ala Lys Ile Pro Pro Phe Val Asn Ala Glu Thr Leu Ser Thr Pro
               260                 265                 270
   Asn Arg Arg Leu Ser Val Ile Cys Asp Val Ser Ala Asp Thr Thr Asn
           275                 280                 285
   Pro His Asn Pro Ile Pro Val Tyr Ser Ile Thr Thr Thr Phe Asp Lys
       290                 295                 300
   Pro Thr Val Pro Val Thr Leu Ser Ala Gly Ala Gln Gly Pro Pro Leu
   305                 310                 315                 320
   Ser Val Ile Ser Ile Asp His Leu Pro Ser Leu Leu Pro Arg Glu Ser
                   325                 330                 335
   Ser Glu Met Phe Ser Glu Ala Leu Leu Pro Ser Leu Leu Gln Leu Lys
               340                 345                 350
   Asp Arg Lys Asn Ala Arg Val Trp Lys Gln Ala Glu Asp Leu Phe Asn
           355                 360                 365
   Glu Lys Val Ala Thr Leu Pro Glu Ser Met Arg Ala
       370                 375                 380
<210> 3
   <211> 1125
   <212> DNA
   <213> Aspergillus fumigatus
<220>
   <221> CDS
   <222> (1)..(1125)
   <223> Lys1
<400> 3
   atg tca agc aat aag atc tgg ttg cgc gcg gaa acc aag cct gcc gag       48
   Met Ser Ser Asn Lys Ile Trp Leu Arg Ala Glu Thr Lys Pro Ala Glu
   1               5                   10                  15
   gct cgg tct gct ttg acc ccg act acc tgc aag gcc ctt atg gat gct       96
   Ala Arg Ser Ala Leu Thr Pro Thr Thr Cys Lys Ala Leu Met Asp Ala
               20                  25                  30
   ggc tac gag gtg acc gtg gaa cgt tcc aga cag cgg att ttc gac gtt      144
   Gly Tyr Glu Val Thr Val Glu Arg Ser Arg Gln Arg Ile Phe Asp Val
           35                  40                  45
   gta cag atc ggc gcc ccc ctc gtc gag gaa ggt tca tgg gta aag gac      192
   Val Gln Ile Gly Ala Pro Leu Val Glu Glu Gly Ser Trp Val Lys Asp
       50                  55                  60
   gca ccc aaa gat gcc tac atc ctc ggt ctg aag gag ctt ccc gag gac      240
   Ala Pro Lys Asp Ala Tyr Ile Leu Gly Leu Lys Glu Leu Pro Glu Asp
   65                  70                  75                  80
   gat ttt ccg ctt gag cac gta cac atc tcc ttt gcg cac tgc tac aag      288
   Asp Phe Pro Leu Glu His Val His Ile Ser Phe Ala His Cys Tyr Lys
                   85                  90                  95
   caa cag gct ggc tgg gag aag gtg ctc agc cgg tgg ccc cgc gga ggc      336
   Gln Gln Ala Gly Trp Glu Lys Val Leu Ser Arg Trp Pro Arg Gly Gly
               100                 105                 110
   ggc acc ctc ttg gac ttg gag ttc ctc aca gat gag act gga cgc cga      384
   Gly Thr Leu Leu Asp Leu Glu Phe Leu Thr Asp Glu Thr Gly Arg Arg
           115                 120                 125
   gta gct gct ttt ggg tac tcc gcc ggt tac gca ggt tct gct ttg gcc      432
   Val Ala Ala Phe Gly Tyr Ser Ala Gly Tyr Ala Gly Ser Ala Leu Ala
       130                 135                 140
   att aag aac tgg gcc tgg caa ttg acg cat cct gag ggc gag ccg ctt      480
   Ile Lys Asn Trp Ala Trp Gln Leu Thr His Pro Glu Gly Glu Pro Leu
   145                 150                 155                 160
   cct ggc gag act ccc tac gca aac cag gat ctg ttg att gag tca gtg      528
   Pro Gly Glu Thr Pro Tyr Ala Asn Gln Asp Leu Leu Ile Glu Ser Val
                   165                 170                 175
   aag gag tcg ttg gag tct ggc aag aag ctg tcc ggc agg ccg ccc aag      576
   Lys Glu Ser Leu Glu Ser Gly Lys Lys Leu Ser Gly Arg Pro Pro Lys
               180                 185                 190
   gtg ctt gtc att gga gct ctt gga cgc tgt ggc aaa gga gcg gtt cag      624
   Val Leu Val Ile Gly Ala Leu Gly Arg Cys Gly Lys Gly Ala Val Gln
           195                 200                 205
   ctg gcc aag gat gtc ggc att cct gag tcg gat atc atc cag tgg gat      672
   Leu Ala Lys Asp Val Gly Ile Pro Glu Ser Asp Ile Ile Gln Trp Asp
       210                 215                 220
   ata gaa gag acc aag aag ggt ggc ccc ttc aga gag att gtc gag gat      720
   Ile Glu Glu Thr Lys Lys Gly Gly Pro Phe Arg Glu Ile Val Glu Asp
   225                 230                 235                 240
   gca gac att ttc atc aac tgc atc tac ctc tcc gct aag atc cct cct      768
   Ala Asp Ile Phe Ile Asn Cys Ile Tyr Leu Ser Ala Lys Ile Pro Pro
                   245                 250                 255
   ttc gtc aac acc gaa act ctg tct tct cct aac cgc cgc ttg tcc gtc      816
   Phe Val Asn Thr Glu Thr Leu Ser Ser Pro Asn Arg Arg Leu Ser Val
               260                 265                 270
   att tgt gac gtg agc gcc gac aca acc aat ccc aac aat cct atc cct      864
   Ile Cys Asp Val Ser Ala Asp Thr Thr Asn Pro Asn Asn Pro Ile Pro
           275                 280                 285
   gtc tat tcc atc aca acc act ttc gac aag ccc aca gtc act gtt cct      912
   Val Tyr Ser Ile Thr Thr Thr Phe Asp Lys Pro Thr Val Thr Val Pro
       290                 295                 300
   ctt ccg gaa ctg gcc cag ggc cct cca ttg agc gtg atc agc atc gac      960
   Leu Pro Glu Leu Ala Gln Gly Pro Pro Leu Ser Val Ile Ser Ile Asp
   305                 310                 315                 320
   cac ctc ccc tcc ctc ctt cct cgt gaa agc tcc gag atg ttc agc gaa     1008
   His Leu Pro Ser Leu Leu Pro Arg Glu Ser Ser Glu Met Phe Ser Glu
                   325                 330                 335
   gcc tta ctg ccg agc cta ctg caa ctc aag gat aga aag aac gct cgt     1056
   Ala Leu Leu Pro Ser Leu Leu Gln Leu Lys Asp Arg Lys Asn Ala Arg
               340                 345                 350
   gtc tgg aag caa gca gag gac ttg ttc aac gaa aag gtt gct acc ttg       1104
   Val Trp Lys Gln Ala Glu Asp Leu Phe Asn Glu Lys Val Ala Thr Leu
           355                 360                 365
   ccc gag tcg atg cgc gct taa                                           1125
   Pro Glu Ser Met Arg Ala
       370
<210> 4
   <211> 374
   <212> PRT
   <213> Aspergillus fumigatus
<400> 4
   Met Ser Ser Asn Lys Ile Trp Leu Arg Ala Glu Thr Lys Pro Ala Glu
   1               5                   10                  15
   Ala Arg Ser Ala Leu Thr Pro Thr Thr Cys Lys Ala Leu Met Asp Ala
               20                  25                  30
   Gly Tyr Glu Val Thr Val Glu Arg Ser Arg Gln Arg Ile Phe Asp Val
           35                  40                  45
   Val Gln Ile Gly Ala Pro Leu Val Glu Glu Gly Ser Trp Val Lys Asp
       50                  55                  60
   Ala Pro Lys Asp Ala Tyr Ile Leu Gly Leu Lys Glu Leu Pro Glu Asp
   65                  70                  75                  80
   Asp Phe Pro Leu Glu His Val His Ile Ser Phe Ala His Cys Tyr Lys
                   85                  90                  95
   Gln Gln Ala Gly Trp Glu Lys Val Leu Ser Arg Trp Pro Arg Gly Gly
               100                 105                 110
   Gly Thr Leu Leu Asp Leu Glu Phe Leu Thr Asp Glu Thr Gly Arg Arg
           115                 120                 125
   Val Ala Ala Phe Gly Tyr Ser Ala Gly Tyr Ala Gly Ser Ala Leu Ala
       130                 135                 140
   Ile Lys Asn Trp Ala Trp Gln Leu Thr His Pro Glu Gly Glu Pro Leu
   145                 150                 155                 160
   Pro Gly Glu Thr Pro Tyr Ala Asn Gln Asp Leu Leu Ile Glu Ser Val
                   165                 170                 175
   Lys Glu Ser Leu Glu Ser Gly Lys Lys Leu Ser Gly Arg Pro Pro Lys
               180                 185                 190
   Val Leu Val Ile Gly Ala Leu Gly Arg Cys Gly Lys Gly Ala Val Gln
           195                 200                 205
   Leu Ala Lys Asp Val Gly Ile Pro Glu Ser Asp Ile Ile Gln Trp Asp
       210                 215                 220
   Ile Glu Glu Thr Lys Lys Gly Gly Pro Phe Arg Glu Ile Val Glu Asp
   225                 230                 235                 240
   Ala Asp Ile Phe Ile Asn Cys Ile Tyr Leu Ser Ala Lys Ile Pro Pro
                   245                 250                 255
   Phe Val Asn Thr Glu Thr Leu Ser Ser Pro Asn Arg Arg Leu Ser Val
               260                 265                 270
   Ile Cys Asp Val Ser Ala Asp Thr Thr Asn Pro Asn Asn Pro Ile Pro
           275                 280                 285
   Val Tyr Ser Ile Thr Thr Thr Phe Asp Lys Pro Thr Val Thr Val Pro
       290                 295                 300
   Leu Pro Glu Leu Ala Gln Gly Pro Pro Leu Ser Val Ile Ser Ile Asp
   305                 310                 315                 320
   His Leu Pro Ser Leu Leu Pro Arg Glu Ser Ser Glu Met Phe Ser Glu
                   325                 330                 335
   Ala Leu Leu Pro Ser Leu Leu Gln Leu Lys Asp Arg Lys Asn Ala Arg
               340                 345                 350
   Val Trp Lys Gln Ala Glu Asp Leu Phe Asn Glu Lys Val Ala Thr Leu
           355                 360                 365
   Pro Glu Ser Met Arg Ala
       370
<210> 5
   <211> 1119
   <212> DNA
   <213> Botrytis cinerea
<220>
   <221> CDS
   <222> (1)..(1119)
   <223> Lys1
<400> 5
   atg tct gga act act ctt cac cta cgt tct gag ttg ggc aaa gct ctt          48
   Met Ser Gly Thr Thr Leu His Leu Arg Ser Glu Leu Gly Lys Ala Leu
   1               5                   10                  15
   gag cat cga tcc gct ctt acc ccc acc acc gcc aaa gct ctc atc gat          96
   Glu His Arg Ser Ala Leu Thr Pro Thr Thr Ala Lys Ala Leu Ile Asp
                   20                  25                  30
   gct gga tac aca atc aac gtt gag cgc agt cca gag cgt ata ttc gac       144
   Ala Gly Tyr Thr Ile Asn Val Glu Arg Ser Pro Glu Arg Ile Phe Asp
           35                  40                  45
   gat gaa gag ttc gag aag gtt ggt gct act ctt gtg cca gag aac aca       192
   Asp Glu Glu Phe Glu Lys Val Gly Ala Thr Leu Val Pro Glu Asn Thr
       50                  55                  60
   tgg aga caa gca cca aag gat cac att atc att ggg ttg aag gaa ctg       240
   Trp Arg Gln Ala Pro Lys Asp His Ile Ile Ile Gly Leu Lys Glu Leu
   65                  70                  75                  80
   ccc gtc gaa gaa ttt cct ctc gag cat gtt cac gta caa ttc gca cat       288
   Pro Val Glu Glu Phe Pro Leu Glu His Val His Val Gln Phe Ala His
                   85                  90                  95
   tgt tat aaa caa cag ggc ggc tgg gac act gtt cta tca cga ttc cct       336
   Cys Tyr Lys Gln Gln Gly Gly Trp Asp Thr Val Leu Ser Arg Phe Pro
               100                 105                 110
   cgt gga ggt gga act ctc tta gat ctt gag ttt ttg aca gac gac aga       384
   Arg Gly Gly Gly Thr Leu Leu Asp Leu Glu Phe Leu Thr Asp Asp Arg
           115                 120                 125
   ggc aga aga gtt gca gcc ttt gga tac cat gct gga ttt gct ggt gca       432
   Gly Arg Arg Val Ala Ala Phe Gly Tyr His Ala Gly Phe Ala Gly Ala
       130                 135                 140
   gca ttg gca ctc gaa aat tgg gcg tgg caa ctc acc cac cca gca tcc       480
   Ala Leu Ala Leu Glu Asn Trp Ala Trp Gln Leu Thr His Pro Ala Ser
   145                 150                 155                 160
   gag ccc ttc cca agt gta tcg agc tac ccc aac gaa gat gaa ttg att       528
   Glu Pro Phe Pro Ser Val Ser Ser Tyr Pro Asn Glu Asp Glu Leu Ile
                   165                 170                 175
   gtg gat gtt aag aag gca atc gca gct gga caa gag aag acc ggc aag       576
   Val Asp Val Lys Lys Ala Ile Ala Ala Gly Gln Glu Lys Thr Gly Lys
               180                 185                 190
   gca cca cga gtt ttg gtt att ggt gca tta ggc aga tgt gga agt gga       624
   Ala Pro Arg Val Leu Val Ile Gly Ala Leu Gly Arg Cys Gly Ser Gly
           195                 200                 205
   gca gtt gac ctc tgc ttg aga gct ggt gtg cca acc gaa aat gtg ttg       672
   Ala Val Asp Leu Cys Leu Arg Ala Gly Val Pro Thr Glu Asn Val Leu
       210                 215                 220
   aag tgg gat atg gcc gag acc gct aag gga ggg cca ttc cca gag atc       720
   Lys Trp Asp Met Ala Glu Thr Ala Lys Gly Gly Pro Phe Pro Glu Ile
   225                 230                 235                 240
   gtt gag agt gac atc ttc atc aac tgc ata tat ctc atg tcc aag att       768
   Val Glu Ser Asp Ile Phe Ile Asn Cys Ile Tyr Leu Met Ser Lys Ile
                   245                 250                 255
   cca aac ttt gtc gac atg caa agc ctc gat acc cca aac cgc aaa ttg       816
   Pro Asn Phe Val Asp Met Gln Ser Leu Asp Thr Pro Asn Arg Lys Leu
               260                 265                 270
   tca gtc gtc tgc gat gtc agt gct gat acc acc aac ccc aac aac cca       864
   Ser Val Val Cys Asp Val Ser Ala Asp Thr Thr Asn Pro Asn Asn Pro
           275                 280                 285
   att cca atc tat act gtt gca acc aca ttt tca gag cca act gtt cca        912
   Ile Pro Ile Tyr Thr Val Ala Thr Thr Phe Ser Glu Pro Thr Val Pro
       290                 295                 300
   gtt gag gtc aag gga gaa cca aga tta agt gtc atc agt att gat cac        960
   Val Glu Val Lys Gly Glu Pro Arg Leu Ser Val Ile Ser Ile Asp His
   305                 310                 315                 320
   ttg cca agt tta ttg cca aga gag gca agt gag gca ttt agc aag gac       1008
   Leu Pro Ser Leu Leu Pro Arg Glu Ala Ser Glu Ala Phe Ser Lys Asp
                   325                 330                 335
   tta tta cca agt ttg ttg tct ttg aag gat tgg aga aat act cca gtc       1056
   Leu Leu Pro Ser Leu Leu Ser Leu Lys Asp Trp Arg Asn Thr Pro Val
               340                 345                 350
   tgg gcc aag gca gag aag tta ttc cag gaa aag gtt gct act ttg ccc       1104
   Trp Ala Lys Ala Glu Lys Leu Phe Gln Glu Lys Val Ala Thr Leu Pro
           355                 360                 365
   aag aac gag gca tga                                                   1119
   Lys Asn Glu Ala
       370
<210> 6
   <211> 372
   <212> PRT
   <213> Botrytis cinerea
<400> 6
   Met Ser Gly Thr Thr Leu His Leu Arg Ser Glu Leu Gly Lys Ala Leu
   1               5                   10                  15
   Glu His Arg Ser Ala Leu Thr Pro Thr Thr Ala Lys Ala Leu Ile Asp
               20                  25                  30
   Ala Gly Tyr Thr Ile Asn Val Glu Arg Ser Pro Glu Arg Ile Phe Asp
           35                  40                  45
   Asp Glu Glu Phe Glu Lys Val Gly Ala Thr Leu Val Pro Glu Asn Thr
       50                  55                  60
   Trp Arg Gln Ala Pro Lys Asp His Ile Ile Ile Gly Leu Lys Glu Leu
   65                  70                  75                  80
   Pro Val Glu Glu Phe Pro Leu Glu His Val His Val Gln Phe Ala His
                   85                  90                  95
   Cys Tyr Lys Gln Gln Gly Gly Trp Asp Thr Val Leu Ser Arg Phe Pro
               100                 105                 110
   Arg Gly Gly Gly Thr Leu Leu Asp Leu Glu Phe Leu Thr Asp Asp Arg
           115                 120                 125
   Gly Arg Arg Val Ala Ala Phe Gly Tyr His Ala Gly Phe Ala Gly Ala
       130                 135                 140
   Ala Leu Ala Leu Glu Asn Trp Ala Trp Gln Leu Thr His Pro Ala Ser
   145                 150                 155                 160
   Glu Pro Phe Pro Ser Val Ser Ser Tyr Pro Asn Glu Asp Glu Leu Ile
                   165                 170                 175
   Val Asp Val Lys Lys Ala Ile Ala Ala Gly Gln Glu Lys Thr Gly Lys
               180                 185                 190
   Ala Pro Arg Val Leu Val Ile Gly Ala Leu Gly Arg Cys Gly Ser Gly
           195                 200                 205
   Ala Val Asp Leu Cys Leu Arg Ala Gly Val Pro Thr Glu Asn Val Leu
       210                 215                 220
   Lys Trp Asp Met Ala Glu Thr Ala Lys Gly Gly Pro Phe Pro Glu Ile
   225                 230                 235                 240
   Val Glu Ser Asp Ile Phe Ile Asn Cys Ile Tyr Leu Met Ser Lys Ile
                   245                 250                 255
   Pro Asn Phe Val Asp Met Gln Ser Leu Asp Thr Pro Asn Arg Lys Leu
               260                 265                 270
   Ser Val Val Cys Asp Val Ser Ala Asp Thr Thr Asn Pro Asn Asn Pro
           275                 280                 285
   Ile Pro Ile Tyr Thr Val Ala Thr Thr Phe Ser Glu Pro Thr Val Pro
       290                 295                 300
   Val Glu Val Lys Gly Glu Pro Arg Leu Ser Val Ile Ser Ile Asp His
   305                 310                 315                 320
   Leu Pro Ser Leu Leu Pro Arg Glu Ala Ser Glu Ala Phe Ser Lys Asp
                   325                 330                 335
   Leu Leu Pro Ser Leu Leu Ser Leu Lys Asp Trp Arg Asn Thr Pro Val
               340                 345                 350
   Trp Ala Lys Ala Glu Lys Leu Phe Gln Glu Lys Val Ala Thr Leu Pro
           355                 360                 365
   Lys Asn Glu Ala
       370
<210> 7
   <211> 1173
   <212> DNA
   <213> Fusarium graminearum
<220>
   <221> CDS
   <222> (1)..(1173)
   <223> Lys1
<400> 7
   atg tct caa tac ccc cac atc ctc ctg cgc gct gag gag aag ccc ctc        48
   Met Ser Gln Tyr Pro His Ile Leu Leu Arg Ala Glu Glu Lys Pro Leu
   1               5                   10                  15
   gag cac cga tct ttc tct ccc tca att atc aag act ctt gtc gac gct        96
   Glu His Arg Ser Phe Ser Pro Ser Ile Ile Lys Thr Leu Val Asp Ala
               20                  25                  30
   gga tat ccc gtc tct gtc gag cga tca tct aca gac ccc aag ttc aag       144
   Gly Tyr Pro Val Ser Val Glu Arg Ser Ser Thr Asp Pro Lys Phe Lys
           35                  40                  45
   cgc atc ttt gaa gac tcc gag tat gag gct gct ggt gcg cgc ctc gtc       192
   Arg Ile Phe Glu Asp Ser Glu Tyr Glu Ala Ala Gly Ala Arg Leu Val
       50                  55                  60
   aac gag ggc acc tgg ccc aac gcc gaa gcc gga act ttg att ctg ggc       240
   Asn Glu Gly Thr Trp Pro Asn Ala Glu Ala Gly Thr Leu Ile Leu Gly
   65                  70                  75                  80
   ctc aag gag att ccc gag gaa gac ttt cct ctc aag aac gac cac att       288
   Leu Lys Glu Ile Pro Glu Glu Asp Phe Pro Leu Lys Asn Asp His Ile
                   85                  90                  95
   tca ttt gcc cat tgt tac aag aac caa ggt gga tgg gaa aag gtc ctc       336
   Ser Phe Ala His Cys Tyr Lys Asn Gln Gly Gly Trp Glu Lys Val Leu
               100                 105                 110
   ggc cgc ttc cct caa gga agc agc gtt cta tac gat ttg gag ttc ctt       384
   Gly Arg Phe Pro Gln Gly Ser Ser Val Leu Tyr Asp Leu Glu Phe Leu
           115                 120                 125
   gtc gat gag caa gga cgc cga gtt tct gct ttc ggt ttc cac gct gga       432
   Val Asp Glu Gln Gly Arg Arg Val Ser Ala Phe Gly Phe His Ala Gly
       130                 135                 140
   ttc gct ggc gct gcc ctc ggt atc aag act ctt gcc cac cag ctt cag       480
   Phe Ala Gly Ala Ala Leu Gly Ile Lys Thr Leu Ala His Gln Leu Gln
   145                 150                 155                 160
   gac ccc tcc tcc aag ctc ccc tca gtg gag aca ttc acc gat ggt cgt       528
   Asp Pro Ser Ser Lys Leu Pro Ser Val Glu Thr Phe Thr Asp Gly Arg
                   165                 170                 175
   gga tac tac cta aac gag gag gag ctc gtt aac cag atc cga gaa gac       576
   Gly Tyr Tyr Leu Asn Glu Glu Glu Leu Val Asn Gln Ile Arg Glu Asp
               180                 185                 190
   ctt gcc aag gcc gag aag tct ctt gga cgc aag ccc act gct ctc gtc       624
   Leu Ala Lys Ala Glu Lys Ser Leu Gly Arg Lys Pro Thr Ala Leu Val
           195                 200                 205
   ctc ggt gct ctt gga cga tgt ggt aag ggt gcc gtt gac ctg ttc ctc       672
   Leu Gly Ala Leu Gly Arg Cys Gly Lys Gly Ala Val Asp Leu Phe Leu
           210                 215                 220
   aag gct ggc atg ccc gac gag aac atc acc cgc tgg gac ttg aac gag        720
   Lys Ala Gly Met Pro Asp Glu Asn Ile Thr Arg Trp Asp Leu Asn Glu
   225                 230                 235                 240
   acc aag gac cga gat ggt cct tat gag gag att gcc cag gcc gat gtc        768
   Thr Lys Asp Arg Asp Gly Pro Tyr Glu Glu Ile Ala Gln Ala Asp Val
                   245                 250                 255
   ttc ctt aac gcc atc tac ctt tcc aag ccc atc cct cct ttc atc aac        816
   Phe Leu Asn Ala Ile Tyr Leu Ser Lys Pro Ile Pro Pro Phe Ile Asn
               260                 265                 270
   gag gag ctt ctc gcc aag tct ggc cgc aac ttg gct gtt gtt atc gat        864
   Glu Glu Leu Leu Ala Lys Ser Gly Arg Asn Leu Ala Val Val Ile Asp
           275                 280                 285
   gtc tcc tgc gac acc acc aac cct cac aac ccc atc ccc atc tac tct        912
   Val Ser Cys Asp Thr Thr Asn Pro His Asn Pro Ile Pro Ile Tyr Ser
       290                 295                 300
   atc aac acc aca ttc gag gag ccc acc gtc ccc gtc gag atc aag aac        960
   Ile Asn Thr Thr Phe Glu Glu Pro Thr Val Pro Val Glu Ile Lys Asn
   305                 310                 315                 320
   gac cag aac tct ctc cct cta tca gtt att agc atc gac cat ctc ccc       1008
   Asp Gln Asn Ser Leu Pro Leu Ser Val Ile Ser Ile Asp His Leu Pro
                   325                 330                 335
   tca atg ctg ccc cgt gag gct agt gag gcc ttt agc gag ggc ctc aag       1056
   Ser Met Leu Pro Arg Glu Ala Ser Glu Ala Phe Ser Glu Gly Leu Lys
               340                 345                 350
   gag tct ctc ctt aca ctc aag gac cgc aag act tcg cga gtg tgg gcc       1104
   Glu Ser Leu Leu Thr Leu Lys Asp Arg Lys Thr Ser Arg Val Trp Ala
           355                 360                 365
   gac gcc gag aag ctc ttc aac gag aag gtt gct ctg ctt ccc gag tcc       1152
   Asp Ala Glu Lys Leu Phe Asn Glu Lys Val Ala Leu Leu Pro Glu Ser
       370                 375                 380
   ctg cga acc aag aga gtt taa                                           1173
   Leu Arg Thr Lys Arg Val
   385                 390
<210> 8
   <211> 390
   <212> PRT
   <213> Fusarium graminearum
<400> 8
   Met Ser Gln Tyr Pro His Ile Leu Leu Arg Ala Glu Glu Lys Pro Leu
   1               5                   10                  15
   Glu His Arg Ser Phe Ser Pro Ser Ile Ile Lys Thr Leu Val Asp Ala
               20                  25                  30
   Gly Tyr Pro Val Ser Val Glu Arg Ser Ser Thr Asp Pro Lys Phe Lys
           35                  40                  45
   Arg Ile Phe Glu Asp Ser Glu Tyr Glu Ala Ala Gly Ala Arg Leu Val
       50                  55                  60
   Asn Glu Gly Thr Trp Pro Asn Ala Glu Ala Gly Thr Leu Ile Leu Gly
   65                  70                  75                  80
   Leu Lys Glu Ile Pro Glu Glu Asp Phe Pro Leu Lys Asn Asp His Ile
                   85                  90                  95
   Ser Phe Ala His Cys Tyr Lys Asn Gln Gly Gly Trp Glu Lys Val Leu
               100                 105                 110
   Gly Arg Phe Pro Gln Gly Ser Ser Val Leu Tyr Asp Leu Glu Phe Leu
           115                 120                 125
   Val Asp Glu Gln Gly Arg Arg Val Ser Ala Phe Gly Phe His Ala Gly
       130                 135                 140
   Phe Ala Gly Ala Ala Leu Gly Ile Lys Thr Leu Ala His Gln Leu Gln
   145                 150                 155                 160
   Asp Pro Ser Ser Lys Leu Pro Ser Val Glu Thr Phe Thr Asp Gly Arg
                   165                 170                 175
   Gly Tyr Tyr Leu Asn Glu Glu Glu Leu Val Asn Gln Ile Arg Glu Asp
               180                 185                 190
   Leu Ala Lys Ala Glu Lys Ser Leu Gly Arg Lys Pro Thr Ala Leu Val
           195                 200                 205
   Leu Gly Ala Leu Gly Arg Cys Gly Lys Gly Ala Val Asp Leu Phe Leu
       210                 215                 220
   Lys Ala Gly Met Pro Asp Glu Asn Ile Thr Arg Trp Asp Leu Asn Glu
   225                 230                 235                 240
   Thr Lys Asp Arg Asp Gly Pro Tyr Glu Glu Ile Ala Gln Ala Asp Val
                   245                 250                 255
   Phe Leu Asn Ala Ile Tyr Leu Ser Lys Pro Ile Pro Pro Phe Ile Asn
               260                 265                 270
   Glu Glu Leu Leu Ala Lys Ser Gly Arg Asn Leu Ala Val Val Ile Asp
           275                 280                 285
   Val Ser Cys Asp Thr Thr Asn Pro His Asn Pro Ile Pro Ile Tyr Ser
       290                 295                 300
   Ile Asn Thr Thr Phe Glu Glu Pro Thr Val Pro Val Glu Ile Lys Asn
   305                 310                 315                 320
   Asp Gln Asn Ser Leu Pro Leu Ser Val Ile Ser Ile Asp His Leu Pro
                   325                 330                 335
   Ser Met Leu Pro Arg Glu Ala Ser Glu Ala Phe Ser Glu Gly Leu Lys
               340                 345                 350
   Glu Ser Leu Leu Thr Leu Lys Asp Arg Lys Thr Ser Arg Val Trp Ala
           355                 360                 365
   Asp Ala Glu Lys Leu Phe Asn Glu Lys Val Ala Leu Leu Pro Glu Ser
       370                 375                 380
   Leu Arg Thr Lys Arg Val
   385                 390
<210> 9
   <211> 591
   <212> DNA
   <213> Fusarium oxysporum
<220>
   <221> CDS
   <222> (1)..(591)
   <223> Lys1
<400> 9
   atg tct gaa tat ccc cac atc cta ctt cgc gct gag gag aag cct ctc          48
   Met Ser Glu Tyr Pro His Ile Leu Leu Arg Ala Glu Glu Lys Pro Leu
   1               5                   10                  15
   gag cac cga tct ttc tcc ccc gcc gtt atc aag aca ctc gtc gat gct          96
   Glu His Arg Ser Phe Ser Pro Ala Val Ile Lys Thr Leu Val Asp Ala
               20                  25                  30
   gga tac ccc att tcc gtc gag cga tcg tcc aca gac ccc aag ttt aag         144
   Gly Tyr Pro Ile Ser Val Glu Arg Ser Ser Thr Asp Pro Lys Phe Lys
           35                  40                  45
   cgt atc ttt gag gac tca gag tat gag gct gct ggc gct cgt ctt gtc         192
   Arg Ile Phe Glu Asp Ser Glu Tyr Glu Ala Ala Gly Ala Arg Leu Val
       50                  55                  60
   gat acg ggt gtc tgg ccc aac gct gag cct ggg aca atc atc ctg ggg         240
   Asp Thr Gly Val Trp Pro Asn Ala Glu Pro Gly Thr Ile Ile Leu Gly
   65                  70                  75                  80
   ctg aag gag ata ccc gag gag gac ttc ccc ctc aag aat gac cac atc         288
   Leu Lys Glu Ile Pro Glu Glu Asp Phe Pro Leu Lys Asn Asp His Ile
                   85                  90                  95
   aca ttc gct cac tgc tac aag aat caa ggg ggc tgg gag aag gta ctc         336
   Thr Phe Ala His Cys Tyr Lys Asn Gln Gly Gly Trp Glu Lys Val Leu
               100                 105                 110
   ggt cgc tgg gca caa ggc ggc tca gtt ttg tat gac ttg gag ttc ttg         384
   Gly Arg Trp Ala Gln Gly Gly Ser Val Leu Tyr Asp Leu Glu Phe Leu
           115                 120                 125
   cac gat tct gaa ggt cgt cgg gtt tcg gcc ttc ggt ttc cac gct ggt         432
   His Asp Ser Glu Gly Arg Arg Val Ser Ala Phe Gly Phe His Ala Gly
       130                 135                 140
   ttt gca gga gct gcg ctc gga att aag acg ctt gct cac cag ctg cag         480
   Phe Ala Gly Ala Ala Leu Gly Ile Lys Thr Leu Ala His Gln Leu Gln
   145                 150                 155                 160
   gac tcg tct tct aag ctc cct tca gtc gag aca ttc acc gat ggc cgc         528
   Asp Ser Ser Ser Lys Leu Pro Ser Val Glu Thr Phe Thr Asp Gly Arg
                   165                 170                 175
   gga tac tac ctg aac gag gat gag ctc gtc aac cca gat tcg cga gga         576
   Gly Tyr Tyr Leu Asn Glu Asp Glu Leu Val Asn Pro Asp Ser Arg Gly
               180                 185                 190
   tct cac caa ggc tga                                                     591
   Ser His Gln Gly
           195
<210> 10
   <211> 196
   <212> PRT
   <213> Fusarium oxysporum
<400> 10
   Met Ser Glu Tyr Pro His Ile Leu Leu Arg Ala Glu Glu Lys Pro Leu
   1               5                   10                  15
   Glu His Arg Ser Phe Ser Pro Ala Val Ile Lys Thr Leu Val Asp Ala
               20                  25                  30
   Gly Tyr Pro Ile Ser Val Glu Arg Ser Ser Thr Asp Pro Lys Phe Lys
           35                  40                  45
   Arg Ile Phe Glu Asp Ser Glu Tyr Glu Ala Ala Gly Ala Arg Leu Val
       50                  55                  60
   Asp Thr Gly Val Trp Pro Asn Ala Glu Pro Gly Thr Ile Ile Leu Gly
   65                  70                  75                  80
   Leu Lys Glu Ile Pro Glu Glu Asp Phe Pro Leu Lys Asn Asp His Ile
                   85                  90                  95
   Thr Phe Ala His Cys Tyr Lys Asn Gln Gly Gly Trp Glu Lys Val Leu
               100                 105                 110
   Gly Arg Trp Ala Gln Gly Gly Ser Val Leu Tyr Asp Leu Glu Phe Leu
           115                 120                 125
   His Asp Ser Glu Gly Arg Arg Val Ser Ala Phe Gly Phe His Ala Gly
       130                 135                 140
   Phe Ala Gly Ala Ala Leu Gly Ile Lys Thr Leu Ala His Gln Leu Gln
   145                 150                 155                 160
   Asp Ser Ser Ser Lys Leu Pro Ser Val Glu Thr Phe Thr Asp Gly Arg
                   165                 170                 175
   Gly Tyr Tyr Leu Asn Glu Asp Glu Leu Val Asn Pro Asp Ser Arg Gly
               180                 185                 190
   Ser His Gln Gly
           195
<210> 11
   <211> 1104
   <212> DNA
   <213> Fusarium verticillioides
<220>
   <221> CDS
   <222> (1)..(1104)
   <223> Lys1
<400> 11
   atg tcg cct gtc gtc att cat ctc cgg acc gag aca aag cct cta gag       48
   Met Ser Pro Val Val Ile His Leu Arg Thr Glu Thr Lys Pro Leu Glu
   1               5                   10                  15
   cgt cgc tct cca ttg tca cct gcg act gct aag gct ttg ctc ggc gct       96
   Arg Arg Ser Pro Leu Ser Pro Ala Thr Ala Lys Ala Leu Leu Gly Ala
               20                  25                  30
   ggc tac aca gtt cga gtt gag gaa tcc ccg gac cgc att tac aag att      144
   Gly Tyr Thr Val Arg Val Glu Glu Ser Pro Asp Arg Ile Tyr Lys Ile
           35                  40                  45
   gac gag ttc aga gat gtt ggg gct gaa atc gta ccc gct ggc tcg tgg      192
   Asp Glu Phe Arg Asp Val Gly Ala Glu Ile Val Pro Ala Gly Ser Trp
       50                  55                  60
   gtg aat gca ccc aag gag gat att atc ctg gga ttg aag gag att gag      240
   Val Asn Ala Pro Lys Glu Asp Ile Ile Leu Gly Leu Lys Glu Ile Glu
   65                  70                  75                  80
   gcg aat ggt aca ccg tta ctt cac acc tac att cac ttt gct cat gta      288
   Ala Asn Gly Thr Pro Leu Leu His Thr Tyr Ile His Phe Ala His Val
                   85                  90                  95
   ttc aag aag caa agc ggc tgg gct act gag ctg tcc cgc ttc gca aac      336
   Phe Lys Lys Gln Ser Gly Trp Ala Thr Glu Leu Ser Arg Phe Ala Asn
               100                 105                 110
   gcg ggt ggc tta cta tat gac ttg gaa ttt ctt acg gac caa gat gga      384
   Ala Gly Gly Leu Leu Tyr Asp Leu Glu Phe Leu Thr Asp Gln Asp Gly
           115                 120                 125
   cgc cgg gta gct gca ttc gga tat tgg gcg gga tat gcc ggg acc gct      432
   Arg Arg Val Ala Ala Phe Gly Tyr Trp Ala Gly Tyr Ala Gly Thr Ala
       130                 135                 140
   ctg gca ctc ctg tct tgg gct cac cag ctg ctc aac cct ggc gta ccc      480
   Leu Ala Leu Leu Ser Trp Ala His Gln Leu Leu Asn Pro Gly Val Pro
   145                 150                 155                 160
   caa gga ccg gtt ccc gtc ttc gac tct gcc tct gct ttg act gaa ctt      528
   Gln Gly Pro Val Pro Val Phe Asp Ser Ala Ser Ala Leu Thr Glu Leu
                   165                 170                 175
   gtc aag ggt aaa gtc gac gct gca cgc tcc gcc aac cac ggc gcg ctc      576
   Val Lys Gly Lys Val Asp Ala Ala Arg Ser Ala Asn His Gly Ala Leu
               180                 185                 190
   cct cga ctg atc gta atc ggt gcc ctg ggc cgc tgc ggc aaa ggt gcc      624
   Pro Arg Leu Ile Val Ile Gly Ala Leu Gly Arg Cys Gly Lys Gly Ala
           195                 200                 205
   atc gca gca gct gag gcc att ggt gtt agt gac att ctg aaa tgg gac      672
   Ile Ala Ala Ala Glu Ala Ile Gly Val Ser Asp Ile Leu Lys Trp Asp
       210                 215                 220
   att gct gag acg agc aag ggt ggc cca ttt cct gaa gtc gcc tcg tct      720
   Ile Ala Glu Thr Ser Lys Gly Gly Pro Phe Pro Glu Val Ala Ser Ser
   225                 230                 235                 240
   gat att ttt gtg aac tgc gtc tac cta ggt tcc aac aag att cca ccc      768
   Asp Ile Phe Val Asn Cys Val Tyr Leu Gly Ser Asn Lys Ile Pro Pro
                   245                 250                 255
   ttc aca act ttt gaa gca ctc tca gga ccc ggc aga cga ctc cgg gtc      816
   Phe Thr Thr Phe Glu Ala Leu Ser Gly Pro Gly Arg Arg Leu Arg Val
               260                 265                 270
   att tgc gac gtc agt tgc gac ccc aac agc gag aat aac cct gtt ccc      864
   Ile Cys Asp Val Ser Cys Asp Pro Asn Ser Glu Asn Asn Pro Val Pro
           275                 280                 285
   gtc tac tcc agc tac agt tcg ttc gaa aac ccg act gtc cct gct tct      912
   Val Tyr Ser Ser Tyr Ser Ser Phe Glu Asn Pro Thr Val Pro Ala Ser
       290                 295                 300
   gag cat att gat ggt cct gaa ctg cgc atc att gct atc gat cat ctt      960
   Glu His Ile Asp Gly Pro Glu Leu Arg Ile Ile Ala Ile Asp His Leu
   305                 310                 315                 320
   cct acc atg gtt gca cgc gag tcg agt gac gag tat tcc tca cta ctt     1008
   Pro Thr Met Val Ala Arg Glu Ser Ser Asp Glu Tyr Ser Ser Leu Leu
                   325                 330                 335
   ctg cca agt ttg ttg act ctg gac cgc cgg gat act gag ggg gtt tgg     1056
   Leu Pro Ser Leu Leu Thr Leu Asp Arg Arg Asp Thr Glu Gly Val Trp
               340                 345                 350
   cag cga gca gaa cgg atc ttt cgc gag aag gtt gca gag ctg ccc tag     1104
   Gln Arg Ala Glu Arg Ile Phe Arg Glu Lys Val Ala Glu Leu Pro
           355                 360                 365
<210> 12
   <211> 367
   <212> PRT
   <213> Fusarium verticillioides
<400> 12
   Met Ser Pro Val Val Ile His Leu Arg Thr Glu Thr Lys Pro Leu Glu
   1               5                   10                  15
   Arg Arg Ser Pro Leu Ser Pro Ala Thr Ala Lys Ala Leu Leu Gly Ala
               20                  25                  30
   Gly Tyr Thr Val Arg Val Glu Glu Ser Pro Asp Arg Ile Tyr Lys Ile
           35                  40                  45
   Asp Glu Phe Arg Asp Val Gly Ala Glu Ile Val Pro Ala Gly Ser Trp
       50                  55                  60
   Val Asn Ala Pro Lys Glu Asp Ile Ile Leu Gly Leu Lys Glu Ile Glu
   65                  70                  75                  80
   Ala Asn Gly Thr Pro Leu Leu His Thr Tyr Ile His Phe Ala His Val
                   85                  90                  95
   Phe Lys Lys Gln Ser Gly Trp Ala Thr Glu Leu Ser Arg Phe Ala Asn
               100                 105                 110
   Ala Gly Gly Leu Leu Tyr Asp Leu Glu Phe Leu Thr Asp Gln Asp Gly
           115                 120                 125
   Arg Arg Val Ala Ala Phe Gly Tyr Trp Ala Gly Tyr Ala Gly Thr Ala
       130                 135                 140
   Leu Ala Leu Leu Ser Trp Ala His Gln Leu Leu Asn Pro Gly Val Pro
   145                 150                 155                 160
   Gln Gly Pro Val Pro Val Phe Asp Ser Ala Ser Ala Leu Thr Glu Leu
                   165                 170                 175
   Val Lys Gly Lys Val Asp Ala Ala Arg Ser Ala Asn His Gly Ala Leu
               180                 185                 190
   Pro Arg Leu Ile Val Ile Gly Ala Leu Gly Arg Cys Gly Lys Gly Ala
           195                 200                 205
   Ile Ala Ala Ala Glu Ala Ile Gly Val Ser Asp Ile Leu Lys Trp Asp
       210                 215                 220
   Ile Ala Glu Thr Ser Lys Gly Gly Pro Phe Pro Glu Val Ala Ser Ser
   225                 230                 235                 240
   Asp Ile Phe Val Asn Cys Val Tyr Leu Gly Ser Asn Lys Ile Pro Pro
                   245                 250                 255
   Phe Thr Thr Phe Glu Ala Leu Ser Gly Pro Gly Arg Arg Leu Arg Val
                   260                 265                 270
   Ile Cys Asp Val Ser Cys Asp Pro Asn Ser Glu Asn Asn Pro Val Pro
           275                 280                 285
   Val Tyr Ser Ser Tyr Ser Ser Phe Glu Asn Pro Thr Val Pro Ala Ser
       290                 295                 300
   Glu His Ile Asp Gly Pro Glu Leu Arg Ile Ile Ala Ile Asp His Leu
   305                 310                 315                 320
   Pro Thr Met Val Ala Arg Glu Ser Ser Asp Glu Tyr Ser Ser Leu Leu
                   325                 330                 335
   Leu Pro Ser Leu Leu Thr Leu Asp Arg Arg Asp Thr Glu Gly Val Trp
               340                 345                 350
   Gln Arg Ala Glu Arg Ile Phe Arg Glu Lys Val Ala Glu Leu Pro
           355                 360                 365
<210> 13
   <211> 1173
   <212> DNA
   <213> Fusarium verticillioides
<220>
   <221> CDS
   <222> (1)..(1173)
   <223> Lys1
<400> 13
   atg tct gaa tat cct cac atc ctt ctt cgc gcc gag gag aag cct ctc      48
   Met Ser Glu Tyr Pro His Ile Leu Leu Arg Ala Glu Glu Lys Pro Leu
   1               5                   10                  15
   gag cac cga tct ttc tcc ccc gcg gtt atc aag aca ctc gtc gat gct      96
   Glu His Arg Ser Phe Ser Pro Ala Val Ile Lys Thr Leu Val Asp Ala
               20                  25                  30
   gga tac ccc atc tcc gtc gag cgg tca tcc acc gat ccc aaa ttt agg     144
   Gly Tyr Pro Ile Ser Val Glu Arg Ser Ser Thr Asp Pro Lys Phe Arg
           35                  40                  45
   cgt atc ttt gag gac tct gaa tat gaa gct gct ggt gct cgt ctt gtc     192
   Arg Ile Phe Glu Asp Ser Glu Tyr Glu Ala Ala Gly Ala Arg Leu Val
       50                  55                  60
   gac acg ggt gtc tgg ccc aac gct gag cct ggg aca atc ata ctc gga     240
   Asp Thr Gly Val Trp Pro Asn Ala Glu Pro Gly Thr Ile Ile Leu Gly
   65                  70                  75                  80
   ttg aag gag att ccc gag gag gat ttc cct ctg aag aat gat cac atc     288
   Leu Lys Glu Ile Pro Glu Glu Asp Phe Pro Leu Lys Asn Asp His Ile
                   85                  90                  95
   aca ttt gca cac tgc tac aaa aac cag gga ggc tgg gag aag gtt ctc     336
   Thr Phe Ala His Cys Tyr Lys Asn Gln Gly Gly Trp Glu Lys Val Leu
               100                 105                 110
   ggt cgt tgg tct cgc ggc ggg tct act ctg tat gac ttg gag ttt ttg          384
   Gly Arg Trp Ser Arg Gly Gly Ser Thr Leu Tyr Asp Leu Glu Phe Leu
           115                 120                 125
   cat gac gct gaa ggc cga cgt gtt tct gcg ttt ggt ttc cac gca ggc          432
   His Asp Ala Glu Gly Arg Arg Val Ser Ala Phe Gly Phe His Ala Gly
       130                 135                 140
   ttc gcc ggg gct gcg ctt gga ata aag aca ctt tcc cac cag ctg cag          480
   Phe Ala Gly Ala Ala Leu Gly Ile Lys Thr Leu Ser His Gln Leu Gln
   145                 150                 155                 160
   gac ccg tca tct aag ctt ccg tct gtc gag aca ttc acc gat ggc cgc          528
   Asp Pro Ser Ser Lys Leu Pro Ser Val Glu Thr Phe Thr Asp Gly Arg
                   165                 170                 175
   gga tat tac ctg aac gaa gat gag ctc gtc aac cag att cgc gag gat          576
   Gly Tyr Tyr Leu Asn Glu Asp Glu Leu Val Asn Gln Ile Arg Glu Asp
               180                 185                 190
   ctc gcc aag gct gag aag gct ctc gga cgc aag ccc act gct ctc gtc          624
   Leu Ala Lys Ala Glu Lys Ala Leu Gly Arg Lys Pro Thr Ala Leu Val
           195                 200                 205
   ctt ggt gct ctt gga cga tgt ggc aag ggt gct gtg gac ctt ttc ctc          672
   Leu Gly Ala Leu Gly Arg Cys Gly Lys Gly Ala Val Asp Leu Phe Leu
       210                 215                 220
   aag gcc ggc atg cct gat gag aac atc acc cgt tgg gac ttg aac gaa          720
   Lys Ala Gly Met Pro Asp Glu Asn Ile Thr Arg Trp Asp Leu Asn Glu
   225                 230                 235                 240
   acc aag gac cgt gat gga cct tac gag gaa att gcg aag gct gat gtc          768
   Thr Lys Asp Arg Asp Gly Pro Tyr Glu Glu Ile Ala Lys Ala Asp Val
                   245                 250                 255
   ttc ctt aac gcc atc tac ctc tcc aag ccc atc ccc cct ttc atc aac          816
   Phe Leu Asn Ala Ile Tyr Leu Ser Lys Pro Ile Pro Pro Phe Ile Asn
               260                 265                 270
   caa gaa ctc ctc gcc aag aag ggt cgc aac ctc gct gta gtc atc gac          864
   Gln Glu Leu Leu Ala Lys Lys Gly Arg Asn Leu Ala Val Val Ile Asp
           275                 280                 285
   gtt tct tgt gat acc aca aac ccc cac aac ccc atc ccc atc tac tcc          912
   Val Ser Cys Asp Thr Thr Asn Pro His Asn Pro Ile Pro Ile Tyr Ser
       290                 295                 300
   atc aac acc acc ttc gag gac ccc acc gtc ccc gtc gag atc aag gac          960
   Ile Asn Thr Thr Phe Glu Asp Pro Thr Val Pro Val Glu Ile Lys Asp
   305                 310                 315                 320
   gac cag aac aac ctc ccc ctt tcc gtc atc agc atc gac cac ctt ccc         1008
   Asp Gln Asn Asn Leu Pro Leu Ser Val Ile Ser Ile Asp His Leu Pro
                   325                 330                 335
   tcc atg ctt ccc cgc gag gct agt gag gcc ttt agt gag ggt ctc aag         1056
   Ser Met Leu Pro Arg Glu Ala Ser Glu Ala Phe Ser Glu Gly Leu Lys
               340                 345                 350
   gag tct ctg ctt aca ctc aag gat cgc aaa act tcg cgg gtg tgg gct         1104
   Glu Ser Leu Leu Thr Leu Lys Asp Arg Lys Thr Ser Arg Val Trp Ala
           355                 360                 365
   gat gct gag aag ctc ttc cat gag aag gtt gct acg ttg ccg gag gag       1152
   Asp Ala Glu Lys Leu Phe His Glu Lys Val Ala Thr Leu Pro Glu Glu
       370                 375                 380
   ttg aga acc aag aac gtt taa                                           1173
   Leu Arg Thr Lys Asn Val
   385                 390
<210> 14
   <211> 390
   <212> PRT
   <213> Fusarium verticillioides
<400> 14
   Met Ser Glu Tyr Pro His Ile Leu Leu Arg Ala Glu Glu Lys Pro Leu
   1               5                   10                  15
   Glu His Arg Ser Phe Ser Pro Ala Val Ile Lys Thr Leu Val Asp Ala
               20                  25                  30
   Gly Tyr Pro Ile Ser Val Glu Arg Ser Ser Thr Asp Pro Lys Phe Arg
           35                  40                  45
   Arg Ile Phe Glu Asp Ser Glu Tyr Glu Ala Ala Gly Ala Arg Leu Val
       50                  55                  60
   Asp Thr Gly Val Trp Pro Asn Ala Glu Pro Gly Thr Ile Ile Leu Gly
   65                  70                  75                  80
   Leu Lys Glu Ile Pro Glu Glu Asp Phe Pro Leu Lys Asn Asp His Ile
                   85                  90                  95
   Thr Phe Ala His Cys Tyr Lys Asn Gln Gly Gly Trp Glu Lys Val Leu
               100                 105                 110
   Gly Arg Trp Ser Arg Gly Gly Ser Thr Leu Tyr Asp Leu Glu Phe Leu
           115                 120                 125
   His Asp Ala Glu Gly Arg Arg Val Ser Ala Phe Gly Phe His Ala Gly
       130                 135                 140
   Phe Ala Gly Ala Ala Leu Gly Ile Lys Thr Leu Ser His Gln Leu Gln
   145                 150                 155                 160
   Asp Pro Ser Ser Lys Leu Pro Ser Val Glu Thr Phe Thr Asp Gly Arg
                   165                 170                 175
   Gly Tyr Tyr Leu Asn Glu Asp Glu Leu Val Asn Gln Ile Arg Glu Asp
               180                 185                 190
   Leu Ala Lys Ala Glu Lys Ala Leu Gly Arg Lys Pro Thr Ala Leu Val
           195                 200                 205
   Leu Gly Ala Leu Gly Arg Cys Gly Lys Gly Ala Val Asp Leu Phe Leu
       210                 215                 220
   Lys Ala Gly Met Pro Asp Glu Asn Ile Thr Arg Trp Asp Leu Asn Glu
   225                 230                 235                 240
   Thr Lys Asp Arg Asp Gly Pro Tyr Glu Glu Ile Ala Lys Ala Asp Val
                   245                 250                 255
   Phe Leu Asn Ala Ile Tyr Leu Ser Lys Pro Ile Pro Pro Phe Ile Asn
               260                 265                 270
   Gln Glu Leu Leu Ala Lys Lys Gly Arg Asn Leu Ala Val Val Ile Asp
           275                 280                 285
   Val Ser Cys Asp Thr Thr Asn Pro His Asn Pro Ile Pro Ile Tyr Ser
       290                 295                 300
   Ile Asn Thr Thr Phe Glu Asp Pro Thr Val Pro Val Glu Ile Lys Asp
   305                 310                 315                 320
   Asp Gln Asn Asn Leu Pro Leu Ser Val Ile Ser Ile Asp His Leu Pro
                   325                 330                 335
   Ser Met Leu Pro Arg Glu Ala Ser Glu Ala Phe Ser Glu Gly Leu Lys
               340                 345                 350
   Glu Ser Leu Leu Thr Leu Lys Asp Arg Lys Thr Ser Arg Val Trp Ala
           355                 360                 365
   Asp Ala Glu Lys Leu Phe His Glu Lys Val Ala Thr Leu Pro Glu Glu
       370                 375                 380
   Leu Arg Thr Lys Asn Val
   385                 390
<210> 15
   <211> 1223
   <212> DNA
   <213> Mycosphaerella fijiensis
<220>
   <221> CDS
   <222> (3)..(1223)
   <223> Lys1
<400> 15
   ga agg tca aca ctt aaa aaa gac tca tat tcc gcg tcg act ata atc 47
   Arg Ser Thr Leu Lys Lys Asp Ser Tyr Ser Ala Ser Thr Ile Ile
   1               5                   10                  15
   gta caa gat cag tgt ctc aca atg tcg cct aca gtc ctt cat ttc aga        95
   Val Gln Asp Gln Cys Leu Thr Met Ser Pro Thr Val Leu His Phe Arg
               20                  25                  30
   gca gag acc aag cca tta gag cat cgc tcg gca gtc aca ccg aca atc       143
   Ala Glu Thr Lys Pro Leu Glu His Arg Ser Ala Val Thr Pro Thr Ile
           35                  40                  45
   gca aag aaa ctt gtc gaa gct ggc tat gaa gtc cac gtc gaa cga tcg       191
   Ala Lys Lys Leu Val Glu Ala Gly Tyr Glu Val His Val Glu Arg Ser
       50                  55                  60
   cag ctc agc ata ttc gca gac tcc gag tat gaa ggc act ggt gct aag       239
   Gln Leu Ser Ile Phe Ala Asp Ser Glu Tyr Glu Gly Thr Gly Ala Lys
   65                  70                  75
   cta gtg cct act gga tcg tgg aca gag gca ccg aag gag cac att gtc       287
   Leu Val Pro Thr Gly Ser Trp Thr Glu Ala Pro Lys Glu His Ile Val
   80                  85                  90                  95
   att ggc ttg aag gaa ctc ccc gag gag gac ttt cct ttg aag cat gtt       335
   Ile Gly Leu Lys Glu Leu Pro Glu Glu Asp Phe Pro Leu Lys His Val
               100                 105                 110
   cat gtg caa ttt gct cac tgc tac aaa gga caa ggt ggc tgg gac aag       383
   His Val Gln Phe Ala His Cys Tyr Lys Gly Gln Gly Gly Trp Asp Lys
           115                 120                 125
   gtt ctc agt aga ttt cca aac ggc aga ggc aca tta ctt gac ctt gaa       431
   Val Leu Ser Arg Phe Pro Asn Gly Arg Gly Thr Leu Leu Asp Leu Glu
       130                 135                 140
   ttc ttg gaa gat gag caa gga cga aga gtc gct gca ttt ggc tac cat       479
   Phe Leu Glu Asp Glu Gln Gly Arg Arg Val Ala Ala Phe Gly Tyr His
   145                 150                 155
   gcc gga ttt gct ggt gcc gca ctt gct ctc atg gcc tgg tct cac cag       527
   Ala Gly Phe Ala Gly Ala Ala Leu Ala Leu Met Ala Trp Ser His Gln
   160                 165                 170                 175
   ctc gtg cac ggc aag gac agc cct ctt ccg gga gtg aca cca tac gaa       575
   Leu Val His Gly Lys Asp Ser Pro Leu Pro Gly Val Thr Pro Tyr Glu
               180                 185                 190
   aac gaa ggt ctc ctt att tcc gat gtc aag aag gcc atc gaa gct ggc       623
   Asn Glu Gly Leu Leu Ile Ser Asp Val Lys Lys Ala Ile Glu Ala Gly
           195                 200                 205
   aag gcc aag ggc ggt tgc ctc cca aga gtg ctc gtt att ggc gga ctc       671
   Lys Ala Lys Gly Gly Cys Leu Pro Arg Val Leu Val Ile Gly Gly Leu
       210                 215                 220
   ggc aga tgc ggt cgt ggt gcc gtt gac ctc tgt gtc aag gct ggc gtg       719
   Gly Arg Cys Gly Arg Gly Ala Val Asp Leu Cys Val Lys Ala Gly Val
   225                 230                 235
   gaa gac att cta aag tgg gat ctt cca gaa aca agt gcc aaa cca gga       767
   Glu Asp Ile Leu Lys Trp Asp Leu Pro Glu Thr Ser Ala Lys Pro Gly
   240                 245                 250                 255
   cca tac cag gag atc atc gag tct gac gtg ttt gtc aac tgc atc tac       815
   Pro Tyr Gln Glu Ile Ile Glu Ser Asp Val Phe Val Asn Cys Ile Tyr
                   260                 265                 270
   ttg tcg gcc aag atc ccg ccc ttc atc gac cag gca tct ctc gca tcg        863
   Leu Ser Ala Lys Ile Pro Pro Phe Ile Asp Gln Ala Ser Leu Ala Ser
               275                 280                 285
   cca aac cgc aaa ctg tct gtg gtc tgt gat gta tcc tgc gat acc acg        911
   Pro Asn Arg Lys Leu Ser Val Val Cys Asp Val Ser Cys Asp Thr Thr
           290                 295                 300
   aac cca aac aat cct atc cca atc tac gac atc aac acg act ttc gac        959
   Asn Pro Asn Asn Pro Ile Pro Ile Tyr Asp Ile Asn Thr Thr Phe Asp
       305                 310                 315
   aag cca acg gtg cct gtc aag ctc tcc gct gag gcc aat gat ctt ccg       1007
   Lys Pro Thr Val Pro Val Lys Leu Ser Ala Glu Ala Asn Asp Leu Pro
   320                 325                 330                 335
   ttg agc gta atc agt atc gac cat ctc cca tcc tta ctg ccg cga gaa       1055
   Leu Ser Val Ile Ser Ile Asp His Leu Pro Ser Leu Leu Pro Arg Glu
                   340                 345                 350
   gct tcc gaa gct ttc agc tcc gcg ctg ctg ccc agc ttg ctt caa ttg       1103
   Ala Ser Glu Ala Phe Ser Ser Ala Leu Leu Pro Ser Leu Leu Gln Leu
               355                 360                 365
   aac gac tgg aag aac gca cgt gtt tgg caa caa gcc gag aag ctg ttc       1151
   Asn Asp Trp Lys Asn Ala Arg Val Trp Gln Gln Ala Glu Lys Leu Phe
           370                 375                 380
   aag gac aaa tgc gcc act ctc cca gaa ggc gca atc gat agc cat gcc       1199
   Lys Asp Lys Cys Ala Thr Leu Pro Glu Gly Ala Ile Asp Ser His Ala
       385                 390                 395
   gag ctt ctt gct tcg caa tct tga                                       1223
   Glu Leu Leu Ala Ser Gln Ser
   400                 405
<210> 16
   <211> 406
   <212> PRT
   <213> Mycosphaerella fijiensis
<400> 16
   Arg Ser Thr Leu Lys Lys Asp Ser Tyr Ser Ala Ser Thr Ile Ile Val
   1               5                   10                  15
   Gln Asp Gln Cys Leu Thr Met Ser Pro Thr Val Leu His Phe Arg Ala
               20                  25                  30
   Glu Thr Lys Pro Leu Glu His Arg Ser Ala Val Thr Pro Thr Ile Ala
           35                  40                  45
   Lys Lys Leu Val Glu Ala Gly Tyr Glu Val His Val Glu Arg Ser Gln
       50                  55                  60
   Leu Ser Ile Phe Ala Asp Ser Glu Tyr Glu Gly Thr Gly Ala Lys Leu
   65                  70                  75                  80
   Val Pro Thr Gly Ser Trp Thr Glu Ala Pro Lys Glu His Ile Val Ile
                   85                  90                  95
   Gly Leu Lys Glu Leu Pro Glu Glu Asp Phe Pro Leu Lys His Val His
               100                 105                 110
   Val Gln Phe Ala His Cys Tyr Lys Gly Gln Gly Gly Trp Asp Lys Val
           115                 120                 125
   Leu Ser Arg Phe Pro Asn Gly Arg Gly Thr Leu Leu Asp Leu Glu Phe
       130                 135                 140
   Leu Glu Asp Glu Gln Gly Arg Arg Val Ala Ala Phe Gly Tyr His Ala
   145                 150                 155                 160
   Gly Phe Ala Gly Ala Ala Leu Ala Leu Met Ala Trp Ser His Gln Leu
                   165                 170                 175
   Val His Gly Lys Asp Ser Pro Leu Pro Gly Val Thr Pro Tyr Glu Asn
               180                 185                 190
   Glu Gly Leu Leu Ile Ser Asp Val Lys Lys Ala Ile Glu Ala Gly Lys
           195                 200                 205
   Ala Lys Gly Gly Cys Leu Pro Arg Val Leu Val Ile Gly Gly Leu Gly
       210                 215                 220
   Arg Cys Gly Arg Gly Ala Val Asp Leu Cys Val Lys Ala Gly Val Glu
   225                 230                 235                 240
   Asp Ile Leu Lys Trp Asp Leu Pro Glu Thr Ser Ala Lys Pro Gly Pro
                   245                 250                 255
   Tyr Gln Glu Ile Ile Glu Ser Asp Val Phe Val Asn Cys Ile Tyr Leu
               260                 265                 270
   Ser Ala Lys Ile Pro Pro Phe Ile Asp Gln Ala Ser Leu Ala Ser Pro
           275                 280                 285
   Asn Arg Lys Leu Ser Val Val Cys Asp Val Ser Cys Asp Thr Thr Asn
       290                 295                 300
   Pro Asn Asn Pro Ile Pro Ile Tyr Asp Ile Asn Thr Thr Phe Asp Lys
   305                 310                 315                 320
   Pro Thr Val Pro Val Lys Leu Ser Ala Glu Ala Asn Asp Leu Pro Leu
                   325                 330                 335
   Ser Val Ile Ser Ile Asp His Leu Pro Ser Leu Leu Pro Arg Glu Ala
               340                 345                 350
   Ser Glu Ala Phe Ser Ser Ala Leu Leu Pro Ser Leu Leu Gln Leu Asn
           355                 360                 365
   Asp Trp Lys Asn Ala Arg Val Trp Gln Gln Ala Glu Lys Leu Phe Lys
       370                 375                 380
   Asp Lys Cys Ala Thr Leu Pro Glu Gly Ala Ile Asp Ser His Ala Glu
   385                 390                 395                 400
   Leu Leu Ala Ser Gln Ser
                   405
<210> 17
   <211> 1098
   <212> DNA
   <213> Magnaporthe grisea
<220>
   <221> CDS
   <222> (1)..(1098)
   <223> Lys1
<400> 17
   atg tcg tcc att ctg cac ttg cgg tca gag acc aag cca ttg gag cac        48
   Met Ser Ser Ile Leu His Leu Arg Ser Glu Thr Lys Pro Leu Glu His
   1               5                   10                  15
   cgg tcc gcc cta act ccc act acc acc aag gcc ttg att gag gcc ggt        96
   Arg Ser Ala Leu Thr Pro Thr Thr Thr Lys Ala Leu Ile Glu Ala Gly
               20                  25                  30
   tac acc gtc aat gtc gag cgc agt ccc gtc cgc atc ttc gac gac gcc       144
   Tyr Thr Val Asn Val Glu Arg Ser Pro Val Arg Ile Phe Asp Asp Ala
           35                  40                  45
   gag ttc gag gct gtt ggc gca acc ctc gtc ccc gag ggc agc tgg gag       192
   Glu Phe Glu Ala Val Gly Ala Thr Leu Val Pro Glu Gly Ser Trp Glu
       50                  55                  60
   cag att ccc aag gac cac atc gtc att gga ctg aag gag ttg gag gag       240
   Gln Ile Pro Lys Asp His Ile Val Ile Gly Leu Lys Glu Leu Glu Glu
   65                  70                  75                  80
   aag gac ttc cct ttg aag cac aca cac atc cag ttc gcc cac tgc tac       288
   Lys Asp Phe Pro Leu Lys His Thr His Ile Gln Phe Ala His Cys Tyr
                   85                  90                  95
   aag aac caa gga ggc tgg gac aag gtc ctc cgg agg tat cac gat ggc       336
   Lys Asn Gln Gly Gly Trp Asp Lys Val Leu Arg Arg Tyr His Asp Gly
               100                 105                 110
   gag gga atg ctc ctc gac att gag ttc ctc gag aag gac ggc cgt cgt       384
   Glu Gly Met Leu Leu Asp Ile Glu Phe Leu Glu Lys Asp Gly Arg Arg
           115                 120                 125
   gtt gct gct ttc ggc tac tgg gcc ggc ttt gcc ggt gcc gcg ctg gcc       432
   Val Ala Ala Phe Gly Tyr Trp Ala Gly Phe Ala Gly Ala Ala Leu Ala
       130                 135                 140
   ctt cag aac tgg gcc tgg cag ctg acc aac agc ggc gag ccc ctg ccg       480
   Leu Gln Asn Trp Ala Trp Gln Leu Thr Asn Ser Gly Glu Pro Leu Pro
   145                 150                 155                 160
   agc gtc gag agc cgc cct aac gag gct gcc ttg gtc ggc gac atc aag       528
   Ser Val Glu Ser Arg Pro Asn Glu Ala Ala Leu Val Gly Asp Ile Lys
                   165                 170                 175
   gag gct ctt gct ggg ggc aag gaa aag gcc ggc agg ctg ccc cgc gtc       576
   Glu Ala Leu Ala Gly Gly Lys Glu Lys Ala Gly Arg Leu Pro Arg Val
               180                 185                 190
   atc gtc atc ggt gcc ctg ggc cgc tgc ggt cgt ggt gcc gtc gac atg       624
   Ile Val Ile Gly Ala Leu Gly Arg Cys Gly Arg Gly Ala Val Asp Met
           195                 200                 205
   tgc aag aag gcc ggc ata ccg gat gag aac atc ctc aag tgg gat atg       672
   Cys Lys Lys Ala Gly Ile Pro Asp Glu Asn Ile Leu Lys Trp Asp Met
       210                 215                 220
   gag gag acg gcc ccc ggt gga ccg ttc aag gag att gtc gag agt gac       720
   Glu Glu Thr Ala Pro Gly Gly Pro Phe Lys Glu Ile Val Glu Ser Asp
   225                 230                 235                 240
   atc ttt gtc aac tgc atc tac ctg aac aag cag atc ccg ccg ttt gtg       768
   Ile Phe Val Asn Cys Ile Tyr Leu Asn Lys Gln Ile Pro Pro Phe Val
                   245                 250                 255
   acc atg gag tcg ctc aac acg cca gag cgc aag ctc tcc gtc atc aac       816
   Thr Met Glu Ser Leu Asn Thr Pro Glu Arg Lys Leu Ser Val Ile Asn
               260                 265                 270
   gac tgc tcg gct gac acg acc aac ccc ttt aac cct gtt ccc gtg tac       864
   Asp Cys Ser Ala Asp Thr Thr Asn Pro Phe Asn Pro Val Pro Val Tyr
           275                 280                 285
   act gtg gcc acg act ttc gac aag ccc act gtg ccg gtc gat ggc ttg       912
   Thr Val Ala Thr Thr Phe Asp Lys Pro Thr Val Pro Val Asp Gly Leu
       290                 295                 300
   agc cat ggc ccg ccc ctg agc gtt atc agc atc gac cac ctg ccg agt       960
   Ser His Gly Pro Pro Leu Ser Val Ile Ser Ile Asp His Leu Pro Ser
   305                 310                 315                 320
   ctg ctc ccg cgg gag gct tcg gag tcg ttc agc aac gac ttg ttg cca      1008
   Leu Leu Pro Arg Glu Ala Ser Glu Ser Phe Ser Asn Asp Leu Leu Pro
                   325                 330                 335
   tat ctc ctg aag ctg aag gac tgg aag agt gac ccg gtg tgg gct ggc      1056
   Tyr Leu Leu Lys Leu Lys Asp Trp Lys Ser Asp Pro Val Trp Ala Gly
               340                 345                 350
   gcc gag aag ctg ttc cac gag aag gtc aag acc ctg ccg tag              1098
   Ala Glu Lys Leu Phe His Glu Lys Val Lys Thr Leu Pro
           355                 360                 365
<210> 18
   <211> 365
   <212> PRT
   <213> Magnaporthe grisea
<400> 18
   Met Ser Ser Ile Leu His Leu Arg Ser Glu Thr Lys Pro Leu Glu His
   1               5                   10                  15
   Arg Ser Ala Leu Thr Pro Thr Thr Thr Lys Ala Leu Ile Glu Ala Gly
               20                  25                  30
   Tyr Thr Val Asn Val Glu Arg Ser Pro Val Arg Ile Phe Asp Asp Ala
           35                  40                  45
   Glu Phe Glu Ala Val Gly Ala Thr Leu Val Pro Glu Gly Ser Trp Glu
       50                  55                  60
   Gln Ile Pro Lys Asp His Ile Val Ile Gly Leu Lys Glu Leu Glu Glu
   65                  70                  75                  80
   Lys Asp Phe Pro Leu Lys His Thr His Ile Gln Phe Ala His Cys Tyr
                   85                  90                  95
   Lys Asn Gln Gly Gly Trp Asp Lys Val Leu Arg Arg Tyr His Asp Gly
               100                 105                 110
   Glu Gly Met Leu Leu Asp Ile Glu Phe Leu Glu Lys Asp Gly Arg Arg
           115                 120                 125
   Val Ala Ala Phe Gly Tyr Trp Ala Gly Phe Ala Gly Ala Ala Leu Ala
       130                 135                 140
   Leu Gln Asn Trp Ala Trp Gln Leu Thr Asn Ser Gly Glu Pro Leu Pro
   145                 150                 155                 160
   Ser Val Glu Ser Arg Pro Asn Glu Ala Ala Leu Val Gly Asp Ile Lys
                   165                 170                 175
   Glu Ala Leu Ala Gly Gly Lys Glu Lys Ala Gly Arg Leu Pro Arg Val
               180                 185                 190
   Ile Val Ile Gly Ala Leu Gly Arg Cys Gly Arg Gly Ala Val Asp Met
           195                 200                 205
   Cys Lys Lys Ala Gly Ile Pro Asp Glu Asn Ile Leu Lys Trp Asp Met
       210                 215                 220
   Glu Glu Thr Ala Pro Gly Gly Pro Phe Lys Glu Ile Val Glu Ser Asp
   225                 230                 235                 240
   Ile Phe Val Asn Cys Ile Tyr Leu Asn Lys Gln Ile Pro Pro Phe Val
                   245                 250                 255
   Thr Met Glu Ser Leu Asn Thr Pro Glu Arg Lys Leu Ser Val Ile Asn
               260                 265                 270
   Asp Cys Ser Ala Asp Thr Thr Asn Pro Phe Asn Pro Val Pro Val Tyr
           275                 280                 285
   Thr Val Ala Thr Thr Phe Asp Lys Pro Thr Val Pro Val Asp Gly Leu
       290                 295                 300
   Ser His Gly Pro Pro Leu Ser Val Ile Ser Ile Asp His Leu Pro Ser
   305                 310                 315                 320
   Leu Leu Pro Arg Glu Ala Ser Glu Ser Phe Ser Asn Asp Leu Leu Pro
                   325                 330                 335
   Tyr Leu Leu Lys Leu Lys Asp Trp Lys Ser Asp Pro Val Trp Ala Gly
               340                 345                 350
   Ala Glu Lys Leu Phe His Glu Lys Val Lys Thr Leu Pro
           355                 360                 365
<210> 19
   <211> 453
   <212> DNA
   <213> Moniliophthora perniciosa
<220>
   <221> CDS
   <222> (1)..(453)
   <223> Lys1
<400> 19
   atg aaa ttc aca ctc cct cgt ctt cga cca ctc acc att gga atc cgc        48
   Met Lys Phe Thr Leu Pro Arg Leu Arg Pro Leu Thr Ile Gly Ile Arg
   1               5                   10                  15
   cgg gaa gac cct act cga att tgg gaa cga cgg gct cct cta acc ccc        96
   Arg Glu Asp Pro Thr Arg Ile Trp Glu Arg Arg Ala Pro Leu Thr Pro
               20                  25                  30
   gac tcc gtt tac gag ctt gtc aag gac aag gcg gta caa gtt cat gtc       144
   Asp Ser Val Tyr Glu Leu Val Lys Asp Lys Ala Val Gln Val His Val
           35                  40                  45
   gaa ggc tgc gac agg agg ata ttc aag gac gag gaa tac ata aag gcc       192
   Glu Gly Cys Asp Arg Arg Ile Phe Lys Asp Glu Glu Tyr Ile Lys Ala
       50                  55                  60
   ggt gca aca atc cga ccc aac ctg aac gat gca cat gtt gta atg ggc       240
   Gly Ala Thr Ile Arg Pro Asn Leu Asn Asp Ala His Val Val Met Gly
   65                  70                  75                  80
   att aaa gag ccc cca ttg gac aga tta ttg ctt gat cct ctt cct cta       288
   Ile Lys Glu Pro Pro Leu Asp Arg Leu Leu Leu Asp Pro Leu Pro Leu
                   85                  90                  95
   tcc aac acc acg tcg aaa cat gag cga aca tac atg aag ttt tct cat       336
   Ser Asn Thr Thr Ser Lys His Glu Arg Thr Tyr Met Lys Phe Ser His
               100                 105                 110
   act tgg aaa ggt caa gcc tac aat atg cca tta ctt tct gca ttc tta       384
   Thr Trp Lys Gly Gln Ala Tyr Asn Met Pro Leu Leu Ser Ala Phe Leu
           115                 120                 125
   aac att cac cca ttc cat ggc gct tat aat gac cca ctg gcc cac act       432
   Asn Ile His Pro Phe His Gly Ala Tyr Asn Asp Pro Leu Ala His Thr
       130                 135                 140
   aat tgg ata atg aac tcc tga                                           453
   Asn Trp Ile Met Asn Ser
   145                 150
<210> 20
   <211> 150
   <212> PRT
   <213> Moniliophthora perniciosa
<400> 20
   Met Lys Phe Thr Leu Pro Arg Leu Arg Pro Leu Thr Ile Gly Ile Arg
   1               5                   10                  15
   Arg Glu Asp Pro Thr Arg Ile Trp Glu Arg Arg Ala Pro Leu Thr Pro
               20                  25                  30
   Asp Ser Val Tyr Glu Leu Val Lys Asp Lys Ala Val Gln Val His Val
           35                  40                  45
   Glu Gly Cys Asp Arg Arg Ile Phe Lys Asp Glu Glu Tyr Ile Lys Ala
       50                  55                  60
   Gly Ala Thr Ile Arg Pro Asn Leu Asn Asp Ala His Val Val Met Gly
   65                  70                  75                  80
   Ile Lys Glu Pro Pro Leu Asp Arg Leu Leu Leu Asp Pro Leu Pro Leu
                   85                  90                  95
   Ser Asn Thr Thr Ser Lys His Glu Arg Thr Tyr Met Lys Phe Ser His
               100                 105                 110
   Thr Trp Lys Gly Gln Ala Tyr Asn Met Pro Leu Leu Ser Ala Phe Leu
           115                 120                 125
   Asn Ile His Pro Phe His Gly Ala Tyr Asn Asp Pro Leu Ala His Thr
       130                 135                 140
   Asn Trp Ile Met Asn Ser
   145                 150
<210> 21
   <211> 1116
   <212> DNA
   <213> Puccinia graminis
<220>
   <221> CDS
   <222> (1)..(1116)
   <223> Lys1
<400> 21
   atg tca ggc gat caa cca ctc cta tgg ctg cga tgc gag acc aaa ccg        48
   Met Ser Gly Asp Gln Pro Leu Leu Trp Leu Arg Cys Glu Thr Lys Pro
   1               5                   10                  15
   ttc gag cat cgc tca gca tta act ccc ttg acc gcc aaa aaa ctc atc        96
   Phe Glu His Arg Ser Ala Leu Thr Pro Leu Thr Ala Lys Lys Leu Ile
               20                  25                  30
   gat gct gga ttc aag ctg gta gta gaa cgg gat cct caa cgg ttc ttc       144
   Asp Ala Gly Phe Lys Leu Val Val Glu Arg Asp Pro Gln Arg Phe Phe
           35                  40                  45
   gct gat gac gaa ttt gca aaa gtc gga tgt gaa ctg gtc gaa cac aac       192
   Ala Asp Asp Glu Phe Ala Lys Val Gly Cys Glu Leu Val Glu His Asn
       50                  55                  60
   tcg tgg ccc aag gct ccc tcc aat gca att atc atc gga ttg aag gag       240
   Ser Trp Pro Lys Ala Pro Ser Asn Ala Ile Ile Ile Gly Leu Lys Glu
   65                  70                  75                  80
   ttg cct ccg aat gat gat tcg ccc ctc atc cat acc cat gtc atg ttc       288
   Leu Pro Pro Asn Asp Asp Ser Pro Leu Ile His Thr His Val Met Phe
                   85                  90                  95
   ggc cat tgt tac aag caa caa gca ggc tac cag gac atc cta agt cga       336
   Gly His Cys Tyr Lys Gln Gln Ala Gly Tyr Gln Asp Ile Leu Ser Arg
               100                 105                 110
   ttc aaa cga ggc ggg gga act ttg ctc gac atg gaa ttc ttg cag gac       384
   Phe Lys Arg Gly Gly Gly Thr Leu Leu Asp Met Glu Phe Leu Gln Asp
           115                 120                 125
   gag cac acc aaa cgc aga gtg gct gcc ttt ggc ttc cac gcc gga ttc       432
   Glu His Thr Lys Arg Arg Val Ala Ala Phe Gly Phe His Ala Gly Phe
       130                 135                 140
   aat ggc tct gct gtc ggt cta ctt gcg ctt ggg tcg atg tta tcc ggg       480
   Asn Gly Ser Ala Val Gly Leu Leu Ala Leu Gly Ser Met Leu Ser Gly
   145                 150                 155                 160
   gag gga agt ttg aaa gga ttg aag cct ttc aaa gat gaa gac gaa ttg       528
   Glu Gly Ser Leu Lys Gly Leu Lys Pro Phe Lys Asp Glu Asp Glu Leu
                   165                 170                 175
   att act cga ggg aag aaa gaa ttc gat cga gtg gtc gcc aag cta ggt       576
   Ile Thr Arg Gly Lys Lys Glu Phe Asp Arg Val Val Ala Lys Leu Gly
               180                 185                 190
   cga cat cct aag gcc cta gtg att ggt tca ttg gga cgc tgt ggc tcc       624
   Arg His Pro Lys Ala Leu Val Ile Gly Ser Leu Gly Arg Cys Gly Ser
           195                 200                 205
   gga gct gtt aca ttt ttc aag aaa att gga ttg aac aag gat gac gtc       672
   Gly Ala Val Thr Phe Phe Lys Lys Ile Gly Leu Asn Lys Asp Asp Val
       210                 215                 220
   gtt gaa tgg gac atg gca gag act gcc aaa ggt ggc cca ttc caa gaa          720
   Val Glu Trp Asp Met Ala Glu Thr Ala Lys Gly Gly Pro Phe Gln Glu
   225                 230                 235                 240
   att ctt gaa gcc gat atc ttc atc aat tgc atc tac ttg agt agc aaa          768
   Ile Leu Glu Ala Asp Ile Phe Ile Asn Cys Ile Tyr Leu Ser Ser Lys
                   245                 250                 255
   atc ccc agc ttt gtc acc cgg gag aca ata gcc gca gca gga gat tct          816
   Ile Pro Ser Phe Val Thr Arg Glu Thr Ile Ala Ala Ala Gly Asp Ser
               260                 265                 270
   cgc cag ctg agg gtt gta gtc gat gtg tcc tgt gat acc acc aat ccg          864
   Arg Gln Leu Arg Val Val Val Asp Val Ser Cys Asp Thr Thr Asn Pro
           275                 280                 285
   aac aat cct att ccg ata tat gac gtc aac act aca ttc gat tct cca          912
   Asn Asn Pro Ile Pro Ile Tyr Asp Val Asn Thr Thr Phe Asp Ser Pro
       290                 295                 300
   acc gtg cct gtc cag cta gat gct gga ctg cca agc tta gaa gtt tgc          960
   Thr Val Pro Val Gln Leu Asp Ala Gly Leu Pro Ser Leu Glu Val Cys
   305                 310                 315                 320
   tcg ata gat cat ctg cca aca ctt ctg ccc aga gaa gcc tcc gaa caa         1008
   Ser Ile Asp His Leu Pro Thr Leu Leu Pro Arg Glu Ala Ser Glu Gln
                   325                 330                 335
   ttt tcg aac gat ctc ctt cct acg ctt ttg caa tta aaa acg ttg gat         1056
   Phe Ser Asn Asp Leu Leu Pro Thr Leu Leu Gln Leu Lys Thr Leu Asp
               340                 345                 350
   caa tct aaa gtt tgg act gaa gca agg gat ttg ttt cac aag atg gtc         1104
   Gln Ser Lys Val Trp Thr Glu Ala Arg Asp Leu Phe His Lys Met Val
           355                 360                 365
   aat tcc atc tag                                                         1116
   Asn Ser Ile
       370
<210> 22
   <211> 371
   <212> PRT
   <213> Puccinia graminis
<400> 22
   Met Ser Gly Asp Gln Pro Leu Leu Trp Leu Arg Cys Glu Thr Lys Pro
   1               5                   10                  15
   Phe Glu His Arg Ser Ala Leu Thr Pro Leu Thr Ala Lys Lys Leu Ile
               20                  25                  30
   Asp Ala Gly Phe Lys Leu Val Val Glu Arg Asp Pro Gln Arg Phe Phe
           35                  40                  45
   Ala Asp Asp Glu Phe Ala Lys Val Gly Cys Glu Leu Val Glu His Asn
       50                  55                  60
   Ser Trp Pro Lys Ala Pro Ser Asn Ala Ile Ile Ile Gly Leu Lys Glu
   65                  70                  75                  80
   Leu Pro Pro Asn Asp Asp Ser Pro Leu Ile His Thr His Val Met Phe
                   85                  90                  95
   Gly His Cys Tyr Lys Gln Gln Ala Gly Tyr Gln Asp Ile Leu Ser Arg
               100                 105                 110
   Phe Lys Arg Gly Gly Gly Thr Leu Leu Asp Met Glu Phe Leu Gln Asp
           115                 120                 125
   Glu His Thr Lys Arg Arg Val Ala Ala Phe Gly Phe His Ala Gly Phe
       130                 135                 140
   Asn Gly Ser Ala Val Gly Leu Leu Ala Leu Gly Ser Met Leu Ser Gly
   145                 150                 155                 160
   Glu Gly Ser Leu Lys Gly Leu Lys Pro Phe Lys Asp Glu Asp Glu Leu
                   165                 170                 175
   Ile Thr Arg Gly Lys Lys Glu Phe Asp Arg Val Val Ala Lys Leu Gly
               180                 185                 190
   Arg His Pro Lys Ala Leu Val Ile Gly Ser Leu Gly Arg Cys Gly Ser
           195                 200                 205
   Gly Ala Val Thr Phe Phe Lys Lys Ile Gly Leu Asn Lys Asp Asp Val
       210                 215                 220
   Val Glu Trp Asp Met Ala Glu Thr Ala Lys Gly Gly Pro Phe Gln Glu
   225                 230                 235                 240
   Ile Leu Glu Ala Asp Ile Phe Ile Asn Cys Ile Tyr Leu Ser Ser Lys
                   245                 250                 255
   Ile Pro Ser Phe Val Thr Arg Glu Thr Ile Ala Ala Ala Gly Asp Ser
               260                 265                 270
   Arg Gln Leu Arg Val Val Val Asp Val Ser Cys Asp Thr Thr Asn Pro
           275                 280                 285
   Asn Asn Pro Ile Pro Ile Tyr Asp Val Asn Thr Thr Phe Asp Ser Pro
       290                 295                 300
   Thr Val Pro Val Gln Leu Asp Ala Gly Leu Pro Ser Leu Glu Val Cys
   305                 310                 315                 320
   Ser Ile Asp His Leu Pro Thr Leu Leu Pro Arg Glu Ala Ser Glu Gln
                   325                 330                 335
   Phe Ser Asn Asp Leu Leu Pro Thr Leu Leu Gln Leu Lys Thr Leu Asp
               340                 345                 350
   Gln Ser Lys Val Trp Thr Glu Ala Arg Asp Leu Phe His Lys Met Val
           355                 360                 365
   Asn Ser Ile
       370
<210> 23
   <211> 3045
   <212> DNA
   <213> Phytophthora infestans
<220>
   <221> CDS
   <222> (1)..(3045)
   <223> Lys1
<400> 23
   atg tcg ggc aag tgt gta ggc atc gtc cgc gag gta tac aac aaa tgg      48
   Met Ser Gly Lys Cys Val Gly Ile Val Arg Glu Val Tyr Asn Lys Trp
   1               5                   10                  15
   gag cgt cga gcg ccg ctc aca ccc gca cac gtt aag gag cta gtg gcg      96
   Glu Arg Arg Ala Pro Leu Thr Pro Ala His Val Lys Glu Leu Val Ala
               20                  25                  30
   cgt ggc gtg cag gtg ctg gtt cag ccg tcc acg gca cgc gtc ttc tca     144
   Arg Gly Val Gln Val Leu Val Gln Pro Ser Thr Ala Arg Val Phe Ser
           35                  40                  45
   gac gac cag tat gtt cgc gcc gga gcc aca ctg tcc gag aat ttg gcg     192
   Asp Asp Gln Tyr Val Arg Ala Gly Ala Thr Leu Ser Glu Asn Leu Ala
       50                  55                  60
   ccg gcg aat gtc att gtg ggg gtc aag cag gtg ccc gag ccg gca ctg     240
   Pro Ala Asn Val Ile Val Gly Val Lys Gln Val Pro Glu Pro Ala Leu
   65                  70                  75                  80
   ctg gcg gac aag acg tac ctc ttc ttc agt cat acc atc aaa gcg cag     288
   Leu Ala Asp Lys Thr Tyr Leu Phe Phe Ser His Thr Ile Lys Ala Gln
                   85                  90                  95
   ccc gag aac atg gcg ctg ctg gac gcc gtg cta cag cgt cgc gtc acg     336
   Pro Glu Asn Met Ala Leu Leu Asp Ala Val Leu Gln Arg Arg Val Thr
               100                 105                 110
   cta atc gac tac gaa tgt atc aca gag gag agt ggt aag cgt ctc att     384
   Leu Ile Asp Tyr Glu Cys Ile Thr Glu Glu Ser Gly Lys Arg Leu Ile
           115                 120                 125
   gcc ttc ggc gga aat gcc ggt cgt gca gga atg att gcg ggc ttc cga     432
   Ala Phe Gly Gly Asn Ala Gly Arg Ala Gly Met Ile Ala Gly Phe Arg
       130                 135                 140
   ggt ctc gga gaa cgt ctc atc aac atg gga atc tcc tcg ccc ttc gtc     480
   Gly Leu Gly Glu Arg Leu Ile Asn Met Gly Ile Ser Ser Pro Phe Val
   145                 150                 155                 160
   aac atc gcg tcc tcc tac atg tac gca gac tta gag cac gct aaa gac       528
   Asn Ile Ala Ser Ser Tyr Met Tyr Ala Asp Leu Glu His Ala Lys Asp
                   165                 170                 175
   gca gtg gaa gct gca ggt aga agg att cga aca gat ggt ctg ccg ggt       576
   Ala Val Glu Ala Ala Gly Arg Arg Ile Arg Thr Asp Gly Leu Pro Gly
               180                 185                 190
   gac ttg gcg cca atg aca ttc gcc ttt act ggc aat ggc aat gtg tct       624
   Asp Leu Ala Pro Met Thr Phe Ala Phe Thr Gly Asn Gly Asn Val Ser
           195                 200                 205
   aaa gga gct cag gag atc ttc aag ctc atg ccg cac gag atg gtg cat       672
   Lys Gly Ala Gln Glu Ile Phe Lys Leu Met Pro His Glu Met Val His
       210                 215                 220
   cct tcg gaa cta cca aag ctg cca aaa aac aat cgt att ctc tac ggt       720
   Pro Ser Glu Leu Pro Lys Leu Pro Lys Asn Asn Arg Ile Leu Tyr Gly
   225                 230                 235                 240
   aca gtc atc gac gat ccg gcc tac ttt gtt aag cct cag ccg ggt ttc       768
   Thr Val Ile Asp Asp Pro Ala Tyr Phe Val Lys Pro Gln Pro Gly Phe
                   245                 250                 255
   acg ggt gag act acg aga gct cac tac tac cag aat cca cac caa tac       816
   Thr Gly Glu Thr Thr Arg Ala His Tyr Tyr Gln Asn Pro His Gln Tyr
               260                 265                 270
   gaa gct gct ttc cat gaa aaa gtg ttg cca tac acc tca atg ctc gtc       864
   Glu Ala Ala Phe His Glu Lys Val Leu Pro Tyr Thr Ser Met Leu Val
           275                 280                 285
   aac tgt atg tac tgg gac gac cgt ttt cca aga ttg gcc acg cga gag       912
   Asn Cys Met Tyr Trp Asp Asp Arg Phe Pro Arg Leu Ala Thr Arg Glu
       290                 295                 300
   cag att cgt gag ctt cgc aag tct ggg aac cac aaa ttg ctc ggg atc       960
   Gln Ile Arg Glu Leu Arg Lys Ser Gly Asn His Lys Leu Leu Gly Ile
   305                 310                 315                 320
   gct gat atc tcg tgc gat atc ggc gga agt atc gag ttt ctg gag cgt      1008
   Ala Asp Ile Ser Cys Asp Ile Gly Gly Ser Ile Glu Phe Leu Glu Arg
                   325                 330                 335
   gct act gaa att gag cgt ccg ttc gct ctg tac gac gtt gtt gaa gat      1056
   Ala Thr Glu Ile Glu Arg Pro Phe Ala Leu Tyr Asp Val Val Glu Asp
               340                 345                 350
   aaa atg cgc gaa gat gga gac agc cga ggc ctc gaa gga gac gac ggc      1104
   Lys Met Arg Glu Asp Gly Asp Ser Arg Gly Leu Glu Gly Asp Asp Gly
           355                 360                 365
   att atg atg atg ggt gtc gat atc tta ccg agt gaa ctg gca cgc gag      1152
   Ile Met Met Met Gly Val Asp Ile Leu Pro Ser Glu Leu Ala Arg Glu
       370                 375                 380
   tcg agt cag cag ttc ggt gac cgc ttg gtc gga tac gtc acg gcg ctg      1200
   Ser Ser Gln Gln Phe Gly Asp Arg Leu Val Gly Tyr Val Thr Ala Leu
   385                 390                 395                 400
   tcg agt gct tct tcg tcc aac gtt cct cta cat gaa cag aaa gag ctg      1248
   Ser Ser Ala Ser Ser Ser Asn Val Pro Leu His Glu Gln Lys Glu Leu
                   405                 410                 415
   cct gct gag cta cgt gga gct tgt atc gcc agc aat ggt gtg ctt gca        1296
   Pro Ala Glu Leu Arg Gly Ala Cys Ile Ala Ser Asn Gly Val Leu Ala
               420                 425                 430
   cct aaa tac gag tat atc cac cgt atg cga gcc gag cgc gag cgc agc        1344
   Pro Lys Tyr Glu Tyr Ile His Arg Met Arg Ala Glu Arg Glu Arg Ser
           435                 440                 445
   aag cag tac aag ttc ttg gat gct caa caa cag gtc gct ggg agt acg        1392
   Lys Gln Tyr Lys Phe Leu Asp Ala Gln Gln Gln Val Ala Gly Ser Thr
       450                 455                 460
   tgc gtt cta ctg gag ggg cac ctg ttt gat acc gga ctt att aac cag        1440
   Cys Val Leu Leu Glu Gly His Leu Phe Asp Thr Gly Leu Ile Asn Gln
   465                 470                 475                 480
   gta ctc aac ctc att gaa gac cat gat ggg ggt ttc cac ctt cta gac        1488
   Val Leu Asn Leu Ile Glu Asp His Asp Gly Gly Phe His Leu Leu Asp
                   485                 490                 495
   tgc gaa gta cgc cca aat gtt gga gtc ggg gac agt ggt gac agc acc        1536
   Cys Glu Val Arg Pro Asn Val Gly Val Gly Asp Ser Gly Asp Ser Thr
               500                 505                 510
   agt tcg aac gcc atc gtg cag att agt atg agt gac cgt gaa gcg ctg        1584
   Ser Ser Asn Ala Ile Val Gln Ile Ser Met Ser Asp Arg Glu Ala Leu
           515                 520                 525
   gat gct atc atc acc aag atc cgc tcc tta gct gag ctt act tct ggt        1632
   Asp Ala Ile Ile Thr Lys Ile Arg Ser Leu Ala Glu Leu Thr Ser Gly
       530                 535                 540
   gcg aag gct acg gtc aca gaa cta ccc gat ctg tgc ggc aca gac tac        1680
   Ala Lys Ala Thr Val Thr Glu Leu Pro Asp Leu Cys Gly Thr Asp Tyr
   545                 550                 555                 560
   tcc aag acg cga ggt gtc gtg aga aaa gat gca gca gcc aat act atg        1728
   Ser Lys Thr Arg Gly Val Val Arg Lys Asp Ala Ala Ala Asn Thr Met
                   565                 570                 575
   gct gaa gta tcg gtc tcc agc ccg aag aag cgg aag gtt gtg tgt ttt        1776
   Ala Glu Val Ser Val Ser Ser Pro Lys Lys Arg Lys Val Val Cys Phe
               580                 585                 590
   ggc gct gga ttg gtg gcg tca cca ctt gtg gag tat cta tca aga gag        1824
   Gly Ala Gly Leu Val Ala Ser Pro Leu Val Glu Tyr Leu Ser Arg Glu
           595                 600                 605
   caa gga aac gaa gtt cat gtg gtg tcg ggt att gag agg gaa gta aag        1872
   Gln Gly Asn Glu Val His Val Val Ser Gly Ile Glu Arg Glu Val Lys
       610                 615                 620
   gag atg atg cgc aaa atc tct cgt cga aac atc aag cct cac gta gtg        1920
   Glu Met Met Arg Lys Ile Ser Arg Arg Asn Ile Lys Pro His Val Val
   625                 630                 635                 640
   aac gtc gct gaa gat gct gct gga gtc gac aaa ctt tgc gct gaa gcc        1968
   Asn Val Ala Glu Asp Ala Ala Gly Val Asp Lys Leu Cys Ala Glu Ala
                   645                 650                 655
   gat tgc gtt gtg tcg cta ctg cca gcg acc atg cac acg aca att gct        2016
   Asp Cys Val Val Ser Leu Leu Pro Ala Thr Met His Thr Thr Ile Ala
               660                 665                 670
   cag cgc tgc att cag cat gcg act cct ctc gtg acg gct agc tat gtg      2064
   Gln Arg Cys Ile Gln His Ala Thr Pro Leu Val Thr Ala Ser Tyr Val
           675                 680                 685
   tca cca gaa atg aag gag ctg gac gct agg gca aag aag gca ggt att      2112
   Ser Pro Glu Met Lys Glu Leu Asp Ala Arg Ala Lys Lys Ala Gly Ile
       690                 695                 700
   ccg atc ctc tgt gag att ggt ctc gac cct ggt atg gat cac atg agt      2160
   Pro Ile Leu Cys Glu Ile Gly Leu Asp Pro Gly Met Asp His Met Ser
   705                 710                 715                 720
   gcc atg aaa gtc att gat gaa gtg aag gcc ctc tcc ggg aag gtc atg      2208
   Ala Met Lys Val Ile Asp Glu Val Lys Ala Leu Ser Gly Lys Val Met
                   725                 730                 735
   acc ttc tcg tct gtc tgt ggc ggc ttg cca gct cct gaa gca gcg gac      2256
   Thr Phe Ser Ser Val Cys Gly Gly Leu Pro Ala Pro Glu Ala Ala Asp
               740                 745                 750
   aac gcc att ggt tac aag ttc agt tgg agt ccg cgt ggt gtg ctc acg      2304
   Asn Ala Ile Gly Tyr Lys Phe Ser Trp Ser Pro Arg Gly Val Leu Thr
           755                 760                 765
   gcg gca ctg aat gca gcc cag tac cgc aaa gac ggc aaa gtc atc aac      2352
   Ala Ala Leu Asn Ala Ala Gln Tyr Arg Lys Asp Gly Lys Val Ile Asn
       770                 775                 780
   gtg gca ggt gaa gac tta ctc aat aga agc gaa cgc gta aac ttc ttg      2400
   Val Ala Gly Glu Asp Leu Leu Asn Arg Ser Glu Arg Val Asn Phe Leu
   785                 790                 795                 800
   ccg gcg ttc aac atc gag cag atc ccg aac cgt aat tcg ctt cct tac      2448
   Pro Ala Phe Asn Ile Glu Gln Ile Pro Asn Arg Asn Ser Leu Pro Tyr
                   805                 810                 815
   ggc gat atc tat ggc atc cca gag gca cac tcg ctg tat cgt gga act      2496
   Gly Asp Ile Tyr Gly Ile Pro Glu Ala His Ser Leu Tyr Arg Gly Thr
               820                 825                 830
   ctg cga tac ggt gga tgc tgt caa att ctg tac cag cta cgc aag ctt      2544
   Leu Arg Tyr Gly Gly Cys Cys Gln Ile Leu Tyr Gln Leu Arg Lys Leu
           835                 840                 845
   ggc ctc ttt gac atg gac cca tcc aaa ccc att cca gcg act tgg ccg      2592
   Gly Leu Phe Asp Met Asp Pro Ser Lys Pro Ile Pro Ala Thr Trp Pro
       850                 855                 860
   gat ctt ctg acg cag ttg ggt ggt ttc caa ggt ctt cgc gaa gac gcc      2640
   Asp Leu Leu Thr Gln Leu Gly Gly Phe Gln Gly Leu Arg Glu Asp Ala
   865                 870                 875                 880
   cat ggg ttc ctt cag tgg ctt ggt gcc ttt gat aaa tca aat cct gtg      2688
   His Gly Phe Leu Gln Trp Leu Gly Ala Phe Asp Lys Ser Asn Pro Val
                   885                 890                 895
   gtt aaa gcc ccg tcc atc ctt gac gct ttc tgc gct ctc ctg caa gat      2736
   Val Lys Ala Pro Ser Ile Leu Asp Ala Phe Cys Ala Leu Leu Gln Asp
               900                 905                 910
   aag ttg tcg tac caa cca gga gaa cgc gac atg gcc atc atg cat cat      2784
   Lys Leu Ser Tyr Gln Pro Gly Glu Arg Asp Met Ala Ile Met His His
           915                 920                 925
   gag ttc ggc atc gag tac gag gat ggc aag aag gaa aag cgt acg tca        2832
   Glu Phe Gly Ile Glu Tyr Glu Asp Gly Lys Lys Glu Lys Arg Thr Ser
       930                 935                 940
   aca ttc gtg ggc tac ggg tcc gag aag ggc gac acg atc atg gct aaa        2880
   Thr Phe Val Gly Tyr Gly Ser Glu Lys Gly Asp Thr Ile Met Ala Lys
   945                 950                 955                 960
   acg gtt gga tta agt gct gct atc ggc gtc cag ttg atc ttg cag gac        2928
   Thr Val Gly Leu Ser Ala Ala Ile Gly Val Gln Leu Ile Leu Gln Asp
                   965                 970                 975
   gca gtt caa ggt cgt ggt gtg ctt acc ccc aca acc ccc gac atc tac        2976
   Ala Val Gln Gly Arg Gly Val Leu Thr Pro Thr Thr Pro Asp Ile Tyr
               980                 985                 990
   ggt cct gca ctg gcc cgt ctc gag  gtc gaa ggt gtg cgc  ttc att gaa      3024
   Gly Pro Ala Leu Ala Arg Leu Glu  Val Glu Gly Val Arg  Phe Ile Glu
           995                 1000                1005
   aaa aca  ttc cca cag cat taa                                           3045
   Lys Thr  Phe Pro Gln His
       1010
<210> 24
   <211> 1014
   <212> PRT
   <213> Phytophthora infestans
<400> 24
   Met Ser Gly Lys Cys Val Gly Ile Val Arg Glu Val Tyr Asn Lys Trp
   1               5                   10                  15
   Glu Arg Arg Ala Pro Leu Thr Pro Ala His Val Lys Glu Leu Val Ala
               20                  25                  30
   Arg Gly Val Gln Val Leu Val Gln Pro Ser Thr Ala Arg Val Phe Ser
           35                  40                  45
   Asp Asp Gln Tyr Val Arg Ala Gly Ala Thr Leu Ser Glu Asn Leu Ala
       50                  55                  60
   Pro Ala Asn Val Ile Val Gly Val Lys Gln Val Pro Glu Pro Ala Leu
   65                  70                  75                  80
   Leu Ala Asp Lys Thr Tyr Leu Phe Phe Ser His Thr Ile Lys Ala Gln
                   85                  90                  95
   Pro Glu Asn Met Ala Leu Leu Asp Ala Val Leu Gln Arg Arg Val Thr
               100                 105                 110
   Leu Ile Asp Tyr Glu Cys Ile Thr Glu Glu Ser Gly Lys Arg Leu Ile
           115                 120                 125
   Ala Phe Gly Gly Asn Ala Gly Arg Ala Gly Met Ile Ala Gly Phe Arg
   130 135 140
   Gly Leu Gly Glu Arg Leu Ile Asn Met Gly Ile Ser Ser Pro Phe Val
   145 150 155 160
   Asn Ile Ala Ser Ser Tyr Met Tyr Ala Asp Leu Glu His Ala Lys Asp
   165 170 175
   Ala Val Glu Ala Ala Gly Arg Arg Ile Arg Thr Asp Gly Leu Pro Gly
   180 185 190
   Asp Leu Ala Pro Met Thr Phe Ala Phe Thr Gly Asn Gly Asn Val Ser
   195 200 205
   Lys Gly Ala Gln Glu Ile Phe Lys Leu Met Pro His Glu Met Val His
   210 215 220
   Pro Ser Glu Leu Pro Lys Leu Pro Lys Asn Asn Arg Ile Leu Tyr Gly
   225 230 235 240
   Thr Val Ile Asp Asp Pro Ala Tyr Phe Val Lys Pro Gln Pro Gly Phe
   245 250 255
   Thr Gly Glu Thr Thr Arg Ala His Tyr Tyr Gln Asn Pro His Gln Tyr
   260 265 270
   Glu Ala Ala Phe His Glu Lys Val Leu Pro Tyr Thr Ser Met Leu Val
   275 280 285
   Asn Cys Met Tyr Trp Asp Asp Arg Phe Pro Arg Leu Ala Thr Arg Glu
   290 295 300
   Gln Ile Arg Glu Leu Arg Lys Ser Gly Asn His Lys Leu Leu Gly Ile
   305 310 315 320
   Ala Asp Ile Ser Cys Asp Ile Gly Gly Ser Ile Glu Phe Leu Glu Arg
   325 330 335
   Ala Thr Glu Ile Glu Arg Pro Phe Ala Leu Tyr Asp Val Val Glu Asp
   340 345 350
   Lys Met Arg Glu Asp Gly Asp Ser Arg Gly Leu Glu Gly Asp Asp Gly
   355 360 365
   Ile Met Met Met Gly Val Asp Ile Leu Pro Ser Glu Leu Ala Arg Glu
   370 375 380
   Ser Ser Gln Gln Phe Gly Asp Arg Leu Val Gly Tyr Val Thr Ala Leu
   385 390 395 400
   Ser Ser Ala Ser Ser Ser Asn Val Pro Leu His Glu Gln Lys Glu Leu
   405 410 415
   Pro Ala Glu Leu Arg Gly Ala Cys Ile Ala Ser Asn Gly Val Leu Ala
   420 425 430
   Pro Lys Tyr Glu Tyr Ile His Arg Met Arg Ala Glu Arg Glu Arg Ser
   435 440 445
   Lys Gln Tyr Lys Phe Leu Asp Ala Gln Gln Gln Val Ala Gly Ser Thr
   450 455 460
   Cys Val Leu Leu Glu Gly His Leu Phe Asp Thr Gly Leu Ile Asn Gln
   465 470 475 480
   Val Leu Asn Leu Ile Glu Asp His Asp Gly Gly Phe His Leu Leu Asp
   485 490 495
   Cys Glu Val Arg Pro Asn Val Gly Val Gly Asp Ser Gly Asp Ser Thr
   500 505 510
   Ser Ser Asn Ala Ile Val Gln Ile Ser Met Ser Asp Arg Glu Ala Leu
   515 520 525
   Asp Ala Ile Ile Thr Lys Ile Arg Ser Leu Ala Glu Leu Thr Ser Gly
   530 535 540
   Ala Lys Ala Thr Val Thr Glu Leu Pro Asp Leu Cys Gly Thr Asp Tyr
   545 550 555 560
   Ser Lys Thr Arg Gly Val Val Arg Lys Asp Ala Ala Ala Asn Thr Met
   565 570 575
   Ala Glu Val Ser Val Ser Ser Pro Lys Lys Arg Lys Val Val Cys Phe
   580 585 590
   Gly Ala Gly Leu Val Ala Ser Pro Leu Val Glu Tyr Leu Ser Arg Glu
   595 600 605
   Gln Gly Asn Glu Val His Val Val Ser Gly Ile Glu Arg Glu Val Lys
   610 615 620
   Glu Met Met Arg Lys Ile Ser Arg Arg Asn Ile Lys Pro His Val Val
   625 630 635 640
   Asn Val Ala Glu Asp Ala Ala Gly Val Asp Lys Leu Cys Ala Glu Ala
   645 650 655
   Asp Cys Val Val Ser Leu Leu Pro Ala Thr Met His Thr Thr Ile Ala
               660                 665                 670
   Gln Arg Cys Ile Gln His Ala Thr Pro Leu Val Thr Ala Ser Tyr Val
           675                 680                 685
   Ser Pro Glu Met Lys Glu Leu Asp Ala Arg Ala Lys Lys Ala Gly Ile
       690                 695                 700
   Pro Ile Leu Cys Glu Ile Gly Leu Asp Pro Gly Met Asp His Met Ser
   705                 710                 715                 720
   Ala Met Lys Val Ile Asp Glu Val Lys Ala Leu Ser Gly Lys Val Met
                   725                 730                 735
   Thr Phe Ser Ser Val Cys Gly Gly Leu Pro Ala Pro Glu Ala Ala Asp
               740                 745                 750
   Asn Ala Ile Gly Tyr Lys Phe Ser Trp Ser Pro Arg Gly Val Leu Thr
           755                 760                 765
   Ala Ala Leu Asn Ala Ala Gln Tyr Arg Lys Asp Gly Lys Val Ile Asn
       770                 775                 780
   Val Ala Gly Glu Asp Leu Leu Asn Arg Ser Glu Arg Val Asn Phe Leu
   785                 790                 795                 800
   Pro Ala Phe Asn Ile Glu Gln Ile Pro Asn Arg Asn Ser Leu Pro Tyr
                   805                 810                 815
   Gly Asp Ile Tyr Gly Ile Pro Glu Ala His Ser Leu Tyr Arg Gly Thr
               820                 825                 830
   Leu Arg Tyr Gly Gly Cys Cys Gln Ile Leu Tyr Gln Leu Arg Lys Leu
           835                 840                 845
   Gly Leu Phe Asp Met Asp Pro Ser Lys Pro Ile Pro Ala Thr Trp Pro
       850                 855                 860
   Asp Leu Leu Thr Gln Leu Gly Gly Phe Gln Gly Leu Arg Glu Asp Ala
   865                 870                 875                 880
   His Gly Phe Leu Gln Trp Leu Gly Ala Phe Asp Lys Ser Asn Pro Val
                   885                 890                 895
   Val Lys Ala Pro Ser Ile Leu Asp Ala Phe Cys Ala Leu Leu Gln Asp
               900                 905                 910
   Lys Leu Ser Tyr Gln Pro Gly Glu Arg Asp Met Ala Ile Met His His
   915 920 925
   Glu Phe Gly Ile Glu Tyr Glu Asp Gly Lys Lys Glu Lys Arg Thr Ser
   930 935 940
   Thr Phe Val Gly Tyr Gly Ser Glu Lys Gly Asp Thr Ile Met Ala Lys
   945 950 955 960
   Thr Val Gly Leu Ser Ala Ala Ile Gly Val Gln Leu Ile Leu Gln Asp
   965 970 975
   Ala Val Gln Gly Arg Gly Val Leu Thr Pro Thr Thr Pro Asp Ile Tyr
   980 985 990
   Gly Pro Ala Leu Ala Arg Leu Glu Val Glu Gly Val Arg Phe Ile Glu
   995 1000 1005
   Lys Thr Phe Pro Gln His
   1010
<210> 25
   <211> 3015
   <212> DNA
   <213> Phytophthora ramorum
<220>
   <221> CDS
   <222> (1)..(3015)
   <223> Lys1
<400> 25
   gtg ggc atc gtg cgc gag gtg tac aac aag tgg gag cgc cgc gcg ccg          48
   Val Gly Ile Val Arg Glu Val Tyr Asn Lys Trp Glu Arg Arg Ala Pro
   1               5                   10                  15
   ctc acc ccc gcg cac gtc cgc gag ctc gtg cag cgc ggc gtg cag gtg          96
   Leu Thr Pro Ala His Val Arg Glu Leu Val Gln Arg Gly Val Gln Val
               20                  25                  30
   ttg gtg cag ccg tct acg gcg cgc gtc ttc tca gac gag cag tac gtg         144
   Leu Val Gln Pro Ser Thr Ala Arg Val Phe Ser Asp Glu Gln Tyr Val
           35                  40                  45
   cga gcg ggc gcc aag ctg gcc gag gac ctg gcg ccc gcc aat atc atc         192
   Arg Ala Gly Ala Lys Leu Ala Glu Asp Leu Ala Pro Ala Asn Ile Ile
       50                  55                  60
   gtg ggc gtc aag cag gta ccg gag ccg gcg ctt ctg gcg gac aag aca         240
   Val Gly Val Lys Gln Val Pro Glu Pro Ala Leu Leu Ala Asp Lys Thr
   65                  70                  75                  80
   tac ctc ttc ttc agc cat acc atc aag gcg cag cct gag aac atg gcg         288
   Tyr Leu Phe Phe Ser His Thr Ile Lys Ala Gln Pro Glu Asn Met Ala
                   85                  90                  95
   ctc ctg gat gcc gtg ctg cag cgc cgc gtc acg ctc atc gac tac gag         336
   Leu Leu Asp Ala Val Leu Gln Arg Arg Val Thr Leu Ile Asp Tyr Glu
               100                 105                 110
   tgc atc aca gag gag agc ggc aag cgc ctc atc gcc ttc gga ggc aac      384
   Cys Ile Thr Glu Glu Ser Gly Lys Arg Leu Ile Ala Phe Gly Gly Asn
           115                 120                 125
   gcg ggg cga gca gga atg atc gcg ggc ttc cgg ggg ctt ggg gaa cgc      432
   Ala Gly Arg Ala Gly Met Ile Ala Gly Phe Arg Gly Leu Gly Glu Arg
       130                 135                 140
   ctc atc aac atg ggc atc tcc tcg ccc ttt gtg aac gta gcg tcg gcc      480
   Leu Ile Asn Met Gly Ile Ser Ser Pro Phe Val Asn Val Ala Ser Ala
   145                 150                 155                 160
   tac atg tac tcg gac ttg gag cac gca aaa gac gcc gta gaa gct gcg      528
   Tyr Met Tyr Ser Asp Leu Glu His Ala Lys Asp Ala Val Glu Ala Ala
                   165                 170                 175
   ggc agg agg att cgg tcc gat gga ttg cca ggc gag ctg gcg ccc atg      576
   Gly Arg Arg Ile Arg Ser Asp Gly Leu Pro Gly Glu Leu Ala Pro Met
               180                 185                 190
   gcc ttc gcc ttc acg ggc aat ggc aac gtg tcc aaa ggg gcg cag gag      624
   Ala Phe Ala Phe Thr Gly Asn Gly Asn Val Ser Lys Gly Ala Gln Glu
           195                 200                 205
   atc ttc aag ctc atg cca cat gaa atg gtg cat ccg tca gag ctg cca      672
   Ile Phe Lys Leu Met Pro His Glu Met Val His Pro Ser Glu Leu Pro
       210                 215                 220
   aac ttg ccg aag aac aac cac atc ctg tac ggc acc gtg gtt gat gac      720
   Asn Leu Pro Lys Asn Asn His Ile Leu Tyr Gly Thr Val Val Asp Asp
   225                 230                 235                 240
   ccg gcc ttc ttt gtg aag ccg cag gct ggc aac agc ggc acg gcc tcg      768
   Pro Ala Phe Phe Val Lys Pro Gln Ala Gly Asn Ser Gly Thr Ala Ser
                   245                 250                 255
   cgc gca cac tac tac cag aac cca cac cag tac gac cct gcc ttc cac      816
   Arg Ala His Tyr Tyr Gln Asn Pro His Gln Tyr Asp Pro Ala Phe His
               260                 265                 270
   gag aag gtg ctg ccg tac acg tcc atg ttg gtg aac tgc atg tac tgg      864
   Glu Lys Val Leu Pro Tyr Thr Ser Met Leu Val Asn Cys Met Tyr Trp
           275                 280                 285
   gac gac cgt ttc ccg cga ttg gtt acg cgc gaa cag atc cgt gag ctt      912
   Asp Asp Arg Phe Pro Arg Leu Val Thr Arg Glu Gln Ile Arg Glu Leu
       290                 295                 300
   cga ggc tct gga aac caa aag ctg ctg ggc att gcc gat att tcg tgc      960
   Arg Gly Ser Gly Asn Gln Lys Leu Leu Gly Ile Ala Asp Ile Ser Cys
   305                 310                 315                 320
   gat att ggc ggc agt gtt gaa ttt ttg gag cgc gcg acg gaa att gaa     1008
   Asp Ile Gly Gly Ser Val Glu Phe Leu Glu Arg Ala Thr Glu Ile Glu
                   325                 330                 335
   cgc ccg ttc gcg ctg tac gat gtc gct gaa gac aaa atg cgc gag gat     1056
   Arg Pro Phe Ala Leu Tyr Asp Val Ala Glu Asp Lys Met Arg Glu Asp
               340                 345                 350
   ggc gat agt cga ggg ctc gaa ggg gat aac ggc atc atg atg atg ggg     1104
   Gly Asp Ser Arg Gly Leu Glu Gly Asp Asn Gly Ile Met Met Met Gly
           355                 360                 365
   gtc gat ata ttg cca agc gaa ctt cca cgc gag tct agt caa cag ttc        1152
   Val Asp Ile Leu Pro Ser Glu Leu Pro Arg Glu Ser Ser Gln Gln Phe
       370                 375                 380
   ggc gac cgt ctg gtc gca tac gtg gcg gcg ctg tcg aat gct tcc tca        1200
   Gly Asp Arg Leu Val Ala Tyr Val Ala Ala Leu Ser Asn Ala Ser Ser
   385                 390                 395                 400
   tcc agt gtc cca ctt cat gag caa aag gag cta cct gct gag ctc cgt        1248
   Ser Ser Val Pro Leu His Glu Gln Lys Glu Leu Pro Ala Glu Leu Arg
                   405                 410                 415
   ggc gcc tgc atc gca agc aaa gga gta ctc gcc cca aaa tac gag tac        1296
   Gly Ala Cys Ile Ala Ser Lys Gly Val Leu Ala Pro Lys Tyr Glu Tyr
               420                 425                 430
   att cac cgt atg cgt gcc gaa cgt gag cgc agt aag cag tac aag ttc        1344
   Ile His Arg Met Arg Ala Glu Arg Glu Arg Ser Lys Gln Tyr Lys Phe
           435                 440                 445
   ctg gat gcg cag cag gaa gtt gcg ggg agc acg tgt att ctg ctt gaa        1392
   Leu Asp Ala Gln Gln Glu Val Ala Gly Ser Thr Cys Ile Leu Leu Glu
       450                 455                 460
   ggt cac ctc ttc gat aca gga ctt atc aac caa gtg ctc aac ctc att        1440
   Gly His Leu Phe Asp Thr Gly Leu Ile Asn Gln Val Leu Asn Leu Ile
   465                 470                 475                 480
   gag gac cat gat ggc gga ttc cac ctt gtg gac tgt gaa gtg cgg ccg        1488
   Glu Asp His Asp Gly Gly Phe His Leu Val Asp Cys Glu Val Arg Pro
                   485                 490                 495
   aac gtt gct gtc agc gac agt ggc gac tgt acc ata tcg aat gct atc        1536
   Asn Val Ala Val Ser Asp Ser Gly Asp Cys Thr Ile Ser Asn Ala Ile
               500                 505                 510
   gtc cag ata agc atg aac gat cga gct gcg ctg gat gaa att atc tca        1584
   Val Gln Ile Ser Met Asn Asp Arg Ala Ala Leu Asp Glu Ile Ile Ser
           515                 520                 525
   aag gtt cgc tct ctt gct gac ctt acc tcc ggt gcc aag gct act gtc        1632
   Lys Val Arg Ser Leu Ala Asp Leu Thr Ser Gly Ala Lys Ala Thr Val
       530                 535                 540
   act gag ctt ccc gac ttg tgc ggt aca aac tac tcg aag aca cgt ggt        1680
   Thr Glu Leu Pro Asp Leu Cys Gly Thr Asn Tyr Ser Lys Thr Arg Gly
   545                 550                 555                 560
   gct gta gta cgg aag gac gcg gct gcc aat acg act acc gac gtg tcg        1728
   Ala Val Val Arg Lys Asp Ala Ala Ala Asn Thr Thr Thr Asp Val Ser
                   565                 570                 575
   gtc tca agc cca aag agg cga aag att gta tgc ttt ggc gcc ggg ttg        1776
   Val Ser Ser Pro Lys Arg Arg Lys Ile Val Cys Phe Gly Ala Gly Leu
               580                 585                 590
   gtg gca tcg ccc ctt gtg gag tat ctg tcg cgt gaa caa gtc aac gaa        1824
   Val Ala Ser Pro Leu Val Glu Tyr Leu Ser Arg Glu Gln Val Asn Glu
           595                 600                 605
   gtt cat gtg gtg tct ggc ctc gag ggt gag gta aag gga att atg cgc        1872
   Val His Val Val Ser Gly Leu Glu Gly Glu Val Lys Gly Ile Met Arg
       610                 615                 620
   aag atc tct cgc cga aac atc aag cct cat gtt gta aac gta gct gaa      1920
   Lys Ile Ser Arg Arg Asn Ile Lys Pro His Val Val Asn Val Ala Glu
   625                 630                 635                 640
   gac act gcc gga gtt gac aag ctt tgt gca gaa gct gat tgt gtc gtg      1968
   Asp Thr Ala Gly Val Asp Lys Leu Cys Ala Glu Ala Asp Cys Val Val
                   645                 650                 655
   tca ctt ttg cca gcg acc atg cac aca acg att gcc gag cgc tgt att      2016
   Ser Leu Leu Pro Ala Thr Met His Thr Thr Ile Ala Glu Arg Cys Ile
               660                 665                 670
   cag cac gcg aca cca ctt gtg act gca agt tat gtg tct ccg gag atg      2064
   Gln His Ala Thr Pro Leu Val Thr Ala Ser Tyr Val Ser Pro Glu Met
           675                 680                 685
   aaa gag ctg gac tcc aag gca aag caa gct ggc atc cct atc ctc tgt      2112
   Lys Glu Leu Asp Ser Lys Ala Lys Gln Ala Gly Ile Pro Ile Leu Cys
       690                 695                 700
   gaa att ggt ctt gac cct ggc atg gat cac atg agc gcg atg aaa gtt      2160
   Glu Ile Gly Leu Asp Pro Gly Met Asp His Met Ser Ala Met Lys Val
   705                 710                 715                 720
   att gat gag gtg aag gcg cac tct ggg aag atc aag tct ttc tcg tct      2208
   Ile Asp Glu Val Lys Ala His Ser Gly Lys Ile Lys Ser Phe Ser Ser
                   725                 730                 735
   gta tgt ggc ggg ctg ccg gcg cct gag gca gcg gac aac gct att ggt      2256
   Val Cys Gly Gly Leu Pro Ala Pro Glu Ala Ala Asp Asn Ala Ile Gly
               740                 745                 750
   tac aag ttc agc tgg agt ccg cgc ggt gtg cta aca gca gcg ctg aat      2304
   Tyr Lys Phe Ser Trp Ser Pro Arg Gly Val Leu Thr Ala Ala Leu Asn
           755                 760                 765
   gca gct caa tac cgc aaa gac gac aaa gtc att aat gtg gcc ggt gaa      2352
   Ala Ala Gln Tyr Arg Lys Asp Asp Lys Val Ile Asn Val Ala Gly Glu
       770                 775                 780
   gac ttg cta aac agc agt gag cgc gtg aac ttc ctg cca gcg ttc aac      2400
   Asp Leu Leu Asn Ser Ser Glu Arg Val Asn Phe Leu Pro Ala Phe Asn
   785                 790                 795                 800
   att gag cag att cca aac cgt aat tcg ctg ccg tac ggc gat atc tac      2448
   Ile Glu Gln Ile Pro Asn Arg Asn Ser Leu Pro Tyr Gly Asp Ile Tyr
                   805                 810                 815
   ggc att ccg gaa gca cac tca ttg tat cgc ggc act ctg cgc tat ggt      2496
   Gly Ile Pro Glu Ala His Ser Leu Tyr Arg Gly Thr Leu Arg Tyr Gly
               820                 825                 830
   ggc tgc tgt caa att ttg tac caa cta cgt aag ctc ggc ctc ttc gac      2544
   Gly Cys Cys Gln Ile Leu Tyr Gln Leu Arg Lys Leu Gly Leu Phe Asp
           835                 840                 845
   atg gac cca tcc aag ccc att cca gct act tgg cca gac ctt ctc act      2592
   Met Asp Pro Ser Lys Pro Ile Pro Ala Thr Trp Pro Asp Leu Leu Thr
       850                 855                 860
   caa tta ggt gga cac caa gac ctt cgt gaa gat gcc agc ggg ttc ctt      2640
   Gln Leu Gly Gly His Gln Asp Leu Arg Glu Asp Ala Ser Gly Phe Leu
   865                 870                 875                 880
   cag tgg ctt ggt gcc ttt gat cgc acc acg cct att gtt aag gca ccg        2688
   Gln Trp Leu Gly Ala Phe Asp Arg Thr Thr Pro Ile Val Lys Ala Pro
                   885                 890                 895
   tcc gtt ctc gac gct ttc tgt aca ttg ctg cag gac aag tta tcg tac        2736
   Ser Val Leu Asp Ala Phe Cys Thr Leu Leu Gln Asp Lys Leu Ser Tyr
               900                 905                 910
   cag ccg ggt gaa cgc gac atg gcc atc atg cac cat gaa ttc ggc att        2784
   Gln Pro Gly Glu Arg Asp Met Ala Ile Met His His Glu Phe Gly Ile
           915                 920                 925
   gag tac gaa gat ggc aaa agg gaa aaa cgc acg tca acg ttc gtg ggc        2832
   Glu Tyr Glu Asp Gly Lys Arg Glu Lys Arg Thr Ser Thr Phe Val Gly
       930                 935                 940
   tat ggc tcc gag aag ggc gac aca atc atg gca aaa act gtc ggc ttg        2880
   Tyr Gly Ser Glu Lys Gly Asp Thr Ile Met Ala Lys Thr Val Gly Leu
   945                 950                 955                 960
   agt gca gct att ggc gtg cag ctg att ttg cag gac gcc gtc cag ggc        2928
   Ser Ala Ala Ile Gly Val Gln Leu Ile Leu Gln Asp Ala Val Gln Gly
                   965                 970                 975
   cga ggt gtg cta acc ccg aca acc cct gac atc tac ggc cca gcc ctc        2976
   Arg Gly Val Leu Thr Pro Thr Thr Pro Asp Ile Tyr Gly Pro Ala Leu
               980                 985                 990
   gcc cgt ctc gaa gtc gaa ggt gtg  cgc ttc atc gag aag                   3015
   Ala Arg Leu Glu Val Glu Gly Val  Arg Phe Ile Glu Lys
           995                 1000                1005
<210> 26
   <211> 1005
   <212> PRT
   <213> Phytophthora ramorum
<400> 26
   Val Gly Ile Val Arg Glu Val Tyr Asn Lys Trp Glu Arg Arg Ala Pro
   1               5                   10                  15
   Leu Thr Pro Ala His Val Arg Glu Leu Val Gln Arg Gly Val Gln Val
               20                  25                  30
   Leu Val Gln Pro Ser Thr Ala Arg Val Phe Ser Asp Glu Gln Tyr Val
           35                  40                  45
   Arg Ala Gly Ala Lys Leu Ala Glu Asp Leu Ala Pro Ala Asn Ile Ile
       50                  55                  60
   Val Gly Val Lys Gln Val Pro Glu Pro Ala Leu Leu Ala Asp Lys Thr
   65                  70                  75                  80
   Tyr Leu Phe Phe Ser His Thr Ile Lys Ala Gln Pro Glu Asn Met Ala
                   85                  90                  95
   Leu Leu Asp Ala Val Leu Gln Arg Arg Val Thr Leu Ile Asp Tyr Glu
   100 105 110
   Cys Ile Thr Glu Glu Ser Gly Lys Arg Leu Ile Ala Phe Gly Gly Asn
   115 120 125
   Ala Gly Arg Ala Gly Met Ile Ala Gly Phe Arg Gly Leu Gly Glu Arg
   130 135 140
   Leu Ile Asn Met Gly Ile Ser Ser Pro Phe Val Asn Val Ala Ser Ala
   145 150 155 160
   Tyr Met Tyr Ser Asp Leu Glu His Ala Lys Asp Ala Val Glu Ala Ala
   165 170 175
   Gly Arg Arg Ile Arg Ser Asp Gly Leu Pro Gly Glu Leu Ala Pro Met
   180 185 190
   Ala Phe Ala Phe Thr Gly Asn Gly Asn Val Ser Lys Gly Ala Gln Glu
   195 200 205
   Ile Phe Lys Leu Met Pro His Glu Met Val His Pro Ser Glu Leu Pro
   210 215 220
   Asn Leu Pro Lys Asn Asn His Ile Leu Tyr Gly Thr Val Val Asp Asp
   225 230 235 240
   Pro Ala Phe Phe Val Lys Pro Gln Ala Gly Asn Ser Gly Thr Ala Ser
   245 250 255
   Arg Ala His Tyr Tyr Gln Asn Pro His Gln Tyr Asp Pro Ala Phe His
   260 265 270
   Glu Lys Val Leu Pro Tyr Thr Ser Met Leu Val Asn Cys Met Tyr Trp
   275 280 285
   Asp Asp Arg Phe Pro Arg Leu Val Thr Arg Glu Gln Ile Arg Glu Leu
   290 295 300
   Arg Gly Ser Gly Asn Gln Lys Leu Leu Gly Ile Ala Asp Ile Ser Cys
   305 310 315 320
   Asp Ile Gly Gly Ser Val Glu Phe Leu Glu Arg Ala Thr Glu Ile Glu
   325 330 335
   Arg Pro Phe Ala Leu Tyr Asp Val Ala Glu Asp Lys Met Arg Glu Asp
   340 345 350
   Gly Asp Ser Arg Gly Leu Glu Gly Asp Asn Gly Ile Met Met Met Gly
   355 360 365
   Val Asp Ile Leu Pro Ser Glu Leu Pro Arg Glu Ser Ser Gln Gln Phe
       370                 375                 380
   Gly Asp Arg Leu Val Ala Tyr Val Ala Ala Leu Ser Asn Ala Ser Ser
   385                 390                 395                 400
   Ser Ser Val Pro Leu His Glu Gln Lys Glu Leu Pro Ala Glu Leu Arg
                   405                 410                 415
   Gly Ala Cys Ile Ala Ser Lys Gly Val Leu Ala Pro Lys Tyr Glu Tyr
               420                 425                 430
   Ile His Arg Met Arg Ala Glu Arg Glu Arg Ser Lys Gln Tyr Lys Phe
           435                 440                 445
   Leu Asp Ala Gln Gln Glu Val Ala Gly Ser Thr Cys Ile Leu Leu Glu
       450                 455                 460
   Gly His Leu Phe Asp Thr Gly Leu Ile Asn Gln Val Leu Asn Leu Ile
   465                 470                 475                 480
   Glu Asp His Asp Gly Gly Phe His Leu Val Asp Cys Glu Val Arg Pro
                   485                 490                 495
   Asn Val Ala Val Ser Asp Ser Gly Asp Cys Thr Ile Ser Asn Ala Ile
               500                 505                 510
   Val Gln Ile Ser Met Asn Asp Arg Ala Ala Leu Asp Glu Ile Ile Ser
           515                 520                 525
   Lys Val Arg Ser Leu Ala Asp Leu Thr Ser Gly Ala Lys Ala Thr Val
       530                 535                 540
   Thr Glu Leu Pro Asp Leu Cys Gly Thr Asn Tyr Ser Lys Thr Arg Gly
   545                 550                 555                 560
   Ala Val Val Arg Lys Asp Ala Ala Ala Asn Thr Thr Thr Asp Val Ser
                   565                 570                 575
   Val Ser Ser Pro Lys Arg Arg Lys Ile Val Cys Phe Gly Ala Gly Leu
               580                 585                 590
   Val Ala Ser Pro Leu Val Glu Tyr Leu Ser Arg Glu Gln Val Asn Glu
           595                 600                 605
   Val His Val Val Ser Gly Leu Glu Gly Glu Val Lys Gly Ile Met Arg
       610                 615                 620
   Lys Ile Ser Arg Arg Asn Ile Lys Pro His Val Val Asn Val Ala Glu
   625                 630                 635                 640
   Asp Thr Ala Gly Val Asp Lys Leu Cys Ala Glu Ala Asp Cys Val Val
                   645                 650                 655
   Ser Leu Leu Pro Ala Thr Met His Thr Thr Ile Ala Glu Arg Cys Ile
               660                 665                 670
   Gln His Ala Thr Pro Leu Val Thr Ala Ser Tyr Val Ser Pro Glu Met
           675                 680                 685
   Lys Glu Leu Asp Ser Lys Ala Lys Gln Ala Gly Ile Pro Ile Leu Cys
       690                 695                 700
   Glu Ile Gly Leu Asp Pro Gly Met Asp His Met Ser Ala Met Lys Val
   705                 710                 715                 720
   Ile Asp Glu Val Lys Ala His Ser Gly Lys Ile Lys Ser Phe Ser Ser
                   725                 730                 735
   Val Cys Gly Gly Leu Pro Ala Pro Glu Ala Ala Asp Asn Ala Ile Gly
               740                 745                 750
   Tyr Lys Phe Ser Trp Ser Pro Arg Gly Val Leu Thr Ala Ala Leu Asn
           755                 760                 765
   Ala Ala Gln Tyr Arg Lys Asp Asp Lys Val Ile Asn Val Ala Gly Glu
       770                 775                 780
   Asp Leu Leu Asn Ser Ser Glu Arg Val Asn Phe Leu Pro Ala Phe Asn
   785                 790                 795                 800
   Ile Glu Gln Ile Pro Asn Arg Asn Ser Leu Pro Tyr Gly Asp Ile Tyr
                   805                 810                 815
   Gly Ile Pro Glu Ala His Ser Leu Tyr Arg Gly Thr Leu Arg Tyr Gly
               820                 825                 830
   Gly Cys Cys Gln Ile Leu Tyr Gln Leu Arg Lys Leu Gly Leu Phe Asp
           835                 840                 845
   Met Asp Pro Ser Lys Pro Ile Pro Ala Thr Trp Pro Asp Leu Leu Thr
       850                 855                 860
   Gln Leu Gly Gly His Gln Asp Leu Arg Glu Asp Ala Ser Gly Phe Leu
   865                 870                 875                 880
   Gln Trp Leu Gly Ala Phe Asp Arg Thr Thr Pro Ile Val Lys Ala Pro
                   885                 890                 895
   Ser Val Leu Asp Ala Phe Cys Thr Leu Leu Gln Asp Lys Leu Ser Tyr
               900                 905                 910
   Gln Pro Gly Glu Arg Asp Met Ala Ile Met His His Glu Phe Gly Ile
           915                 920                 925
   Glu Tyr Glu Asp Gly Lys Arg Glu Lys Arg Thr Ser Thr Phe Val Gly
       930                 935                 940
   Tyr Gly Ser Glu Lys Gly Asp Thr Ile Met Ala Lys Thr Val Gly Leu
   945                 950                 955                 960
   Ser Ala Ala Ile Gly Val Gln Leu Ile Leu Gln Asp Ala Val Gln Gly
                   965                 970                 975
   Arg Gly Val Leu Thr Pro Thr Thr Pro Asp Ile Tyr Gly Pro Ala Leu
               980                 985                 990
   Ala Arg Leu Glu Val Glu Gly Val  Arg Phe Ile Glu Lys
           995                 1000                1005
<210> 27
   <211> 3048
   <212> DNA
   <213> Phytophthora sojae
<220>
   <221> CDS
   <222> (1)..(3048)
   <223> Lys1
<400> 27
   atg acg ggc aag tgc gtg ggc atc gtc cgc gag gtg tac aac aag tgg        48
   Met Thr Gly Lys Cys Val Gly Ile Val Arg Glu Val Tyr Asn Lys Trp
   1               5                   10                  15
   gag cgg cgc gcg ccg ctc acg ccc gcg cac gtc cgg gag ctc gtg cag        96
   Glu Arg Arg Ala Pro Leu Thr Pro Ala His Val Arg Glu Leu Val Gln
               20                  25                  30
   cgc ggc atc cag gtg ctg gtg cag ccg tcc acg gcg cgc gtc ttc tcg       144
   Arg Gly Ile Gln Val Leu Val Gln Pro Ser Thr Ala Arg Val Phe Ser
           35                  40                  45
   gac gag cag tac gtg cgc gcg ggc gcc act ttg tcc gag gac ctg gcg       192
   Asp Glu Gln Tyr Val Arg Ala Gly Ala Thr Leu Ser Glu Asp Leu Ala
       50                  55                  60
   ccc gcc aac gtc atc gtg ggc gtc aag cag gtg ccc gag ccg gcg ctg       240
   Pro Ala Asn Val Ile Val Gly Val Lys Gln Val Pro Glu Pro Ala Leu
   65                  70                  75                  80
   ctg gcc gac aag acg tac ctc ttc ttc agc cac acc atc aag gcg cag       288
   Leu Ala Asp Lys Thr Tyr Leu Phe Phe Ser His Thr Ile Lys Ala Gln
                   85                  90                  95
   ccg gag aac atg gcg ttg ctg gac gcc gtg ctg cag cgc cgc atc acg      336
   Pro Glu Asn Met Ala Leu Leu Asp Ala Val Leu Gln Arg Arg Ile Thr
               100                 105                 110
   ctc gtc gac tac gag tgc atc aca gag gag agc ggc aag cgc ctc atc      384
   Leu Val Asp Tyr Glu Cys Ile Thr Glu Glu Ser Gly Lys Arg Leu Ile
           115                 120                 125
   gcc ttc ggg ggc aac gcc ggc cgc gcc ggt atg atc gcg ggc ttc cgg      432
   Ala Phe Gly Gly Asn Ala Gly Arg Ala Gly Met Ile Ala Gly Phe Arg
       130                 135                 140
   ggg ctt gga gag cgc ctc atc aac atg ggc gtc tcc tcg ccc ttc gtg      480
   Gly Leu Gly Glu Arg Leu Ile Asn Met Gly Val Ser Ser Pro Phe Val
   145                 150                 155                 160
   aac gtc gcg tcc gcc tac atg tac tcg gac ttg gag cac gcc aag gac      528
   Asn Val Ala Ser Ala Tyr Met Tyr Ser Asp Leu Glu His Ala Lys Asp
                   165                 170                 175
   gcg gtg gag gcc gcg ggc aag agg atc cgc tcg gac gga ctg ccg agc      576
   Ala Val Glu Ala Ala Gly Lys Arg Ile Arg Ser Asp Gly Leu Pro Ser
               180                 185                 190
   gag ctg gtg ccc atg acg ttc gca ttc acg ggc aac ggc aat gtg tca      624
   Glu Leu Val Pro Met Thr Phe Ala Phe Thr Gly Asn Gly Asn Val Ser
           195                 200                 205
   aag ggc gcg cag gag atc ttc aag ctt atg ccc cac gag atg gtg cat      672
   Lys Gly Ala Gln Glu Ile Phe Lys Leu Met Pro His Glu Met Val His
       210                 215                 220
   ccg tcg gag ctg ccc aag cta ccg aag aac aac cgt att ctg tac ggc      720
   Pro Ser Glu Leu Pro Lys Leu Pro Lys Asn Asn Arg Ile Leu Tyr Gly
   225                 230                 235                 240
   aca gtg atc gac aac ccg gac tac ttc gtc aag ccc cag ccg gga ttc      768
   Thr Val Ile Asp Asn Pro Asp Tyr Phe Val Lys Pro Gln Pro Gly Phe
                   245                 250                 255
   agc ggc aca ccc tcc cgc gcg cac tac tac cag aac cca cac cag tac      816
   Ser Gly Thr Pro Ser Arg Ala His Tyr Tyr Gln Asn Pro His Gln Tyr
               260                 265                 270
   gag cct gct ttc cac gag aag gtg ctg ccc tac acg tcc atg ctg gtg      864
   Glu Pro Ala Phe His Glu Lys Val Leu Pro Tyr Thr Ser Met Leu Val
           275                 280                 285
   aac tgc atg tac tgg gac gac cgc ttc ccg cgg cta gtc acg cga gag      912
   Asn Cys Met Tyr Trp Asp Asp Arg Phe Pro Arg Leu Val Thr Arg Glu
       290                 295                 300
   cag atc cga gag ctt cga ggc tct ggg aac cat aag ctg ctg ggg atc      960
   Gln Ile Arg Glu Leu Arg Gly Ser Gly Asn His Lys Leu Leu Gly Ile
   305                 310                 315                 320
   gcg gac att tcg tgc gat att ggc ggc agc gtg gag ttc ttg gag cgc     1008
   Ala Asp Ile Ser Cys Asp Ile Gly Gly Ser Val Glu Phe Leu Glu Arg
                   325                 330                 335
   gcc acg gag atc gaa cga cca ttc gct ctg tac gat gtc gct gaa gac     1056
   Ala Thr Glu Ile Glu Arg Pro Phe Ala Leu Tyr Asp Val Ala Glu Asp
               340                 345                 350
   aag atg cgt gag gac ggc gac agt cga ggc ctt gaa ggc gac gat ggc      1104
   Lys Met Arg Glu Asp Gly Asp Ser Arg Gly Leu Glu Gly Asp Asp Gly
           355                 360                 365
   att atg atg atg ggc gtc gat atc ctg ccg agc gag ttg gcg cgc gag      1152
   Ile Met Met Met Gly Val Asp Ile Leu Pro Ser Glu Leu Ala Arg Glu
       370                 375                 380
   tca agc cag cag ttt ggt gac cgc ttg gtg gga tac gtc acg gcg cta      1200
   Ser Ser Gln Gln Phe Gly Asp Arg Leu Val Gly Tyr Val Thr Ala Leu
   385                 390                 395                 400
   tcg agt gtc act tca tcg aat gta cct ctg cac gag caa aag gag ctg      1248
   Ser Ser Val Thr Ser Ser Asn Val Pro Leu His Glu Gln Lys Glu Leu
                   405                 410                 415
   ccg gct gaa ctg cgt ggc gct tgt att gcc agc aaa ggt gtt ttg gcg      1296
   Pro Ala Glu Leu Arg Gly Ala Cys Ile Ala Ser Lys Gly Val Leu Ala
               420                 425                 430
   ccc agg tac gag tac att cat cgg atg cgt gcc gaa cgt gag cgc agc      1344
   Pro Arg Tyr Glu Tyr Ile His Arg Met Arg Ala Glu Arg Glu Arg Ser
           435                 440                 445
   aag cag ttc aag ttc ctg gat gct cag cag gag gtt gca ggc agc acg      1392
   Lys Gln Phe Lys Phe Leu Asp Ala Gln Gln Glu Val Ala Gly Ser Thr
       450                 455                 460
   tgc ctg tta ctg gaa gga cac ctc ttt gat aca ggc ctc atc aac cag      1440
   Cys Leu Leu Leu Glu Gly His Leu Phe Asp Thr Gly Leu Ile Asn Gln
   465                 470                 475                 480
   gta ctg aat ctc atc gag gac cac gat gga ggc ttc cac ctc gtg gac      1488
   Val Leu Asn Leu Ile Glu Asp His Asp Gly Gly Phe His Leu Val Asp
                   485                 490                 495
   tgc gag gtc cga ccg aac att gca gcc gga gac agc ggc ttc agc act      1536
   Cys Glu Val Arg Pro Asn Ile Ala Ala Gly Asp Ser Gly Phe Ser Thr
               500                 505                 510
   gtg tcg aat gct ata gtc cag gtt agc atg agt gac cgt gct gct ttg      1584
   Val Ser Asn Ala Ile Val Gln Val Ser Met Ser Asp Arg Ala Ala Leu
           515                 520                 525
   gac gac att att gcg aag atc cgc tcc ctt gcc gat ctt acg tct ggt      1632
   Asp Asp Ile Ile Ala Lys Ile Arg Ser Leu Ala Asp Leu Thr Ser Gly
       530                 535                 540
   gcc aag gcc atc gtc acg gag ctt ccc gac ttg tgc ggc acg aat tat      1680
   Ala Lys Ala Ile Val Thr Glu Leu Pro Asp Leu Cys Gly Thr Asn Tyr
   545                 550                 555                 560
   tcg aag act cgt ggt gcc gtg gtg cgc aag gat gct gct gct aat acg      1728
   Ser Lys Thr Arg Gly Ala Val Val Arg Lys Asp Ala Ala Ala Asn Thr
                   565                 570                 575
   gcg gcg gac gtg tca gtc tcg agc ccg aag aag cga cag atc gtg tgt      1776
   Ala Ala Asp Val Ser Val Ser Ser Pro Lys Lys Arg Gln Ile Val Cys
               580                 585                 590
   ttt ggc gct gga tta gtg gca tcc ccg ctt gtt gag tac ctg tct cgc      1824
   Phe Gly Ala Gly Leu Val Ala Ser Pro Leu Val Glu Tyr Leu Ser Arg
           595                 600                 605
   gag caa ggc aat gaa gtg cac gtg gtg tcc ggt atc gag ggt gaa gtg        1872
   Glu Gln Gly Asn Glu Val His Val Val Ser Gly Ile Glu Gly Glu Val
       610                 615                 620
   aag ggg gtg atg cgc aaa atc tct cgc cgt aac att aag cct cac gtg        1920
   Lys Gly Val Met Arg Lys Ile Ser Arg Arg Asn Ile Lys Pro His Val
   625                 630                 635                 640
   gtg aac gta gct gag gac ggt gct ggc gtt gac aag ctc tgt gca gaa        1968
   Val Asn Val Ala Glu Asp Gly Ala Gly Val Asp Lys Leu Cys Ala Glu
                   645                 650                 655
   gcc gat tgt gtt gtg tca cta tta cca gct aca atg cac aca acc atc        2016
   Ala Asp Cys Val Val Ser Leu Leu Pro Ala Thr Met His Thr Thr Ile
               660                 665                 670
   gcc cag cgt tgc atc caa cat gga acg cct ctc gtg act gca agc tac        2064
   Ala Gln Arg Cys Ile Gln His Gly Thr Pro Leu Val Thr Ala Ser Tyr
           675                 680                 685
   gtg tct ccg gag atg aaa gag ttg gat gcc aaa gcc aag aaa gcc ggc        2112
   Val Ser Pro Glu Met Lys Glu Leu Asp Ala Lys Ala Lys Lys Ala Gly
       690                 695                 700
   att cca ata ctg tgt gaa att ggc ctt gat cct ggc atg gat cac atg        2160
   Ile Pro Ile Leu Cys Glu Ile Gly Leu Asp Pro Gly Met Asp His Met
   705                 710                 715                 720
   agc gct atg aag gtc atc gac gaa gtg aag gct cac tcc gga aag atc        2208
   Ser Ala Met Lys Val Ile Asp Glu Val Lys Ala His Ser Gly Lys Ile
                   725                 730                 735
   ctg tca ttt tca tcc gtg tgt ggt gga ctt cca gct cct gaa gct gct        2256
   Leu Ser Phe Ser Ser Val Cys Gly Gly Leu Pro Ala Pro Glu Ala Ala
               740                 745                 750
   gac aac gcc att ggt tac aag ttc agc tgg agt ccg cgc ggc gtg ctt        2304
   Asp Asn Ala Ile Gly Tyr Lys Phe Ser Trp Ser Pro Arg Gly Val Leu
           755                 760                 765
   act gca gca ctg aac gcg gcg caa tac cgc aaa gac ggc aag atc gtc        2352
   Thr Ala Ala Leu Asn Ala Ala Gln Tyr Arg Lys Asp Gly Lys Ile Val
       770                 775                 780
   aac gtc gca ggc gaa gac ttg ctt aac agc agc gaa cct gtg aac ttc        2400
   Asn Val Ala Gly Glu Asp Leu Leu Asn Ser Ser Glu Pro Val Asn Phe
   785                 790                 795                 800
   ttg cct gcg ttt aac atc gag cag atc ccg aac cgt gac tca ctg ccg        2448
   Leu Pro Ala Phe Asn Ile Glu Gln Ile Pro Asn Arg Asp Ser Leu Pro
                   805                 810                 815
   tac ggc gag atc tac gtc att cct gag gcg cac tcg ctg tat cgt gga        2496
   Tyr Gly Glu Ile Tyr Val Ile Pro Glu Ala His Ser Leu Tyr Arg Gly
               820                 825                 830
   aca ctg cgc tac gga ggt tgt tgc cga atc ctg tac cag ctg cgc aag        2544
   Thr Leu Arg Tyr Gly Gly Cys Cys Arg Ile Leu Tyr Gln Leu Arg Lys
           835                 840                 845
   ctt ggt ctc ttt gac atg gac ccg tcc aag ccc att cca gct acg tgg        2592
   Leu Gly Leu Phe Asp Met Asp Pro Ser Lys Pro Ile Pro Ala Thr Trp
       850                 855                 860
   cca gac ctt atc tcc cag ctt ggt gga cac caa ggc ctt cgc gaa gac        2640
   Pro Asp Leu Ile Ser Gln Leu Gly Gly His Gln Gly Leu Arg Glu Asp
   865                 870                 875                 880
   gcc aat gga ttc ctt caa tgg ctc ggt gcc ttc gat cac agc act ccc        2688
   Ala Asn Gly Phe Leu Gln Trp Leu Gly Ala Phe Asp His Ser Thr Pro
                   885                 890                 895
   gta gtg cga gcc cca tcc atc ctc gac gca ttc tgc gca ctg ttg cag        2736
   Val Val Arg Ala Pro Ser Ile Leu Asp Ala Phe Cys Ala Leu Leu Gln
               900                 905                 910
   gac aag tta tct tac caa cct gga gaa cgt gat atg gcc atc atg cac        2784
   Asp Lys Leu Ser Tyr Gln Pro Gly Glu Arg Asp Met Ala Ile Met His
           915                 920                 925
   cat gaa ttc ggc atc gag tac gaa gac ggc aag aag gaa aag cgc acg        2832
   His Glu Phe Gly Ile Glu Tyr Glu Asp Gly Lys Lys Glu Lys Arg Thr
       930                 935                 940
   tca acg ttc gtg ggt tac ggc tcc gat aag ggc gac acc atc atg gct        2880
   Ser Thr Phe Val Gly Tyr Gly Ser Asp Lys Gly Asp Thr Ile Met Ala
   945                 950                 955                 960
   aaa acc gtt ggg ttg agt gca gcg att ggt gtg cag ttg atc ttg cag        2928
   Lys Thr Val Gly Leu Ser Ala Ala Ile Gly Val Gln Leu Ile Leu Gln
                   965                 970                 975
   gac gca gtc caa ggc cga ggc gtg ctt aca ccg acg acc cct gac atc        2976
   Asp Ala Val Gln Gly Arg Gly Val Leu Thr Pro Thr Thr Pro Asp Ile
               980                 985                 990
   tac ggg cca gcc ctc gcg cga ctt  gag gtt gaa ggt gtt  cgc ttc atc      3024
   Tyr Gly Pro Ala Leu Ala Arg Leu  Glu Val Glu Gly Val  Arg Phe Ile
           995                 1000                1005
   gaa aag  acg ttc ccg cag cct  taa                                      3048
   Glu Lys  Thr Phe Pro Gln Pro
       1010                1015
<210> 28
   <211> 1015
   <212> PRT
   <213> Phytophthora sojae
<400> 28
   Met Thr Gly Lys Cys Val Gly Ile Val Arg Glu Val Tyr Asn Lys Trp
   1               5                   10                  15
   Glu Arg Arg Ala Pro Leu Thr Pro Ala His Val Arg Glu Leu Val Gln
               20                  25                  30
   Arg Gly Ile Gln Val Leu Val Gln Pro Ser Thr Ala Arg Val Phe Ser
           35                  40                  45
   Asp Glu Gln Tyr Val Arg Ala Gly Ala Thr Leu Ser Glu Asp Leu Ala
       50                  55                  60
   Pro Ala Asn Val Ile Val Gly Val Lys Gln Val Pro Glu Pro Ala Leu
   65                  70                  75                  80
   Leu Ala Asp Lys Thr Tyr Leu Phe Phe Ser His Thr Ile Lys Ala Gln
                   85                  90                  95
   Pro Glu Asn Met Ala Leu Leu Asp Ala Val Leu Gln Arg Arg Ile Thr
               100                 105                 110
   Leu Val Asp Tyr Glu Cys Ile Thr Glu Glu Ser Gly Lys Arg Leu Ile
           115                 120                 125
   Ala Phe Gly Gly Asn Ala Gly Arg Ala Gly Met Ile Ala Gly Phe Arg
       130                 135                 140
   Gly Leu Gly Glu Arg Leu Ile Asn Met Gly Val Ser Ser Pro Phe Val
   145                 150                 155                 160
   Asn Val Ala Ser Ala Tyr Met Tyr Ser Asp Leu Glu His Ala Lys Asp
                   165                 170                 175
   Ala Val Glu Ala Ala Gly Lys Arg Ile Arg Ser Asp Gly Leu Pro Ser
               180                 185                 190
   Glu Leu Val Pro Met Thr Phe Ala Phe Thr Gly Asn Gly Asn Val Ser
           195                 200                 205
   Lys Gly Ala Gln Glu Ile Phe Lys Leu Met Pro His Glu Met Val His
       210                 215                 220
   Pro Ser Glu Leu Pro Lys Leu Pro Lys Asn Asn Arg Ile Leu Tyr Gly
   225                 230                 235                 240
   Thr Val Ile Asp Asn Pro Asp Tyr Phe Val Lys Pro Gln Pro Gly Phe
                   245                 250                 255
   Ser Gly Thr Pro Ser Arg Ala His Tyr Tyr Gln Asn Pro His Gln Tyr
               260                 265                 270
   Glu Pro Ala Phe His Glu Lys Val Leu Pro Tyr Thr Ser Met Leu Val
           275                 280                 285
   Asn Cys Met Tyr Trp Asp Asp Arg Phe Pro Arg Leu Val Thr Arg Glu
       290                 295                 300
   Gln Ile Arg Glu Leu Arg Gly Ser Gly Asn His Lys Leu Leu Gly Ile
   305                 310                 315                 320
   Ala Asp Ile Ser Cys Asp Ile Gly Gly Ser Val Glu Phe Leu Glu Arg
                   325                 330                 335
   Ala Thr Glu Ile Glu Arg Pro Phe Ala Leu Tyr Asp Val Ala Glu Asp
   340 345 350
   Lys Met Arg Glu Asp Gly Asp Ser Arg Gly Leu Glu Gly Asp Asp Gly
   355 360 365
   Ile Met Met Met Gly Val Asp Ile Leu Pro Ser Glu Leu Ala Arg Glu
   370 375 380
   Ser Ser Gln Gln Phe Gly Asp Arg Leu Val Gly Tyr Val Thr Ala Leu
   385 390 395 400
   Ser Ser Val Thr Ser Ser Asn Val Pro Leu His Glu Gln Lys Glu Leu
   405 410 415
   Pro Ala Glu Leu Arg Gly Ala Cys Ile Ala Ser Lys Gly Val Leu Ala
   420 425 430
   Pro Arg Tyr Glu Tyr Ile His Arg Met Arg Ala Glu Arg Glu Arg Ser
   435 440 445
   Lys Gln Phe Lys Phe Leu Asp Ala Gln Gln Glu Val Ala Gly Ser Thr
   450 455 460
   Cys Leu Leu Leu Glu Gly His Leu Phe Asp Thr Gly Leu Ile Asn Gln
   465 470 475 480
   Val Leu Asn Leu Ile Glu Asp His Asp Gly Gly Phe His Leu Val Asp
   485 490 495
   Cys Glu Val Arg Pro Asn Ile Ala Ala Gly Asp Ser Gly Phe Ser Thr
   500 505 510
   Val Ser Asn Ala Ile Val Gln Val Ser Met Ser Asp Arg Ala Ala Leu
   515 520 525
   Asp Asp Ile Ile Ala Lys Ile Arg Ser Leu Ala Asp Leu Thr Ser Gly
   530 535 540
   Ala Lys Ala Ile Val Thr Glu Leu Pro Asp Leu Cys Gly Thr Asn Tyr
   545 550 555 560
   Ser Lys Thr Arg Gly Ala Val Val Arg Lys Asp Ala Ala Ala Asn Thr
   565 570 575
   Ala Ala Asp Val Ser Val Ser Ser Pro Lys Lys Arg Gln Ile Val Cys
   580 585 590
   Phe Gly Ala Gly Leu Val Ala Ser Pro Leu Val Glu Tyr Leu Ser Arg
   595 600 605
   Glu Gln Gly Asn Glu Val His Val Val Ser Gly Ile Glu Gly Glu Val
   610 615 620
   Lys Gly Val Met Arg Lys Ile Ser Arg Arg Asn Ile Lys Pro His Val
   625 630 635 640
   Val Asn Val Ala Glu Asp Gly Ala Gly Val Asp Lys Leu Cys Ala Glu
   645 650 655
   Ala Asp Cys Val Val Ser Leu Leu Pro Ala Thr Met His Thr Thr Ile
   660 665 670
   Ala Gln Arg Cys Ile Gln His Gly Thr Pro Leu Val Thr Ala Ser Tyr
   675 680 685
   Val Ser Pro Glu Met Lys Glu Leu Asp Ala Lys Ala Lys Lys Ala Gly
   690 695 700
   Ile Pro Ile Leu Cys Glu Ile Gly Leu Asp Pro Gly Met Asp His Met
   705 710 715 720
   Ser Ala Met Lys Val Ile Asp Glu Val Lys Ala His Ser Gly Lys Ile
   725 730 735
   Leu Ser Phe Ser Ser Val Cys Gly Gly Leu Pro Ala Pro Glu Ala Ala
   740 745 750
   Asp Asn Ala Ile Gly Tyr Lys Phe Ser Trp Ser Pro Arg Gly Val Leu
   755 760 765
   Thr Ala Ala Leu Asn Ala Ala Gln Tyr Arg Lys Asp Gly Lys Ile Val
   770 775 780
   Asn Val Ala Gly Glu Asp Leu Leu Asn Ser Ser Glu Pro Val Asn Phe
   785 790 795 800
   Leu Pro Ala Phe Asn Ile Glu Gln Ile Pro Asn Arg Asp Ser Leu Pro
   805 810 815
   Tyr Gly Glu Ile Tyr Val Ile Pro Glu Ala His Ser Leu Tyr Arg Gly
   820 825 830
   Thr Leu Arg Tyr Gly Gly Cys Cys Arg Ile Leu Tyr Gln Leu Arg Lys
   835 840 845
   Leu Gly Leu Phe Asp Met Asp Pro Ser Lys Pro Ile Pro Ala Thr Trp
       850                 855                 860
   Pro Asp Leu Ile Ser Gln Leu Gly Gly His Gln Gly Leu Arg Glu Asp
   865                 870                 875                 880
   Ala Asn Gly Phe Leu Gln Trp Leu Gly Ala Phe Asp His Ser Thr Pro
                   885                 890                 895
   Val Val Arg Ala Pro Ser Ile Leu Asp Ala Phe Cys Ala Leu Leu Gln
               900                 905                 910
   Asp Lys Leu Ser Tyr Gln Pro Gly Glu Arg Asp Met Ala Ile Met His
           915                 920                 925
   His Glu Phe Gly Ile Glu Tyr Glu Asp Gly Lys Lys Glu Lys Arg Thr
       930                 935                 940
   Ser Thr Phe Val Gly Tyr Gly Ser Asp Lys Gly Asp Thr Ile Met Ala
   945                 950                 955                 960
   Lys Thr Val Gly Leu Ser Ala Ala Ile Gly Val Gln Leu Ile Leu Gln
                   965                 970                 975
   Asp Ala Val Gln Gly Arg Gly Val Leu Thr Pro Thr Thr Pro Asp Ile
               980                 985                 990
   Tyr Gly Pro Ala Leu Ala Arg Leu  Glu Val Glu Gly Val  Arg Phe Ile
           995                 1000                1005
   Glu Lys  Thr Phe Pro Gln Pro
       1010                1015
<210> 29
   <211> 1164
   <212> DNA
   <213> Pyrenophora tritici-repentis
<220>
   <221> CDS
   <222> (1)..(1164)
   <223> Lys1
<400> 29
   atg gct ttc ccc aca cta cat gcc cgc gca gag gct aaa cct ctt gaa        48
   Met Ala Phe Pro Thr Leu His Ala Arg Ala Glu Ala Lys Pro Leu Glu
   1               5                   10                  15
   cac cgc tcg tgt ctc aca ccc aca acc gca aag aag ctg ctc gat gct        96
   His Arg Ser Cys Leu Thr Pro Thr Thr Ala Lys Lys Leu Leu Asp Ala
               20                  25                  30
   ggc tac cct gtc ctc gtc gag cgc tcg ccc aag gac ccc aac tac gcc       144
   Gly Tyr Pro Val Leu Val Glu Arg Ser Pro Lys Asp Pro Asn Tyr Ala
           35                  40                  45
   cgt atc ttc aaa gac gac gag ttc gag gag gta ggc gcg acg ctc atc      192
   Arg Ile Phe Lys Asp Asp Glu Phe Glu Glu Val Gly Ala Thr Leu Ile
       50                  55                  60
   gag gaa ggc gca tac aag acg gcc ccc aag gac cgc atc atc atc ggc      240
   Glu Glu Gly Ala Tyr Lys Thr Ala Pro Lys Asp Arg Ile Ile Ile Gly
   65                  70                  75                  80
   cta aag gag ctg cca gag gac aag ttc ccg ctc gag cac aca ttc gta      288
   Leu Lys Glu Leu Pro Glu Asp Lys Phe Pro Leu Glu His Thr Phe Val
                   85                  90                  95
   cac ttt gca cat tgc tac aag cag caa ggc gga tgg gaa aac gta ttg      336
   His Phe Ala His Cys Tyr Lys Gln Gln Gly Gly Trp Glu Asn Val Leu
               100                 105                 110
   gcg agg ttc ccc cgt ggc ggt ggc aca ttg tat gac ctc gag ttc ctg      384
   Ala Arg Phe Pro Arg Gly Gly Gly Thr Leu Tyr Asp Leu Glu Phe Leu
           115                 120                 125
   caa gat gag tca ggc agg cgc gtt gct gcc ttt ggc tac cac gcc ggt      432
   Gln Asp Glu Ser Gly Arg Arg Val Ala Ala Phe Gly Tyr His Ala Gly
       130                 135                 140
   ttc gtc ggc gct gct ctt gcc atc aag aca tgg gct tgg caa ctc acc      480
   Phe Val Gly Ala Ala Leu Ala Ile Lys Thr Trp Ala Trp Gln Leu Thr
   145                 150                 155                 160
   cat ccc aac ggc gag ccc ctt cct ggt cta gag acc ttc act gag gga      528
   His Pro Asn Gly Glu Pro Leu Pro Gly Leu Glu Thr Phe Thr Glu Gly
                   165                 170                 175
   cgc ggc tac tac aac aac gag agc gag ttg att acc caa ctc aag gaa      576
   Arg Gly Tyr Tyr Asn Asn Glu Ser Glu Leu Ile Thr Gln Leu Lys Glu
               180                 185                 190
   gat gtc gca gcc ggc gaa aag atc gca gga cac aag ccc agc agt ctg      624
   Asp Val Ala Ala Gly Glu Lys Ile Ala Gly His Lys Pro Ser Ser Leu
           195                 200                 205
   gtg cta ggt gct ctc ggc cgc tgc ggc tct ggt gcc gtc gac ctc ctt      672
   Val Leu Gly Ala Leu Gly Arg Cys Gly Ser Gly Ala Val Asp Leu Leu
       210                 215                 220
   gag aag att ggc tgc cct gag atc aag aag tgg gat ctt gcc gag acc      720
   Glu Lys Ile Gly Cys Pro Glu Ile Lys Lys Trp Asp Leu Ala Glu Thr
   225                 230                 235                 240
   aag gag cgc gac ggc cca tac ccc gaa att gtc gaa tct gac atc ttc      768
   Lys Glu Arg Asp Gly Pro Tyr Pro Glu Ile Val Glu Ser Asp Ile Phe
                   245                 250                 255
   gtc aac tgc atc tac ctt tcc aag ccc atc ccg cct ttt gtc aac aag      816
   Val Asn Cys Ile Tyr Leu Ser Lys Pro Ile Pro Pro Phe Val Asn Lys
               260                 265                 270
   gag agt ctc aag tcg cca aac cgc aga ctc agc gtc gtt tgc gat gtc      864
   Glu Ser Leu Lys Ser Pro Asn Arg Arg Leu Ser Val Val Cys Asp Val
           275                 280                 285
   agc tgt gac act act aac cct cat aac ccc att ccc atc tac gat atc      912
   Ser Cys Asp Thr Thr Asn Pro His Asn Pro Ile Pro Ile Tyr Asp Ile
       290                 295                 300
   aac acc acc ttt gac aag ccc aca gtt gag gtt tct gtc gag ggt gac           960
   Asn Thr Thr Phe Asp Lys Pro Thr Val Glu Val Ser Val Glu Gly Asp
   305                 310                 315                 320
   ggt cct aga cta tct gtc atc tcc att gac cac ctt ccc tct gcg ttg          1008
   Gly Pro Arg Leu Ser Val Ile Ser Ile Asp His Leu Pro Ser Ala Leu
                   325                 330                 335
   cct cgt gag tct tca gaa gcc ttt agc aac gct ctg ctg cct agc ttg          1056
   Pro Arg Glu Ser Ser Glu Ala Phe Ser Asn Ala Leu Leu Pro Ser Leu
               340                 345                 350
   atg gct ctg aag gac cgc gcg aca acg cct gtg tgg cag ggt gct gag          1104
   Met Ala Leu Lys Asp Arg Ala Thr Thr Pro Val Trp Gln Gly Ala Glu
           355                 360                 365
   aag ctc ttc caa gag aag gtt cag acg ctg ccg ggt ggt gtc ccc gca          1152
   Lys Leu Phe Gln Glu Lys Val Gln Thr Leu Pro Gly Gly Val Pro Ala
       370                 375                 380
   aag gag gta tag                                                          1164
   Lys Glu Val
   385
<210> 30
   <211> 387
   <212> PRT
   <213> Pyrenophora tritici-repentis
<400> 30
   Met Ala Phe Pro Thr Leu His Ala Arg Ala Glu Ala Lys Pro Leu Glu
   1               5                   10                  15
   His Arg Ser Cys Leu Thr Pro Thr Thr Ala Lys Lys Leu Leu Asp Ala
               20                  25                  30
   Gly Tyr Pro Val Leu Val Glu Arg Ser Pro Lys Asp Pro Asn Tyr Ala
           35                  40                  45
   Arg Ile Phe Lys Asp Asp Glu Phe Glu Glu Val Gly Ala Thr Leu Ile
       50                  55                  60
   Glu Glu Gly Ala Tyr Lys Thr Ala Pro Lys Asp Arg Ile Ile Ile Gly
   65                  70                  75                  80
   Leu Lys Glu Leu Pro Glu Asp Lys Phe Pro Leu Glu His Thr Phe Val
                   85                  90                  95
   His Phe Ala His Cys Tyr Lys Gln Gln Gly Gly Trp Glu Asn Val Leu
               100                 105                 110
   Ala Arg Phe Pro Arg Gly Gly Gly Thr Leu Tyr Asp Leu Glu Phe Leu
           115                 120                 125
   Gln Asp Glu Ser Gly Arg Arg Val Ala Ala Phe Gly Tyr His Ala Gly
       130                 135                 140
   Phe Val Gly Ala Ala Leu Ala Ile Lys Thr Trp Ala Trp Gln Leu Thr
   145                 150                 155                 160
   His Pro Asn Gly Glu Pro Leu Pro Gly Leu Glu Thr Phe Thr Glu Gly
                   165                 170                 175
   Arg Gly Tyr Tyr Asn Asn Glu Ser Glu Leu Ile Thr Gln Leu Lys Glu
               180                 185                 190
   Asp Val Ala Ala Gly Glu Lys Ile Ala Gly His Lys Pro Ser Ser Leu
           195                 200                 205
   Val Leu Gly Ala Leu Gly Arg Cys Gly Ser Gly Ala Val Asp Leu Leu
       210                 215                 220
   Glu Lys Ile Gly Cys Pro Glu Ile Lys Lys Trp Asp Leu Ala Glu Thr
   225                 230                 235                 240
   Lys Glu Arg Asp Gly Pro Tyr Pro Glu Ile Val Glu Ser Asp Ile Phe
                   245                 250                 255
   Val Asn Cys Ile Tyr Leu Ser Lys Pro Ile Pro Pro Phe Val Asn Lys
               260                 265                 270
   Glu Ser Leu Lys Ser Pro Asn Arg Arg Leu Ser Val Val Cys Asp Val
           275                 280                 285
   Ser Cys Asp Thr Thr Asn Pro His Asn Pro Ile Pro Ile Tyr Asp Ile
       290                 295                 300
   Asn Thr Thr Phe Asp Lys Pro Thr Val Glu Val Ser Val Glu Gly Asp
   305                 310                 315                 320
   Gly Pro Arg Leu Ser Val Ile Ser Ile Asp His Leu Pro Ser Ala Leu
                   325                 330                 335
   Pro Arg Glu Ser Ser Glu Ala Phe Ser Asn Ala Leu Leu Pro Ser Leu
               340                 345                 350
   Met Ala Leu Lys Asp Arg Ala Thr Thr Pro Val Trp Gln Gly Ala Glu
           355                 360                 365
   Lys Leu Phe Gln Glu Lys Val Gln Thr Leu Pro Gly Gly Val Pro Ala
       370                 375                 380
   Lys Glu Val
   385
<210> 31
   <211> 1119
   <212> DNA
   <213> Sclerotinia sclerotiorum
<220>
   <221> CDS
   <222> (1)..(1119)
   <223> Lys1
<400> 31
   atg tct gga act act ctt cac cta cgt tct gag ttg gga aaa gct ctt        48
   Met Ser Gly Thr Thr Leu His Leu Arg Ser Glu Leu Gly Lys Ala Leu
   1               5                   10                  15
   gag cat aga tca gct ctt acc ccc act aca gcc aaa gct ctc atc gat        96
   Glu His Arg Ser Ala Leu Thr Pro Thr Thr Ala Lys Ala Leu Ile Asp
               20                  25                  30
   gct gga tac aca att aat gtc gag cgc agc cca gaa cgt ata ttc gac       144
   Ala Gly Tyr Thr Ile Asn Val Glu Arg Ser Pro Glu Arg Ile Phe Asp
           35                  40                  45
   gat gag gag ttc gag aag gtt gga gct act ctc gtg cca gaa gat aca       192
   Asp Glu Glu Phe Glu Lys Val Gly Ala Thr Leu Val Pro Glu Asp Thr
       50                  55                  60
   tgg aga caa gca cca aag gat cat att att att ggt ttg aag gaa cta       240
   Trp Arg Gln Ala Pro Lys Asp His Ile Ile Ile Gly Leu Lys Glu Leu
   65                  70                  75                  80
   cct gtc gaa gac ttt ccc ctc gag cat gtc cac gta caa ttc gca cac       288
   Pro Val Glu Asp Phe Pro Leu Glu His Val His Val Gln Phe Ala His
                   85                  90                  95
   tgt tac aaa caa caa ggt ggc tgg gac acc gtt cta tca cga ttt cct       336
   Cys Tyr Lys Gln Gln Gly Gly Trp Asp Thr Val Leu Ser Arg Phe Pro
               100                 105                 110
   cgc ggc ggc gga act ctc tta gat ctc gaa ttt ttg aca gac gat aaa       384
   Arg Gly Gly Gly Thr Leu Leu Asp Leu Glu Phe Leu Thr Asp Asp Lys
           115                 120                 125
   ggc aga aga gtt gca gct ttc gga tac cac gct gga ttt gct ggt gca       432
   Gly Arg Arg Val Ala Ala Phe Gly Tyr His Ala Gly Phe Ala Gly Ala
       130                 135                 140
   gca ttg gca ctc gaa act tgg gca tgg caa ctc act cat tca gca tca       480
   Ala Leu Ala Leu Glu Thr Trp Ala Trp Gln Leu Thr His Ser Ala Ser
   145                 150                 155                 160
   gag cca ttc cca agc gta tca agc tat cct aac gaa gat gag ttg att       528
   Glu Pro Phe Pro Ser Val Ser Ser Tyr Pro Asn Glu Asp Glu Leu Ile
                   165                 170                 175
   gcc gac gta aag aaa gca atc gca gct gga caa gaa aag aca ggc aag       576
   Ala Asp Val Lys Lys Ala Ile Ala Ala Gly Gln Glu Lys Thr Gly Lys
               180                 185                 190
   gca cca cga gtt ttg gtt atc gga gca ttg ggc aga tgt gga agt gga       624
   Ala Pro Arg Val Leu Val Ile Gly Ala Leu Gly Arg Cys Gly Ser Gly
           195                 200                 205
   gca gtt gat ctc tgc ttg aga gcc ggt gtg cca acc gag aat gtc ttg        672
   Ala Val Asp Leu Cys Leu Arg Ala Gly Val Pro Thr Glu Asn Val Leu
       210                 215                 220
   aag tgg gat atg gca gag act gcc aag gga gga cca ttt cca gag att        720
   Lys Trp Asp Met Ala Glu Thr Ala Lys Gly Gly Pro Phe Pro Glu Ile
   225                 230                 235                 240
   gtc gag agt gac atc ttc att aac tgc ata tac ctc atg tcc aag att        768
   Val Glu Ser Asp Ile Phe Ile Asn Cys Ile Tyr Leu Met Ser Lys Ile
                   245                 250                 255
   ccg aac ttt gtc gac atg caa agc ctc gat acc cca aac cgc aaa ttg        816
   Pro Asn Phe Val Asp Met Gln Ser Leu Asp Thr Pro Asn Arg Lys Leu
               260                 265                 270
   tca gtc gtc tgc gac gtc agt gct gat acc aca aat ccc aac aat cca        864
   Ser Val Val Cys Asp Val Ser Ala Asp Thr Thr Asn Pro Asn Asn Pro
           275                 280                 285
   att cca atc tat acc gtt gcg acc aca ttt tca gag cca act gtc cca        912
   Ile Pro Ile Tyr Thr Val Ala Thr Thr Phe Ser Glu Pro Thr Val Pro
       290                 295                 300
   gtc gag gtc aag gga gac ccc aga cta agc gtc atc agc att gat cac        960
   Val Glu Val Lys Gly Asp Pro Arg Leu Ser Val Ile Ser Ile Asp His
   305                 310                 315                 320
   tta cca agt cta ttg cca aga gaa gca agc gag gca ttc agc aag gac       1008
   Leu Pro Ser Leu Leu Pro Arg Glu Ala Ser Glu Ala Phe Ser Lys Asp
                   325                 330                 335
   ttg cta cca agt ttg ttg aca tta aag gac tgg aga agt act cca gtc       1056
   Leu Leu Pro Ser Leu Leu Thr Leu Lys Asp Trp Arg Ser Thr Pro Val
               340                 345                 350
   tgg gcg aag gcc gag aag ttg ttt cag gag aag gtt gcc aca ttg cct       1104
   Trp Ala Lys Ala Glu Lys Leu Phe Gln Glu Lys Val Ala Thr Leu Pro
           355                 360                 365
   aag aag gag gca tga                                                   1119
   Lys Lys Glu Ala
       370
<210> 32
   <211> 372
   <212> PRT
   <213> Sclerotinia sclerotiorum
<400> 32
   Met Ser Gly Thr Thr Leu His Leu Arg Ser Glu Leu Gly Lys Ala Leu
   1               5                   10                  15
   Glu His Arg Ser Ala Leu Thr Pro Thr Thr Ala Lys Ala Leu Ile Asp
               20                  25                  30
   Ala Gly Tyr Thr Ile Asn Val Glu Arg Ser Pro Glu Arg Ile Phe Asp
           35                  40                  45
   Asp Glu Glu Phe Glu Lys Val Gly Ala Thr Leu Val Pro Glu Asp Thr
       50                  55                  60
   Trp Arg Gln Ala Pro Lys Asp His Ile Ile Ile Gly Leu Lys Glu Leu
   65                  70                  75                  80
   Pro Val Glu Asp Phe Pro Leu Glu His Val His Val Gln Phe Ala His
                   85                  90                  95
   Cys Tyr Lys Gln Gln Gly Gly Trp Asp Thr Val Leu Ser Arg Phe Pro
               100                 105                 110
   Arg Gly Gly Gly Thr Leu Leu Asp Leu Glu Phe Leu Thr Asp Asp Lys
           115                 120                 125
   Gly Arg Arg Val Ala Ala Phe Gly Tyr His Ala Gly Phe Ala Gly Ala
       130                 135                 140
   Ala Leu Ala Leu Glu Thr Trp Ala Trp Gln Leu Thr His Ser Ala Ser
   145                 150                 155                 160
   Glu Pro Phe Pro Ser Val Ser Ser Tyr Pro Asn Glu Asp Glu Leu Ile
                   165                 170                 175
   Ala Asp Val Lys Lys Ala Ile Ala Ala Gly Gln Glu Lys Thr Gly Lys
               180                 185                 190
   Ala Pro Arg Val Leu Val Ile Gly Ala Leu Gly Arg Cys Gly Ser Gly
           195                 200                 205
   Ala Val Asp Leu Cys Leu Arg Ala Gly Val Pro Thr Glu Asn Val Leu
       210                 215                 220
   Lys Trp Asp Met Ala Glu Thr Ala Lys Gly Gly Pro Phe Pro Glu Ile
   225                 230                 235                 240
   Val Glu Ser Asp Ile Phe Ile Asn Cys Ile Tyr Leu Met Ser Lys Ile
                   245                 250                 255
   Pro Asn Phe Val Asp Met Gln Ser Leu Asp Thr Pro Asn Arg Lys Leu
               260                 265                 270
   Ser Val Val Cys Asp Val Ser Ala Asp Thr Thr Asn Pro Asn Asn Pro
           275                 280                 285
   Ile Pro Ile Tyr Thr Val Ala Thr Thr Phe Ser Glu Pro Thr Val Pro
       290                 295                 300
   Val Glu Val Lys Gly Asp Pro Arg Leu Ser Val Ile Ser Ile Asp His
   305                 310                 315                 320
   Leu Pro Ser Leu Leu Pro Arg Glu Ala Ser Glu Ala Phe Ser Lys Asp
                   325                 330                 335
   Leu Leu Pro Ser Leu Leu Thr Leu Lys Asp Trp Arg Ser Thr Pro Val
               340                 345                 350
   Trp Ala Lys Ala Glu Lys Leu Phe Gln Glu Lys Val Ala Thr Leu Pro
           355                 360                 365
   Lys Lys Glu Ala
       370
<210> 33
   <211> 1170
   <212> DNA
   <213> Trichoderma reesei
<220>
   <221> CDS
   <222> (1)..(1170)
   <223> Lys1
<400> 33
   gtt caa cta ttt tcc ccc tca tta tca tac cac aaa aaa caa caa caa      48
   Val Gln Leu Phe Ser Pro Ser Leu Ser Tyr His Lys Lys Gln Gln Gln
   1               5                   10                  15
   tca att cgt gcc aaa atg cct acc gtc atc cat ctg aga gcc gat acc      96
   Ser Ile Arg Ala Lys Met Pro Thr Val Ile His Leu Arg Ala Asp Thr
               20                  25                  30
   aag ccg ttt gag cgt cgc tcg ccg ctg tcc ccc cca acc gcc aag gcc     144
   Lys Pro Phe Glu Arg Arg Ser Pro Leu Ser Pro Pro Thr Ala Lys Ala
           35                  40                  45
   ctc ctc gac gct ggc tac gtc gtc cgc gtt gag cga tct tca gag cgc     192
   Leu Leu Asp Ala Gly Tyr Val Val Arg Val Glu Arg Ser Ser Glu Arg
       50                  55                  60
   atc tac aag gac gaa gag ttc gag gcc gtt ggc gcc gaa ttg gtc cct     240
   Ile Tyr Lys Asp Glu Glu Phe Glu Ala Val Gly Ala Glu Leu Val Pro
   65                  70                  75                  80
   gcc gga tca tgg atc aag gct ccc aag gag gac atc atc ctg ggc ctc     288
   Ala Gly Ser Trp Ile Lys Ala Pro Lys Glu Asp Ile Ile Leu Gly Leu
                   85                  90                  95
   aag gag ctg ccc gac gac gac att gac ctg cct cac aca tac atc cac     336
   Lys Glu Leu Pro Asp Asp Asp Ile Asp Leu Pro His Thr Tyr Ile His
               100                 105                 110
   ttc cag cac atc ttc aag aag cag ctc ggc tgg gcg ccc tcc ctg aag     384
   Phe Gln His Ile Phe Lys Lys Gln Leu Gly Trp Ala Pro Ser Leu Lys
           115                 120                 125
   cgc ttt gct cgc gcc ggc ggc acg ctc tac gac ctg gaa ttc ctg act        432
   Arg Phe Ala Arg Ala Gly Gly Thr Leu Tyr Asp Leu Glu Phe Leu Thr
       130                 135                 140
   gag gag aac ggc cgc aga atc gcg gcc ttt ggc tac ttt gct gga tat        480
   Glu Glu Asn Gly Arg Arg Ile Ala Ala Phe Gly Tyr Phe Ala Gly Tyr
   145                 150                 155                 160
   gcc ggc gcc gcc att gcc ttc atc tcg tgg gct cac cag atc ctc aac        528
   Ala Gly Ala Ala Ile Ala Phe Ile Ser Trp Ala His Gln Ile Leu Asn
                   165                 170                 175
   ccc ggc gtc ccc cag ccc ccg gtg cct ctg ttc gac agc gcg cct gct        576
   Pro Gly Val Pro Gln Pro Pro Val Pro Leu Phe Asp Ser Ala Pro Ala
               180                 185                 190
   ctg gtc gcg cat gtc aag gct gcg ctc gag ccc gcg att cgt gcc aac        624
   Leu Val Ala His Val Lys Ala Ala Leu Glu Pro Ala Ile Arg Ala Asn
           195                 200                 205
   aac ggc caa ctt ccc cgc gtc att gtg att ggc gct ctg gga cga tgc        672
   Asn Gly Gln Leu Pro Arg Val Ile Val Ile Gly Ala Leu Gly Arg Cys
       210                 215                 220
   ggc aag gga gcc gtg gac ttt tgc cgc gag gtt ggt ctt cct gag gac        720
   Gly Lys Gly Ala Val Asp Phe Cys Arg Glu Val Gly Leu Pro Glu Asp
   225                 230                 235                 240
   tcc atc ctc aag tgg gat atg cag gag act gcc aag gga ggg cct ttc        768
   Ser Ile Leu Lys Trp Asp Met Gln Glu Thr Ala Lys Gly Gly Pro Phe
                   245                 250                 255
   gag gaa atc acc act tcc gac atc ttc atc aac tgc gtc tac ctt ggc        816
   Glu Glu Ile Thr Thr Ser Asp Ile Phe Ile Asn Cys Val Tyr Leu Gly
               260                 265                 270
   cct act ccc act cct ccc ttt gtc aca ttc gaa tcg ctt gcg acc cca        864
   Pro Thr Pro Thr Pro Pro Phe Val Thr Phe Glu Ser Leu Ala Thr Pro
           275                 280                 285
   gag aga cga ctc cgc gtc att gcc gat atc tcg tgt gat ccc aac agc        912
   Glu Arg Arg Leu Arg Val Ile Ala Asp Ile Ser Cys Asp Pro Asn Ser
       290                 295                 300
   gag aac aac ccg atc ccc ttg tac tcc acc tgg tcc agc ttt gac aag        960
   Glu Asn Asn Pro Ile Pro Leu Tyr Ser Thr Trp Ser Ser Phe Asp Lys
   305                 310                 315                 320
   ccc acg atc ccg aca tcc aag ccc gtt gac ggc cct gag ctg aga atc       1008
   Pro Thr Ile Pro Thr Ser Lys Pro Val Asp Gly Pro Glu Leu Arg Ile
                   325                 330                 335
   att gcc atc gac cac ctc ccc acc ttg att gcc cgc gag tcc agc gat       1056
   Ile Ala Ile Asp His Leu Pro Thr Leu Ile Ala Arg Glu Ser Ser Asp
               340                 345                 350
   gag tac tct ggc ctg ctg ctg ccg gcc ctg ctc act ctg gac aag cgg       1104
   Glu Tyr Ser Gly Leu Leu Leu Pro Ala Leu Leu Thr Leu Asp Lys Arg
           355                 360                 365
   gat act gag ggt gtc tgg acg aga gcg gag aag aca tac aag gac aga       1152
   Asp Thr Glu Gly Val Trp Thr Arg Ala Glu Lys Thr Tyr Lys Asp Arg
       370                 375                 380
   gtg gct gag ctg cct tag                                               1170
   Val Ala Glu Leu Pro
   385
<210> 34
   <211> 389
   <212> PRT
   <213> Trichoderma reesei
<400> 34
   Val Gln Leu Phe Ser Pro Ser Leu Ser Tyr His Lys Lys Gln Gln Gln
   1               5                   10                  15
   Ser Ile Arg Ala Lys Met Pro Thr Val Ile His Leu Arg Ala Asp Thr
               20                  25                  30
   Lys Pro Phe Glu Arg Arg Ser Pro Leu Ser Pro Pro Thr Ala Lys Ala
           35                  40                  45
   Leu Leu Asp Ala Gly Tyr Val Val Arg Val Glu Arg Ser Ser Glu Arg
       50                  55                  60
   Ile Tyr Lys Asp Glu Glu Phe Glu Ala Val Gly Ala Glu Leu Val Pro
   65                  70                  75                  80
   Ala Gly Ser Trp Ile Lys Ala Pro Lys Glu Asp Ile Ile Leu Gly Leu
                   85                  90                  95
   Lys Glu Leu Pro Asp Asp Asp Ile Asp Leu Pro His Thr Tyr Ile His
               100                 105                 110
   Phe Gln His Ile Phe Lys Lys Gln Leu Gly Trp Ala Pro Ser Leu Lys
           115                 120                 125
   Arg Phe Ala Arg Ala Gly Gly Thr Leu Tyr Asp Leu Glu Phe Leu Thr
       130                 135                 140
   Glu Glu Asn Gly Arg Arg Ile Ala Ala Phe Gly Tyr Phe Ala Gly Tyr
   145                 150                 155                 160
   Ala Gly Ala Ala Ile Ala Phe Ile Ser Trp Ala His Gln Ile Leu Asn
                   165                 170                 175
   Pro Gly Val Pro Gln Pro Pro Val Pro Leu Phe Asp Ser Ala Pro Ala
               180                 185                 190
   Leu Val Ala His Val Lys Ala Ala Leu Glu Pro Ala Ile Arg Ala Asn
           195                 200                 205
   Asn Gly Gln Leu Pro Arg Val Ile Val Ile Gly Ala Leu Gly Arg Cys
       210                 215                 220
   Gly Lys Gly Ala Val Asp Phe Cys Arg Glu Val Gly Leu Pro Glu Asp
   225                 230                 235                 240
   Ser Ile Leu Lys Trp Asp Met Gln Glu Thr Ala Lys Gly Gly Pro Phe
                   245                 250                 255
   Glu Glu Ile Thr Thr Ser Asp Ile Phe Ile Asn Cys Val Tyr Leu Gly
               260                 265                 270
   Pro Thr Pro Thr Pro Pro Phe Val Thr Phe Glu Ser Leu Ala Thr Pro
           275                 280                 285
   Glu Arg Arg Leu Arg Val Ile Ala Asp Ile Ser Cys Asp Pro Asn Ser
       290                 295                 300
   Glu Asn Asn Pro Ile Pro Leu Tyr Ser Thr Trp Ser Ser Phe Asp Lys
   305                 310                 315                 320
   Pro Thr Ile Pro Thr Ser Lys Pro Val Asp Gly Pro Glu Leu Arg Ile
                   325                 330                 335
   Ile Ala Ile Asp His Leu Pro Thr Leu Ile Ala Arg Glu Ser Ser Asp
               340                 345                 350
   Glu Tyr Ser Gly Leu Leu Leu Pro Ala Leu Leu Thr Leu Asp Lys Arg
           355                 360                 365
   Asp Thr Glu Gly Val Trp Thr Arg Ala Glu Lys Thr Tyr Lys Asp Arg
       370                 375                 380
   Val Ala Glu Leu Pro
   385
<210> 35
   <211> 1173
   <212> DNA
   <213> Ustilago maydis
<220>
   <221> CDS
   <222> (1)..(1173)
   <223> Lys1
<400> 35
   atg tct aca agc cgc caa ccc ctc tac ctc cga tgc gag atg aag ccg          48
   Met Ser Thr Ser Arg Gln Pro Leu Tyr Leu Arg Cys Glu Met Lys Pro
   1               5                   10                  15
   gcc gag cac cgt gcc gcg ctc acc cca acc acc gcc aag gca ctc att          96
   Ala Glu His Arg Ala Ala Leu Thr Pro Thr Thr Ala Lys Ala Leu Ile
               20                  25                  30
   gac gct ggg ttc gac atc acg gtc gag tcc gac cct caa cgt atc ttt      144
   Asp Ala Gly Phe Asp Ile Thr Val Glu Ser Asp Pro Gln Arg Ile Phe
           35                  40                  45
   gac gac aaa gag tac acc gag gta ggg tgc aag ctt gcc cct cac aac      192
   Asp Asp Lys Glu Tyr Thr Glu Val Gly Cys Lys Leu Ala Pro His Asn
       50                  55                  60
   acg ttt cac tcc ctg ccc gcc gac atc ccc atc atc ggt ctc aag gag      240
   Thr Phe His Ser Leu Pro Ala Asp Ile Pro Ile Ile Gly Leu Lys Glu
   65                  70                  75                  80
   ctt gag gag cct ggt ccg gat ctg cct cac acc cac att cag ttt gct      288
   Leu Glu Glu Pro Gly Pro Asp Leu Pro His Thr His Ile Gln Phe Ala
                   85                  90                  95
   cac tgc tac aag aag cag gcc ggt tgg gcg gat gtt ctg ggt cga ttc      336
   His Cys Tyr Lys Lys Gln Ala Gly Trp Ala Asp Val Leu Gly Arg Phe
               100                 105                 110
   aag cgc gga ggc gga aag ctc tac gat ttg gag ttt ttg gag gac aag      384
   Lys Arg Gly Gly Gly Lys Leu Tyr Asp Leu Glu Phe Leu Glu Asp Lys
           115                 120                 125
   aac ggc cgt cgt gta gcc gcg ttt ggt tgg cac gcc ggc ttt gca ggc      432
   Asn Gly Arg Arg Val Ala Ala Phe Gly Trp His Ala Gly Phe Ala Gly
       130                 135                 140
   gct gca ctt ggt ctg ttg gct ttg gct gaa cag gtg caa ggt gaa gac      480
   Ala Ala Leu Gly Leu Leu Ala Leu Ala Glu Gln Val Gln Gly Glu Asp
   145                 150                 155                 160
   caa agg ctg ggt gct caa aag gct tac ccc aac gag cag gcg ctc atc      528
   Gln Arg Leu Gly Ala Gln Lys Ala Tyr Pro Asn Glu Gln Ala Leu Ile
                   165                 170                 175
   gca cac gcc aag cag cag att gag ttc atc aag aaa tcg cgc tcg gac      576
   Ala His Ala Lys Gln Gln Ile Glu Phe Ile Lys Lys Ser Arg Ser Asp
               180                 185                 190
   ggc aaa gtg aag gcg ctc gtc gtc gga gct ttg gga cga tgt ggt cgt      624
   Gly Lys Val Lys Ala Leu Val Val Gly Ala Leu Gly Arg Cys Gly Arg
           195                 200                 205
   gga gcc att gac ttt ttc gaa aag gcc ggc gtc gct tct gag gac att      672
   Gly Ala Ile Asp Phe Phe Glu Lys Ala Gly Val Ala Ser Glu Asp Ile
       210                 215                 220
   gtc cgc tgg gac atc cag gag acc tca gcc aag cac ggt ccc tac caa      720
   Val Arg Trp Asp Ile Gln Glu Thr Ser Ala Lys His Gly Pro Tyr Gln
   225                 230                 235                 240
   gag ctg ctc gac gta gac atc ttt gtc aac tgc atc tac ctc acc tct      768
   Glu Leu Leu Asp Val Asp Ile Phe Val Asn Cys Ile Tyr Leu Thr Ser
                   245                 250                 255
   aaa atc ccg cct ttc ctc gac caa cct acg atc caa gcc gct ggt ccc      816
   Lys Ile Pro Pro Phe Leu Asp Gln Pro Thr Ile Gln Ala Ala Gly Pro
               260                 265                 270
   tcg cgt cgt ctc ggc gtc gtc gtc gac gta tcg tgc gac act acc aac      864
   Ser Arg Arg Leu Gly Val Val Val Asp Val Ser Cys Asp Thr Thr Asn
           275                 280                 285
   ccc aac aac cct ctc ccc atc tac gac atc aac acc acc ttt gac aaa        912
   Pro Asn Asn Pro Leu Pro Ile Tyr Asp Ile Asn Thr Thr Phe Asp Lys
       290                 295                 300
   cct acc gtc gac gtc aac acg ggc aaa ggt aac ccg agc ttg acc gtc        960
   Pro Thr Val Asp Val Asn Thr Gly Lys Gly Asn Pro Ser Leu Thr Val
   305                 310                 315                 320
   atc tcg atc gac cat ctg ccc act ctg ctg cct agg gag agc tcc gag       1008
   Ile Ser Ile Asp His Leu Pro Thr Leu Leu Pro Arg Glu Ser Ser Glu
                   325                 330                 335
   ggt ttc agc aac gac ctg ttg ccc agt ctg ttg cag ttg ccc tac gtg       1056
   Gly Phe Ser Asn Asp Leu Leu Pro Ser Leu Leu Gln Leu Pro Tyr Val
               340                 345                 350
   ctc ggt aag gac acc acc aag ctc gac acg ttg gac gaa gga aag gga       1104
   Leu Gly Lys Asp Thr Thr Lys Leu Asp Thr Leu Asp Glu Gly Lys Gly
           355                 360                 365
   gcc gtc tgg cag aga gcc gaa aag ctc ttc cat aag cac ttg gca gag       1152
   Ala Val Trp Gln Arg Ala Glu Lys Leu Phe His Lys His Leu Ala Glu
       370                 375                 380
   gct gag cag cac aca gct tga                                           1173
   Ala Glu Gln His Thr Ala
   385                 390
<210> 36
   <211> 390
   <212> PRT
   <213> Ustilago maydis
<400> 36
   Met Ser Thr Ser Arg Gln Pro Leu Tyr Leu Arg Cys Glu Met Lys Pro
   1               5                   10                  15
   Ala Glu His Arg Ala Ala Leu Thr Pro Thr Thr Ala Lys Ala Leu Ile
               20                  25                  30
   Asp Ala Gly Phe Asp Ile Thr Val Glu Ser Asp Pro Gln Arg Ile Phe
           35                  40                  45
   Asp Asp Lys Glu Tyr Thr Glu Val Gly Cys Lys Leu Ala Pro His Asn
       50                  55                  60
   Thr Phe His Ser Leu Pro Ala Asp Ile Pro Ile Ile Gly Leu Lys Glu
   65                  70                  75                  80
   Leu Glu Glu Pro Gly Pro Asp Leu Pro His Thr His Ile Gln Phe Ala
                   85                  90                  95
   His Cys Tyr Lys Lys Gln Ala Gly Trp Ala Asp Val Leu Gly Arg Phe
               100                 105                 110
   Lys Arg Gly Gly Gly Lys Leu Tyr Asp Leu Glu Phe Leu Glu Asp Lys
   115 120 125
   Asn Gly Arg Arg Val Ala Ala Phe Gly Trp His Ala Gly Phe Ala Gly
   130 135 140
   Ala Ala Leu Gly Leu Leu Ala Leu Ala Glu Gln Val Gln Gly Glu Asp
   145 150 155 160
   Gln Arg Leu Gly Ala Gln Lys Ala Tyr Pro Asn Glu Gln Ala Leu Ile
   165 170 175
   Ala His Ala Lys Gln Gln Ile Glu Phe Ile Lys Lys Ser Arg Ser Asp
   180 185 190
   Gly Lys Val Lys Ala Leu Val Val Gly Ala Leu Gly Arg Cys Gly Arg
   195 200 205
   Gly Ala Ile Asp Phe Phe Glu Lys Ala Gly Val Ala Ser Glu Asp Ile
   210 215 220
   Val Arg Trp Asp Ile Gln Glu Thr Ser Ala Lys His Gly Pro Tyr Gln
   225 230 235 240
   Glu Leu Leu Asp Val Asp Ile Phe Val Asn Cys Ile Tyr Leu Thr Ser
   245 250 255
   Lys Ile Pro Pro Phe Leu Asp Gln Pro Thr Ile Gln Ala Ala Gly Pro
   260 265 270
   Ser Arg Arg Leu Gly Val Val Val Asp Val Ser Cys Asp Thr Thr Asn
   275 280 285
   Pro Asn Asn Pro Leu Pro Ile Tyr Asp Ile Asn Thr Thr Phe Asp Lys
   290 295 300
   Pro Thr Val Asp Val Asn Thr Gly Lys Gly Asn Pro Ser Leu Thr Val
   305 310 315 320
   Ile Ser Ile Asp His Leu Pro Thr Leu Leu Pro Arg Glu Ser Ser Glu
   325 330 335
   Gly Phe Ser Asn Asp Leu Leu Pro Ser Leu Leu Gln Leu Pro Tyr Val
   340 345 350
   Leu Gly Lys Asp Thr Thr Lys Leu Asp Thr Leu Asp Glu Gly Lys Gly
   355 360 365
   Ala Val Trp Gln Arg Ala Glu Lys Leu Phe His Lys His Leu Ala Glu
   370 375 380
   Ala Glu Gln His Thr Ala
   385                 390
<210> 37
   <211> 1137
   <212> DNA
   <213> Verticillium albo-atrum
<220>
   <221> CDS
   <222> (1)..(1137)
   <223> Lys1
<400> 37
   atg tct ggc atc acc cta cac ctc cgg tcc gag acc aag ccg ctg gag      48
   Met Ser Gly Ile Thr Leu His Leu Arg Ser Glu Thr Lys Pro Leu Glu
   1               5                   10                  15
   cac cgc agt gct ctg acc ccc ttc acg gca tcc gag ctg atc aag gct      96
   His Arg Ser Ala Leu Thr Pro Phe Thr Ala Ser Glu Leu Ile Lys Ala
               20                  25                  30
   ggc tac acc ctc aat gtc gag cgc agc cct gtt cgc atc ttc gac gat     144
   Gly Tyr Thr Leu Asn Val Glu Arg Ser Pro Val Arg Ile Phe Asp Asp
           35                  40                  45
   gcc gag ttc gag aag att ggc gcc acc ctc gtc ccc gag ggt agc tgg     192
   Ala Glu Phe Glu Lys Ile Gly Ala Thr Leu Val Pro Glu Gly Ser Trp
       50                  55                  60
   acc gaa gcc ccc caa gac cac atc att gtt ggc ctc aag gag ctt ctt     240
   Thr Glu Ala Pro Gln Asp His Ile Ile Val Gly Leu Lys Glu Leu Leu
   65                  70                  75                  80
   gag gag gat ttc cct ctg aag cac gtt cac gtc cag ttt gcg cac tgc     288
   Glu Glu Asp Phe Pro Leu Lys His Val His Val Gln Phe Ala His Cys
                   85                  90                  95
   tac aag cag cag ggc ggg tgg gag aac gtc ctg gcc cgc ttc cct cgt     336
   Tyr Lys Gln Gln Gly Gly Trp Glu Asn Val Leu Ala Arg Phe Pro Arg
               100                 105                 110
   ggt ggc ggc acc ctc tac gac ctc gaa ttc ctc gag aag gag gtc gcc     384
   Gly Gly Gly Thr Leu Tyr Asp Leu Glu Phe Leu Glu Lys Glu Val Ala
           115                 120                 125
   ccc ggc cgc ttt gcc cga gtc gcc gcc ttt ggc tgg agc gcc ggt ttc     432
   Pro Gly Arg Phe Ala Arg Val Ala Ala Phe Gly Trp Ser Ala Gly Phe
       130                 135                 140
   tct ggt gct gct ctg gct ctc cag aac tgg gcc tgg cag ctc aac aac     480
   Ser Gly Ala Ala Leu Ala Leu Gln Asn Trp Ala Trp Gln Leu Asn Asn
   145                 150                 155                 160
   ccc ggc aag ccc ctg cct tcg gtc gag agc tat ccc aac gag gac gag     528
   Pro Gly Lys Pro Leu Pro Ser Val Glu Ser Tyr Pro Asn Glu Asp Glu
                   165                 170                 175
   ctc atc act gct gtc aag aag agc att gcc gag ggc aag gag aag gcc     576
   Leu Ile Thr Ala Val Lys Lys Ser Ile Ala Glu Gly Lys Glu Lys Ala
               180                 185                 190
   ggc aag cta ccc cag gtc ctc gtg atc ggc gcc ctc ggc cgc tgc ggc        624
   Gly Lys Leu Pro Gln Val Leu Val Ile Gly Ala Leu Gly Arg Cys Gly
           195                 200                 205
   agc ggt gcc gtc gag ctg tgc cgc cgc gtc ggc ctg ccc gag gag cag        672
   Ser Gly Ala Val Glu Leu Cys Arg Arg Val Gly Leu Pro Glu Glu Gln
       210                 215                 220
   atc ctc aag tgg gat ctc gag gag acg aag aag ggc ggc ccc ttc acc        720
   Ile Leu Lys Trp Asp Leu Glu Glu Thr Lys Lys Gly Gly Pro Phe Thr
   225                 230                 235                 240
   gag att gtt gag agc gac atc ttt gtc aac tgc atc tac ctc aac agc        768
   Glu Ile Val Glu Ser Asp Ile Phe Val Asn Cys Ile Tyr Leu Asn Ser
                   245                 250                 255
   aag att ccc aac ttt gtc gac ttt gag tct ctc aag tcg ccc aag agg        816
   Lys Ile Pro Asn Phe Val Asp Phe Glu Ser Leu Lys Ser Pro Lys Arg
               260                 265                 270
   cag ctg tcc gtc gtc tgc gac gtc tct gct gac acc acc aac ccc aac        864
   Gln Leu Ser Val Val Cys Asp Val Ser Ala Asp Thr Thr Asn Pro Asn
           275                 280                 285
   aac cct gtc ccc atc tac acc gtc gcc aca acc ttc gac aag ccc aca        912
   Asn Pro Val Pro Ile Tyr Thr Val Ala Thr Thr Phe Asp Lys Pro Thr
       290                 295                 300
   gtg ccc gtc gag gga ctc gag aac ccg ccc ctg agc gtc atc agc att        960
   Val Pro Val Glu Gly Leu Glu Asn Pro Pro Leu Ser Val Ile Ser Ile
   305                 310                 315                 320
   gat cac ctt ccc agc ctc ctc ccc cgt gag gcc tcc gag acg tac agc       1008
   Asp His Leu Pro Ser Leu Leu Pro Arg Glu Ala Ser Glu Thr Tyr Ser
                   325                 330                 335
   aag gac ctg ctg ccg tac ctg ctc acg ctg aag gac cgc aag acg gac       1056
   Lys Asp Leu Leu Pro Tyr Leu Leu Thr Leu Lys Asp Arg Lys Thr Asp
               340                 345                 350
   ccc gtc tgg acg agg gct gag aag ctc ttc aac gac aag gtc gct acg       1104
   Pro Val Trp Thr Arg Ala Glu Lys Leu Phe Asn Asp Lys Val Ala Thr
           355                 360                 365
   ctg ccg gcc gac ctg cag aag cct gct cag tga                           1137
   Leu Pro Ala Asp Leu Gln Lys Pro Ala Gln
       370                 375
<210> 38
   <211> 378
   <212> PRT
   <213> Verticillium albo-atrum
<400> 38
   Met Ser Gly Ile Thr Leu His Leu Arg Ser Glu Thr Lys Pro Leu Glu
   1               5                   10                  15
   His Arg Ser Ala Leu Thr Pro Phe Thr Ala Ser Glu Leu Ile Lys Ala
               20                  25                  30
   Gly Tyr Thr Leu Asn Val Glu Arg Ser Pro Val Arg Ile Phe Asp Asp
           35                  40                  45
   Ala Glu Phe Glu Lys Ile Gly Ala Thr Leu Val Pro Glu Gly Ser Trp
       50                  55                  60
   Thr Glu Ala Pro Gln Asp His Ile Ile Val Gly Leu Lys Glu Leu Leu
   65                  70                  75                  80
   Glu Glu Asp Phe Pro Leu Lys His Val His Val Gln Phe Ala His Cys
                   85                  90                  95
   Tyr Lys Gln Gln Gly Gly Trp Glu Asn Val Leu Ala Arg Phe Pro Arg
               100                 105                 110
   Gly Gly Gly Thr Leu Tyr Asp Leu Glu Phe Leu Glu Lys Glu Val Ala
           115                 120                 125
   Pro Gly Arg Phe Ala Arg Val Ala Ala Phe Gly Trp Ser Ala Gly Phe
       130                 135                 140
   Ser Gly Ala Ala Leu Ala Leu Gln Asn Trp Ala Trp Gln Leu Asn Asn
   145                 150                 155                 160
   Pro Gly Lys Pro Leu Pro Ser Val Glu Ser Tyr Pro Asn Glu Asp Glu
                   165                 170                 175
   Leu Ile Thr Ala Val Lys Lys Ser Ile Ala Glu Gly Lys Glu Lys Ala
               180                 185                 190
   Gly Lys Leu Pro Gln Val Leu Val Ile Gly Ala Leu Gly Arg Cys Gly
           195                 200                 205
   Ser Gly Ala Val Glu Leu Cys Arg Arg Val Gly Leu Pro Glu Glu Gln
       210                 215                 220
   Ile Leu Lys Trp Asp Leu Glu Glu Thr Lys Lys Gly Gly Pro Phe Thr
   225                 230                 235                 240
   Glu Ile Val Glu Ser Asp Ile Phe Val Asn Cys Ile Tyr Leu Asn Ser
                   245                 250                 255
   Lys Ile Pro Asn Phe Val Asp Phe Glu Ser Leu Lys Ser Pro Lys Arg
               260                 265                 270
   Gln Leu Ser Val Val Cys Asp Val Ser Ala Asp Thr Thr Asn Pro Asn
           275                 280                 285
   Asn Pro Val Pro Ile Tyr Thr Val Ala Thr Thr Phe Asp Lys Pro Thr
   290 295 300
   Val Pro Val Glu Gly Leu Glu Asn Pro Pro Leu Ser Val Ile Ser Ile
   305 310 315 320
   Asp His Leu Pro Ser Leu Leu Pro Arg Glu Ala Ser Glu Thr Tyr Ser
   325 330 335
   Lys Asp Leu Leu Pro Tyr Leu Leu Thr Leu Lys Asp Arg Lys Thr Asp
   340 345 350
   Pro Val Trp Thr Arg Ala Glu Lys Leu Phe Asn Asp Lys Val Ala Thr
   355 360 365
   Leu Pro Ala Asp Leu Gln Lys Pro Ala Gln
   370 375
<210> 39
   <211> 1125
   <212> DNA
   <213> Mycosphaerella graminicola
<220>
   <221> CDS
   <222> (1)..(1125)
   <223> Lys1
<400> 39
   atg tct tct tca cca cta act ttg cac atc cgc gcg gaa acc aag ccc        48
   Met Ser Ser Ser Pro Leu Thr Leu His Ile Arg Ala Glu Thr Lys Pro
   1               5                   10                  15
   ctc gaa cat cgc acc gct gtt ccg ccc aag gtt gcg agg aaa ctt gtc        96
   Leu Glu His Arg Thr Ala Val Pro Pro Lys Val Ala Arg Lys Leu Val
               20                  25                  30
   gag gcg ggt tac gtg gtc aac gtc gag cgg agt cca ttg agc atc ttc       144
   Glu Ala Gly Tyr Val Val Asn Val Glu Arg Ser Pro Leu Ser Ile Phe
           35                  40                  45
   ccg gac aac gag tac gaa gga aca gga gct acg ctt gtg ccg act ggt       192
   Pro Asp Asn Glu Tyr Glu Gly Thr Gly Ala Thr Leu Val Pro Thr Gly
       50                  55                  60
   tct tgg acg gaa gca ccc aag gac cat atc gtt gtg gga ttg aag gag       240
   Ser Trp Thr Glu Ala Pro Lys Asp His Ile Val Val Gly Leu Lys Glu
   65                  70                  75                  80
   ttg ccg gag gag gat ttc gcg ttg gtg cat acg cat gtt cag ttc gca       288
   Leu Pro Glu Glu Asp Phe Ala Leu Val His Thr His Val Gln Phe Ala
                   85                  90                  95
   cat tgc tat aag aat cag ggc gga tgg gag aaa gtc ttg agt cga ttc       336
   His Cys Tyr Lys Asn Gln Gly Gly Trp Glu Lys Val Leu Ser Arg Phe
               100                 105                 110
   ccg aga ggt gga ggg acg ctg ctt gac ttg gag ttc ctc gaa gat gag       384
   Pro Arg Gly Gly Gly Thr Leu Leu Asp Leu Glu Phe Leu Glu Asp Glu
           115                 120                 125
   caa gga cga cga gtt gcg gct ttc ggc tac cac gcc gga ttc gca gga        432
   Gln Gly Arg Arg Val Ala Ala Phe Gly Tyr His Ala Gly Phe Ala Gly
       130                 135                 140
   gca gct ctc tcc ctc atc aca tgg gcc tgg caa ctc gag cac ggc acc        480
   Ala Ala Leu Ser Leu Ile Thr Trp Ala Trp Gln Leu Glu His Gly Thr
   145                 150                 155                 160
   tcc aag ccc gtg cct gga gtc acc gcc tac gag aac gaa aca ctc ctc        528
   Ser Lys Pro Val Pro Gly Val Thr Ala Tyr Glu Asn Glu Thr Leu Leu
                   165                 170                 175
   gtc aac gac gtg aag aaa gcc gtc gag aag ggc aag tcg atc gcc ggc        576
   Val Asn Asp Val Lys Lys Ala Val Glu Lys Gly Lys Ser Ile Ala Gly
               180                 185                 190
   cac ctt ccg cga gtt ctg gtc atc ggc gct ctc gga cgt tgt ggc cgt        624
   His Leu Pro Arg Val Leu Val Ile Gly Ala Leu Gly Arg Cys Gly Arg
           195                 200                 205
   gga gcc gtc gac ctc tgc gtc aaa gct ggt ctg caa gac atc ctc aaa        672
   Gly Ala Val Asp Leu Cys Val Lys Ala Gly Leu Gln Asp Ile Leu Lys
       210                 215                 220
   tgg gac ctg caa gag acc aaa gcc aaa cca ggc ccc tac caa gaa atc        720
   Trp Asp Leu Gln Glu Thr Lys Ala Lys Pro Gly Pro Tyr Gln Glu Ile
   225                 230                 235                 240
   atc gag tcc gac gtc ttt gta aac tgc atc tac ctc tcc gcc aaa atc        768
   Ile Glu Ser Asp Val Phe Val Asn Cys Ile Tyr Leu Ser Ala Lys Ile
                   245                 250                 255
   cct cca ttc atc gac gca cct tcc ctc gcc tcc ccg acc cgc aaa ctc        816
   Pro Pro Phe Ile Asp Ala Pro Ser Leu Ala Ser Pro Thr Arg Lys Leu
               260                 265                 270
   agc gtc gtc tgt gac gtc tcc tgc gac acc acg aat cca cac aat ccg        864
   Ser Val Val Cys Asp Val Ser Cys Asp Thr Thr Asn Pro His Asn Pro
           275                 280                 285
   att ccg att tac tcc atc aat acg acg ttt gac aag ccc acc gtg cct        912
   Ile Pro Ile Tyr Ser Ile Asn Thr Thr Phe Asp Lys Pro Thr Val Pro
       290                 295                 300
   gtg gaa ctg tcc tca gag gcg aac gat gtg ccg ttg agt gtg att agc        960
   Val Glu Leu Ser Ser Glu Ala Asn Asp Val Pro Leu Ser Val Ile Ser
   305                 310                 315                 320
   atc gac cac ttg ccg agt ttg ttg ccg aga gag gcg agc gag gca ttc       1008
   Ile Asp His Leu Pro Ser Leu Leu Pro Arg Glu Ala Ser Glu Ala Phe
                   325                 330                 335
   agc gag gca ttg ttg ccg agt ttg ttg gag ttg aag gag agg aag acg       1056
   Ser Glu Ala Leu Leu Pro Ser Leu Leu Glu Leu Lys Glu Arg Lys Thr
               340                 345                 350
   gcg cgg gtg tgg agg cag gcg gag aag ttg ttc gag gac aag gtg gct       1104
   Ala Arg Val Trp Arg Gln Ala Glu Lys Leu Phe Glu Asp Lys Val Ala
           355                 360                 365
   agt ctg ccg aag ggt tcg tac                                           1125
   Ser Leu Pro Lys Gly Ser Tyr
       370                 375
<210> 40
   <211> 375
   <212> PRT
   <213> Mycosphaerella graminicola
<400> 40
   Met Ser Ser Ser Pro Leu Thr Leu His Ile Arg Ala Glu Thr Lys Pro
   1               5                   10                  15
   Leu Glu His Arg Thr Ala Val Pro Pro Lys Val Ala Arg Lys Leu Val
               20                  25                  30
   Glu Ala Gly Tyr Val Val Asn Val Glu Arg Ser Pro Leu Ser Ile Phe
           35                  40                  45
   Pro Asp Asn Glu Tyr Glu Gly Thr Gly Ala Thr Leu Val Pro Thr Gly
       50                  55                  60
   Ser Trp Thr Glu Ala Pro Lys Asp His Ile Val Val Gly Leu Lys Glu
   65                  70                  75                  80
   Leu Pro Glu Glu Asp Phe Ala Leu Val His Thr His Val Gln Phe Ala
                   85                  90                  95
   His Cys Tyr Lys Asn Gln Gly Gly Trp Glu Lys Val Leu Ser Arg Phe
               100                 105                 110
   Pro Arg Gly Gly Gly Thr Leu Leu Asp Leu Glu Phe Leu Glu Asp Glu
           115                 120                 125
   Gln Gly Arg Arg Val Ala Ala Phe Gly Tyr His Ala Gly Phe Ala Gly
       130                 135                 140
   Ala Ala Leu Ser Leu Ile Thr Trp Ala Trp Gln Leu Glu His Gly Thr
   145                 150                 155                 160
   Ser Lys Pro Val Pro Gly Val Thr Ala Tyr Glu Asn Glu Thr Leu Leu
                   165                 170                 175
   Val Asn Asp Val Lys Lys Ala Val Glu Lys Gly Lys Ser Ile Ala Gly
               180                 185                 190
   His Leu Pro Arg Val Leu Val Ile Gly Ala Leu Gly Arg Cys Gly Arg
           195                 200                 205
   Gly Ala Val Asp Leu Cys Val Lys Ala Gly Leu Gln Asp Ile Leu Lys
       210                 215                 220
   Trp Asp Leu Gln Glu Thr Lys Ala Lys Pro Gly Pro Tyr Gln Glu Ile
   225                 230                 235                 240
   Ile Glu Ser Asp Val Phe Val Asn Cys Ile Tyr Leu Ser Ala Lys Ile
                   245                 250                 255
   Pro Pro Phe Ile Asp Ala Pro Ser Leu Ala Ser Pro Thr Arg Lys Leu
               260                 265                 270
   Ser Val Val Cys Asp Val Ser Cys Asp Thr Thr Asn Pro His Asn Pro
           275                 280                 285
   Ile Pro Ile Tyr Ser Ile Asn Thr Thr Phe Asp Lys Pro Thr Val Pro
       290                 295                 300
   Val Glu Leu Ser Ser Glu Ala Asn Asp Val Pro Leu Ser Val Ile Ser
   305                 310                 315                 320
   Ile Asp His Leu Pro Ser Leu Leu Pro Arg Glu Ala Ser Glu Ala Phe
                   325                 330                 335
   Ser Glu Ala Leu Leu Pro Ser Leu Leu Glu Leu Lys Glu Arg Lys Thr
               340                 345                 350
   Ala Arg Val Trp Arg Gln Ala Glu Lys Leu Phe Glu Asp Lys Val Ala
           355                 360                 365
   Ser Leu Pro Lys Gly Ser Tyr
       370                 375
<210> 41
   <211> 1170
   <212> DNA
   <213> Fusarium moniliforme
<220>
   <221> CDS
   <222> (1)..(1170)
   <223> Lys1
<400> 41
   atg tct gac tat ccc cac att ctc ctt cgc gct gag gag aag cct ctc        48
   Met Ser Asp Tyr Pro His Ile Leu Leu Arg Ala Glu Glu Lys Pro Leu
   1               5                   10                  15
   gag cac cga tct ttc tcc ccc gca att atc aag aca ctc gtt gat gct        96
   Glu His Arg Ser Phe Ser Pro Ala Ile Ile Lys Thr Leu Val Asp Ala
               20                  25                  30
   gga tac ccc att tcc gtc gag cga tcg tct acc gac ccc aag ttc aag       144
   Gly Tyr Pro Ile Ser Val Glu Arg Ser Ser Thr Asp Pro Lys Phe Lys
           35                  40                  45
   cgt atc ttt gag gac tca gaa tat gag gct gct ggt gct cgt ctt gtc       192
   Arg Ile Phe Glu Asp Ser Glu Tyr Glu Ala Ala Gly Ala Arg Leu Val
       50                  55                  60
   gat acg ggt gtc tgg ccc aac gct gag cct gga aca atc att ctc ggc      240
   Asp Thr Gly Val Trp Pro Asn Ala Glu Pro Gly Thr Ile Ile Leu Gly
   65                  70                  75                  80
   cta aaa gag cta cct tct gag gac ttc ccg ctc aag aat gac cac att      288
   Leu Lys Glu Leu Pro Ser Glu Asp Phe Pro Leu Lys Asn Asp His Ile
                   85                  90                  95
   aca ttt gca cat tgt tac aag aac caa ggc ggg tgg gag cag gtc ctc      336
   Thr Phe Ala His Cys Tyr Lys Asn Gln Gly Gly Trp Glu Gln Val Leu
               100                 105                 110
   ggt cgc tgg gca cgc ggt ggc agc cgg ctt tac gac ctc gag ttt ctt      384
   Gly Arg Trp Ala Arg Gly Gly Ser Arg Leu Tyr Asp Leu Glu Phe Leu
           115                 120                 125
   gtg gac gaa caa ggt cgg cgt gtt tct gca ttt ggg tac cac gcg ggc      432
   Val Asp Glu Gln Gly Arg Arg Val Ser Ala Phe Gly Tyr His Ala Gly
       130                 135                 140
   ttc gcc ggt gct gcg ctc gga atc aag aca ctc gct cac cag ctg cag      480
   Phe Ala Gly Ala Ala Leu Gly Ile Lys Thr Leu Ala His Gln Leu Gln
   145                 150                 155                 160
   ggc tcg tcc tct aaa ctt cct tct gtc gag aca ttc act gat ggc cgc      528
   Gly Ser Ser Ser Lys Leu Pro Ser Val Glu Thr Phe Thr Asp Gly Arg
                   165                 170                 175
   gga tat tac ttg aac gaa gat gag ctc gtc aac cag att cgt gag gat      576
   Gly Tyr Tyr Leu Asn Glu Asp Glu Leu Val Asn Gln Ile Arg Glu Asp
               180                 185                 190
   ctc gct aag gct gaa aag gct ctc gga cgt aag ccc act gct ctc gtc      624
   Leu Ala Lys Ala Glu Lys Ala Leu Gly Arg Lys Pro Thr Ala Leu Val
           195                 200                 205
   ctt ggt gct ctt gga cga tgt ggt aag ggt gcc gtg gac ctt ttc ctg      672
   Leu Gly Ala Leu Gly Arg Cys Gly Lys Gly Ala Val Asp Leu Phe Leu
       210                 215                 220
   aag gcc ggc atg cct gat gac aac att acc cgc tgg gac ttg aac gag      720
   Lys Ala Gly Met Pro Asp Asp Asn Ile Thr Arg Trp Asp Leu Asn Glu
   225                 230                 235                 240
   act aag gac cgt gat ggc cct tac gag gag atc gcc aag gct gat gtc      768
   Thr Lys Asp Arg Asp Gly Pro Tyr Glu Glu Ile Ala Lys Ala Asp Val
                   245                 250                 255
   ttc ctc aac gcc atc tac ctc tcc aag ccc att ccc cct ttc atc aac      816
   Phe Leu Asn Ala Ile Tyr Leu Ser Lys Pro Ile Pro Pro Phe Ile Asn
               260                 265                 270
   caa gaa ctc ctt gcc aag caa ggt cgc aac ctc gct gtt gtc atc gac      864
   Gln Glu Leu Leu Ala Lys Gln Gly Arg Asn Leu Ala Val Val Ile Asp
           275                 280                 285
   gtt tct tgt gac acc aca aac cct cac aac cct atc ccc atc tac tcc      912
   Val Ser Cys Asp Thr Thr Asn Pro His Asn Pro Ile Pro Ile Tyr Ser
       290                 295                 300
   atc aac acc acc ttt gag gac cca acc gtc ccc gtt gag atc aag gac      960
   Ile Asn Thr Thr Phe Glu Asp Pro Thr Val Pro Val Glu Ile Lys Asp
   305                 310                 315                 320
   gat cag aac aac ctc ccc cta tcc gtc atc agc att gat cac ctc cct        1008
   Asp Gln Asn Asn Leu Pro Leu Ser Val Ile Ser Ile Asp His Leu Pro
                   325                 330                 335
   tcc atg ctt ccc cgc gag gct agt gag gcc ttt agt gag ggt ctc aag        1056
   Ser Met Leu Pro Arg Glu Ala Ser Glu Ala Phe Ser Glu Gly Leu Lys
               340                 345                 350
   gag tct ctg ctc aca ctc aag gat cgc gag act tcg cgg gtg tgg act        1104
   Glu Ser Leu Leu Thr Leu Lys Asp Arg Glu Thr Ser Arg Val Trp Thr
           355                 360                 365
   gat gct gag aag ctc ttc cat gag aag gtt gcc ctg ttg ccc gag gag        1152
   Asp Ala Glu Lys Leu Phe His Glu Lys Val Ala Leu Leu Pro Glu Glu
       370                 375                 380
   ttg aga acc aag agt gtt                                                1170
   Leu Arg Thr Lys Ser Val
   385                 390
<210> 42
   <211> 390
   <212> PRT
   <213> Fusarium moniliforme
<400> 42
   Met Ser Asp Tyr Pro His Ile Leu Leu Arg Ala Glu Glu Lys Pro Leu
   1               5                   10                  15
   Glu His Arg Ser Phe Ser Pro Ala Ile Ile Lys Thr Leu Val Asp Ala
               20                  25                  30
   Gly Tyr Pro Ile Ser Val Glu Arg Ser Ser Thr Asp Pro Lys Phe Lys
           35                  40                  45
   Arg Ile Phe Glu Asp Ser Glu Tyr Glu Ala Ala Gly Ala Arg Leu Val
       50                  55                  60
   Asp Thr Gly Val Trp Pro Asn Ala Glu Pro Gly Thr Ile Ile Leu Gly
   65                  70                  75                  80
   Leu Lys Glu Leu Pro Ser Glu Asp Phe Pro Leu Lys Asn Asp His Ile
                   85                  90                  95
   Thr Phe Ala His Cys Tyr Lys Asn Gln Gly Gly Trp Glu Gln Val Leu
               100                 105                 110
   Gly Arg Trp Ala Arg Gly Gly Ser Arg Leu Tyr Asp Leu Glu Phe Leu
           115                 120                 125
   Val Asp Glu Gln Gly Arg Arg Val Ser Ala Phe Gly Tyr His Ala Gly
       130                 135                 140
   Phe Ala Gly Ala Ala Leu Gly Ile Lys Thr Leu Ala His Gln Leu Gln
   145                 150                 155                 160
   Gly Ser Ser Ser Lys Leu Pro Ser Val Glu Thr Phe Thr Asp Gly Arg
                   165                 170                 175
   Gly Tyr Tyr Leu Asn Glu Asp Glu Leu Val Asn Gln Ile Arg Glu Asp
               180                 185                 190
   Leu Ala Lys Ala Glu Lys Ala Leu Gly Arg Lys Pro Thr Ala Leu Val
           195                 200                 205
   Leu Gly Ala Leu Gly Arg Cys Gly Lys Gly Ala Val Asp Leu Phe Leu
       210                 215                 220
   Lys Ala Gly Met Pro Asp Asp Asn Ile Thr Arg Trp Asp Leu Asn Glu
   225                 230                 235                 240
   Thr Lys Asp Arg Asp Gly Pro Tyr Glu Glu Ile Ala Lys Ala Asp Val
                   245                 250                 255
   Phe Leu Asn Ala Ile Tyr Leu Ser Lys Pro Ile Pro Pro Phe Ile Asn
               260                 265                 270
   Gln Glu Leu Leu Ala Lys Gln Gly Arg Asn Leu Ala Val Val Ile Asp
           275                 280                 285
   Val Ser Cys Asp Thr Thr Asn Pro His Asn Pro Ile Pro Ile Tyr Ser
       290                 295                 300
   Ile Asn Thr Thr Phe Glu Asp Pro Thr Val Pro Val Glu Ile Lys Asp
   305                 310                 315                 320
   Asp Gln Asn Asn Leu Pro Leu Ser Val Ile Ser Ile Asp His Leu Pro
                   325                 330                 335
   Ser Met Leu Pro Arg Glu Ala Ser Glu Ala Phe Ser Glu Gly Leu Lys
               340                 345                 350
   Glu Ser Leu Leu Thr Leu Lys Asp Arg Glu Thr Ser Arg Val Trp Thr
           355                 360                 365
   Asp Ala Glu Lys Leu Phe His Glu Lys Val Ala Leu Leu Pro Glu Glu
       370                 375                 380
   Leu Arg Thr Lys Ser Val
   385                 390
<210> 43
   <211> 856
   <212> DNA
   <213> Claviceps purpurea
<220>
   <221> CDS
   <222> (3)..(854)
   <223> Lys1
<400> 43
   ct ggc tac aaa gtt tcc gtg gag cgc tca cca tcg gac ccc gac ttt        47
      Gly Tyr Lys Val Ser Val Glu Arg Ser Pro Ser Asp Pro Asp Phe
      1               5                   10                  15
   gtc cgc atc ttc caa gac tct gaa tat gag gcg gct gga gct tct ctg       95
   Val Arg Ile Phe Gln Asp Ser Glu Tyr Glu Ala Ala Gly Ala Ser Leu
                   20                  25                  30
   gtt ccc act ggc gtg tgg ccc aag gct gcg cca aac act ctg atc gtt      143
   Val Pro Thr Gly Val Trp Pro Lys Ala Ala Pro Asn Thr Leu Ile Val
               35                  40                  45
   ggt ctc aag gag atc ccc gaa gag ggc ttt tct ctc acc aat gac cac      191
   Gly Leu Lys Glu Ile Pro Glu Glu Gly Phe Ser Leu Thr Asn Asp His
           50                  55                  60
   ctg acc ttt gcc cat tgc tac aag aag caa gct ggc tgg aca cag gtt      239
   Leu Thr Phe Ala His Cys Tyr Lys Lys Gln Ala Gly Trp Thr Gln Val
       65                  70                  75
   ctc gga cgc ttt gtc gca ggc aaa tcg acc ctc tac gac ctg gag ttt      287
   Leu Gly Arg Phe Val Ala Gly Lys Ser Thr Leu Tyr Asp Leu Glu Phe
   80                  85                  90                  95
   ctg gtc gat agc act gga aga cgc atc tct gct ttc gga ttc cac gct      335
   Leu Val Asp Ser Thr Gly Arg Arg Ile Ser Ala Phe Gly Phe His Ala
                   100                 105                 110
   ggc ttc acg ggc gct gct ttg gga gtg aag aac tgg gct tgg caa ctg      383
   Gly Phe Thr Gly Ala Ala Leu Gly Val Lys Asn Trp Ala Trp Gln Leu
               115                 120                 125
   gcc aat cca gcc aag aag ctt ccc gcc gtg tcc aca ttc act gac ggc      431
   Ala Asn Pro Ala Lys Lys Leu Pro Ala Val Ser Thr Phe Thr Asp Gly
           130                 135                 140
   aag gga tac tat gtg aac gaa acc gag ttg gtg gag caa atc cgc aag      479
   Lys Gly Tyr Tyr Val Asn Glu Thr Glu Leu Val Glu Gln Ile Arg Lys
       145                 150                 155
   gat ctc gag gca gga gag aag att ctt ggc cgt aag cca aca gcc ttt      527
   Asp Leu Glu Ala Gly Glu Lys Ile Leu Gly Arg Lys Pro Thr Ala Phe
   160                 165                 170                 175
   gtc ctg gga gct ttg ggt cgc tgc ggc agg ggt gct tgc gat ttg ttc      575
   Val Leu Gly Ala Leu Gly Arg Cys Gly Arg Gly Ala Cys Asp Leu Phe
                   180                 185                 190
   ctc aag gca ggc ttg ccc gaa gag aac atc acc cga tgg gat ttg gcc      623
   Leu Lys Ala Gly Leu Pro Glu Glu Asn Ile Thr Arg Trp Asp Leu Ala
               195                 200                 205
   gaa acc cgc gac cgc caa ggc ccg tac gag gag att gct cag cac gat      671
   Glu Thr Arg Asp Arg Gln Gly Pro Tyr Glu Glu Ile Ala Gln His Asp
           210                 215                 220
   atc ttc ctg aac gct atc tat ctc tcc gag ccc atc cca cct ttt gtc        719
   Ile Phe Leu Asn Ala Ile Tyr Leu Ser Glu Pro Ile Pro Pro Phe Val
       225                 230                 235
   aac aac gag ctt ctt tct aag cct ggc cgc aaa ttg agt gtt gtc ata        767
   Asn Asn Glu Leu Leu Ser Lys Pro Gly Arg Lys Leu Ser Val Val Ile
   240                 245                 250                 255
   gac gtc tcc tgc gac acc acc aac ccc cac aac ccc atc ccc atc tac        815
   Asp Val Ser Cys Asp Thr Thr Asn Pro His Asn Pro Ile Pro Ile Tyr
                   260                 265                 270
   agc atc aac acc acc ttc gac agc ccc acc gtt gcc gtc aa                 856
   Ser Ile Asn Thr Thr Phe Asp Ser Pro Thr Val Ala Val
               275                 280
<210> 44
   <211> 284
   <212> PRT
   <213> Claviceps purpurea
<400> 44
   Gly Tyr Lys Val Ser Val Glu Arg Ser Pro Ser Asp Pro Asp Phe Val
   1 5 10 15
   Arg Ile Phe Gln Asp Ser Glu Tyr Glu Ala Ala Gly Ala Ser Leu Val
   20 25 30
   Pro Thr Gly Val Trp Pro Lys Ala Ala Pro Asn Thr Leu Ile Val Gly
   35 40 45
   Leu Lys Glu Ile Pro Glu Glu Gly Phe Ser Leu Thr Asn Asp His Leu
   50 55 60
   Thr Phe Ala His Cys Tyr Lys Lys Gln Ala Gly Trp Thr Gln Val Leu
   65 70 75 80
   Gly Arg Phe Val Ala Gly Lys Ser Thr Leu Tyr Asp Leu Glu Phe Leu
   85 90 95
   Val Asp Ser Thr Gly Arg Arg Ile Ser Ala Phe Gly Phe His Ala Gly
   100 105 110
   Phe Thr Gly Ala Ala Leu Gly Val Lys Asn Trp Ala Trp Gln Leu Ala
   115 120 125
   Asn Pro Ala Lys Lys Leu Pro Ala Val Ser Thr Phe Thr Asp Gly Lys
   130 135 140
   Gly Tyr Tyr Val Asn Glu Thr Glu Leu Val Glu Gln Ile Arg Lys Asp
   145 150 155 160
   Leu Glu Ala Gly Glu Lys Ile Leu Gly Arg Lys Pro Thr Ala Phe Val
                   165                 170                 175
   Leu Gly Ala Leu Gly Arg Cys Gly Arg Gly Ala Cys Asp Leu Phe Leu
               180                 185                 190
   Lys Ala Gly Leu Pro Glu Glu Asn Ile Thr Arg Trp Asp Leu Ala Glu
           195                 200                 205
   Thr Arg Asp Arg Gln Gly Pro Tyr Glu Glu Ile Ala Gln His Asp Ile
       210                 215                 220
   Phe Leu Asn Ala Ile Tyr Leu Ser Glu Pro Ile Pro Pro Phe Val Asn
   225                 230                 235                 240
   Asn Glu Leu Leu Ser Lys Pro Gly Arg Lys Leu Ser Val Val Ile Asp
                   245                 250                 255
   Val Ser Cys Asp Thr Thr Asn Pro His Asn Pro Ile Pro Ile Tyr Ser
               260                 265                 270
   Ile Asn Thr Thr Phe Asp Ser Pro Thr Val Ala Val
           275                 280
<210> 45
   <211> 41
   <212> DNA
   <213> Artificial Sequence
<220>
   <223> primer
<220>
   <221> primer_bind
   <222> (1)..(41)
<400> 45
   taatacgact cactataggg ttgcaggaga gcgcagaaag c 41
<210> 46
   <211> 42
   <212> DNA
   <213> Artificial Sequence
<220>
   <223> primer
<220>
   <221> primer_bind
   <222> (1)..(42)
<400> 46
   taatacgact cactataggg tcagttggag tccgcgtggt gt 42
<210> 47
   <211> 20
   <212> DNA
   <213> Artificial Sequence
<220>
   <223> primer
<220>
   <221> primer_bind
   <222> (1)..(20)
<400> 47
   cgactctggt gacggtgtgt 20
<210> 48
   <211> 20
   <212> DNA
   <213> Artificial Sequence
<220>
   <223> primer
<220>
   <221> primer_bind
   <222> (1)..(20)
<400> 48
   gcgtgaggaa gagcgtaacc 20
<210> 49
   <211> 18
   <212> DNA
   <213> Artificial Sequence
<220>
   <223> primer
<220>
   <221> primer_bind
   <222> (1)..(18)
<400> 49
   ccgcccagac aatttcgt 18
<210> 50
   <211> 20
   <212> DNA
   <213> Artificial Sequence
<220>
   <223> primer
<220>
   <221> primer_bind
   <222> (1)..(20)
<400> 50
   ccttggccca gttgttacca 20
<210> 51
   <211> 21
   <212> DNA
   <213> Artificial Sequence
<220>
   <223> primer
<220>
   <221> primer_bind
   <222> (1)..(21)
<400> 51
   tgggtggttt ccaaggtctt c 21
<210> 52
   <211> 20
   <212> DNA
   <213> Artificial Sequence
<220>
   <223> primer
<220>
   <221> primer_bind
   <222> (1)..(20)
<400> 52
   aaaggcacca agccactgaa 20
<210> 53
   <211> 48
   <212> DNA
   <213> Artificial Sequence
<220>
   <223> primer
<220>
   <221> primer_bind
   <222> (1)..(48)
<400> 53
   tcgacaggcc tggatcctta attaaactag tctcgaggag ctcggtac 48
<210> 54
   <211> 40
   <212> DNA
   <213> Artificial Sequence
<220>
   <223> primer
<220>
   <221> primer_bind
   <222> (1)..(40)
<400> 54
   cgagctcctc gagactagtt taattaagga tccaggcctg 40
<210> 55
   <211> 31
   <212> DNA
   <213> Artificial Sequence
<220>
   <223> primer
<220>
   <221> primer_bind
   <222> (1)..(31)
<400> 55
   tatctcgagt ctagacaacg ccattggtta c 31
<210> 56
   <211> 25
   <212> DNA
   <213> Artificial Sequence
<220>
   <223> primer
<220>
   <221> primer_bind
   <222> (1)..(25)
<400> 56
   agaggtacca agcttgcgta gctgg 25
<210> 57
   <211> 22
   <212> DNA
   <213> Artificial Sequence
<220>
   <223> primer
<220>
   <221> primer_bind
   <222> (1)..(22)
<400> 57
   tcaatagaag cgaacgcgta aa 22
<210> 58
   <211> 20
   <212> DNA
   <213> Artificial Sequence
<220>
   <223> primer
<220>
   <221> primer_bind
   <222> (1)..(20)
<400> 58
   gttcgggatc tgctcgatgt 20

## Description

The present invention relates to control of plant pathogens and pests, particularly fungi or oomycetes, by inhibiting one or more biological functions, particularly by inhibiting fungi saccharopine dehydrogenase gene involved in the α-aminoadipate pathway for lysine biosynthesis and their oomycetes homologs using RNA interference.
The invention provides methods and compositions using RNA interference of fungi or oomycetes target genes for such control. The invention is also directed to methods for making transgenic plants tolerant to said fungi or oomycetes, and to transgenic plants and seeds generated thereof.

The technology used in the context of the present invention is RNA interference or RNAi.
The expression in an organism of a sequence homologous to a target-gene capable of inducing the formation of small double-stranded RNA (dsRNA) makes it possible, very specifically, to extinguish this gene and to observe the phenotype that results therefrom (Xiao *et al.*, 2003).

The dsRNA triggers the specific degradation of a homologous RNA only in the region of identity with the dsRNA (Zamore *et al*., 2000; Tang *et al.*, 2003). The dsRNA is an RNA molecule which contains a double-stranded sequence, generally of at least 19 base pairs (bp) including a sense strand and an antisense strand. The dsRNA molecules are also characterized by the very large degree of complementarity between the two complementary RNA strands. The dsRNA is degraded into RNA fragments of generally 18 to 25 nucleotides (siRNA) and the cleavage sites on the target RNA are evenly spaced apart by 18 to 25 nucleotides. The small siRNAs resulting therefrom exhibit a very high degree of identity with respect to the target RNA; however, mismatches of 3 to 4 nucleotides between the siRNA and the corresponding portion of the target RNA nevertheless make it possible for the system to operate (Tang *et al.*, 2003). It has thus been suggested that these fragments of 18 to 25 nucleotides constitute RNA guides for recognition of the target (Zamore *et al.*, 2000). These small RNAs have also been detected in extracts prepared from Schneider 2 cells of *Drosophila melanogaster* which had been transfected with dsRNAs before cell lysis (Hammond *et al.*, 2000). The guiding role of the fragments of 18 to 25 nucleotides in the cleavage of the mRNAs is supported by the observation that these fragments of 19 to 25 nucleotides isolated from dsRNA are capable of being involved in the degradation of mRNA (Zamore *et al.*, 2000). Sizable homologous RNA molecules also accumulate in plant tissues which undergo the PTGS phenomenon (Post Transcriptional Gene Silencing, Hamilton and Baulcome, 1999). These small RNAs can regulate gene expression at three different levels:
- transcription (TGS for Transcriptional Gene Silencing),
- messenger RNA degradation (PTGS for Post Transcriptional Gene Silencing),
- miRNA pathway
- translation.

Regulation involving messenger RNA degradation appears to exist in all eukaryotes, whereas regulation at the transcriptional level has only been described in mammalians, plants, drosophila and *C. elegans.* As regards the regulation of translation, it has been characterized in *C*. *elegans* and drosophila and appears also to exist in mammals (Hannon, 2002) and plants (Ruiz Ferrer and Voinnet, 2009). In the literature, reference is made to RNAi, to PTGS, to cosuppression or to quelling (reserved for fungi) when referring to this phenomenon, depending on the organisms in which it is studied.

RNAi has in particular proved that it is effective when double-stranded RNA (dsRNA) is injected into the nematode Caenorhabditis elegans (Fire et al. 1998; Montgomery et al., 1998; WO99/32619). Inhibition of the expression of an insect target gene was also observed when this insect is fed with bacteria expressing small double-stranded RNAs corresponding to said insect target gene (WO 01/37654).

More recently, pharmaceutical compositions comprising dsRNA substantially complementary to at least part of a gene suspected to be involved in the human papilloma virus (HPV) infection together with a pharmaceutically acceptable carrier have been disclosed to treating said HPV infection (WO2009/0247607).

The introduction of dsRNA was carried out in plants in order to induce silencing of an endogenous target gene (Hamilton et al., 1998, WO99/15682), to induce resistance to RNA viruses by means of the use of a transgene expressing a dsRNA having substantial identity with respect to the viral genes (Waterhouse *et al.*, 1998; Pandolfini et al., 2003, WO98/36083, WO99/15682, US 5,175,102), but also to induce resistance to nematodes (Chuang and Meyerowitz, 2000, WO01/96584) or alternatively to the bacterium *Agrobacterium* (WO00/26346, Escobar *et a*/*.*, 2001). Houmard Nancy M et al. (Plant Biotechnology Journal, 2007, 5, pp 605-614**) have disclosed maize transformed with a construct encoding a dsRNA targeting the endogenous bifunctional lysine-ketoglutarate reductase/saccharopine dehydrogenase.** More recently, it has been shown that plants expressing dsRNA having substantial identity against a fungal gene essential to the growth of the fungus or to its pathogenicity may also induced resistance to this fungus (WO05/071091).

Nevertheless, since that time, only few preliminary results and no commercial examples exist on RNAi-mediated resistance or tolerance to phytopathogenic fungi where the double-stranded (dsRNA) or small interfering (siRNA) molecules are expressed in the plant, or applied as part of an external composition to the seed, the plant or to the fruit of the plant or to soil or to inert substrate wherein the plant is growing or wherein it is desired to grow.
Among others, one difficulty is to find an appropriate target gene, whose inhibition by dsRNA or siRNA induces a good level of fungi tolerance, up to a level suitable for a commercial use, without deleterious effect on the plant expressing said dsRNA or siRNA or on which a composition comprising said dsRNA or siRNA is applied.

Stärkel C. attempted in her Ph. D. thesis ["Host induced gene silencing - strategies for the improvement of resistance against Cercospora beticola in sugar beet (B. vulgaris L.) and against Fusarium graminearum in wheat (T. aestivum L.) and maize (Z. mays L.)", defended in June 2011] to inhibit the growth of *Fusarium graminearum* by transforming wheat with silencing constructs targeting the homoaconitase gene, an essential gene in the lysine biosynthesis pathway. Nevertheless, no transgenic wheat plants could be generated. Moreover, the attempt to delete or silence the homoaconitase gene in *Fusarium graminearum* by transformation of the fungus with a contruct designed for the inducible silencing of the homoaconitase gene were also unsuccessful.

The present inventors have surprisingly demonstrated that inhibition of fungus or oomycetes saccharopine dehydrogenase gene, which is involved in the α-aminoadipate (AAA) pathway, via RNAi methodology causes cessation of infection, growth, development, reproduction and/or pathogenicity, and eventually results in the death of the organism.

This new target for the RNAi technology is particularly suitable, considering that AAA pathway is specifically found in some plant pathogens, including higher fungi, and not in plants, humans and animals.
Among the 20 common proteinogenic amino acids, L-lysine is the only one known to have a biosynthetic pathway which differs in plants and in higher fungi. In plants and bacteria, L-lysine is obtained through the diaminopimelate (DAP) pathway. In higher fungi and euglenoids, L-lysine is obtained through the the α-aminoadipate (AAA) pathway. Saccharopine dehydrogenase, homocitrate synthase, homoaconitase, homoisocitrate dehydrogenase, α-Aminoadipate aminotransferase, α-Aminoadipate reductase and saccharopine reductase are enzymes involved in the α-aminoadipate pathway for the biosynthesis of L-lysine.
None of the enzymes involved in these two distinct pathways (DAP and AAA pathways) are common (Xu et al., 2006, Bhattacharjee, J. K., 1985; Bhattacharjee, J. K., 1992; Vogel, H. J., 1965). As an example, fungi saccharopine dehydrogenase is involved in the Lysine biosynthesis, when the lysine-ketoglutarate from plant, sometimes also called saccharopine dehydrogenase, is responsible for lysine catabolism (Houmard et al., 2007). For humans and animals, L-lysine is an essential amino acid which can only be obtained from protein in the diet. Additionally, enzymes involved in the fungal AAA pathway are unique to lysine synthesis (Umbargar, H. E., 1978; Bhattacharjee, J. K., 1992).
The presence of a specific α-aminoadipate (AAA) pathway in higher fungi, which is not present nor in plant nor in humans or animals, leads to consider the enzymes involved in said AAA pathway as particularly attractive targets for the control of plant pathogens, particularly for fungal pathogens.

Interestingly, although lysine has been reported to be synthesized in oomycetes via the diaminopimelic pathway (Born and Blanchard, 1999), genes homologous to fungus genes of AAA pathway have been found in oomycetes, which may indicate that both pathways might be present in oomycetes. The inventors have shown that dsRNA against oomycete saccharopine dehydrogenase gene substantially reduced the growth of said oomycete, and that plants expressing dsRNA against oomycete saccharopine dehydrogenase genes could be less susceptible to said oomycete infection.

The presence of the AAA pathway for lysine biosynthesis has been demonstrated and studied for example in *Saccharomyces cerevisiae* (Broquist, H. P., 1971, Bhattacharjee, J. K., 1992), the human pathogenic fungi *Candida albicans* (Garrad, R. C. and Bhattacharjee, J. K., 1992), and the plant pathogen *Magnaporthe grisea* (Umbargar, H. E., 1978). The saccharopine dehydrogenase enzymes involved in the AAA pathway have been intensively studied and compared in different organisms, and their technical features, including their nucleotides and aminoacids sequences, kinetics, substrate specificity, function, 3D- structure, as well as the way to purify and characterize them, are well known from the skilled man (see Xu et al., 2001, the content of which is incorporated herein by way of reference). Numerous genes from different fungi or oomycetes and their sequence data are disclosed and available in searchable public database, such as genBank.
Randall, T.A. et al. (2005) reports a list of genes of AAA pathway from the oomycete *Phytophthora infestans,* identified by their accession numbers and the sequence data thereof, incorporated herein by reference, are available in searchable public database (s) (Randall, T.A., 2005, MPMI, 18, 229-243; see in particular table 8 p 239).

In one embodiment, the present invention provides a dsRNA molecule comprising 1) a first strand comprising a sequence substantially identical to at least 18 contiguous nucleotides of a fungus or oomycete gene and ii) a second strand comprising a sequence substantially complementary to the first strand, wherein said fungus or oomycete gene is a saccharopine dehydrogenase gene.

As used herein, "RNAi" or "RNA interference" refers to the process of sequence- specific gene silencing, mediated by double-stranded RNA (dsRNA). As used herein, "dsRNA" or "dsRNA molecule" refers to RNA that is partially or completely double stranded. Double stranded RNA is also referred to small or short interfering RNA (siRNA), short interfering nucleic acid (siNA), short interfering RNA, micro-RNA (miRNA), circular interfering RNA (ciRNA), short hairpin RNA (shRNA) and the like.

As used herein, taking into consideration the substitution of uracil for thymine when comparing RNA and DNA sequences, the term "substantially identical" or "essentially homologous" as applied to dsRNA means that the nucleotide sequence of one strand of the dsRNA is at least about 80%, at least 85% identical to 18 or more contiguous nucleotides of the target gene, more preferably at least about 90 % identical to 18 or more contiguous nucleotides of the target gene, and most preferably at least about 95%, 96%, 97%, 98% or 99% identical or absolutely identical to 18 or more contiguous nucleotides of the target gene. 18 or more nucleotides means a portion, being at least about 18, 20, 21 , 22, 23, 24, 25, 50, 100, 200, 300, 400, 500, 1000, 1500, or 2000 consecutive bases or up to the full length of the target gene.

As used herein, "complementary" polynucleotides are those that are capable of base pairing according to the standard Watson-Crick complementarity rules. Specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. As used herein, the term "substantially complementary" means that two nucleic acid sequences are complementary over at least 80% of their nucleotides. Preferably, the two nucleic acid sequences are complementary over at least 85%, 90%, 95%, 96%, 97%, 98%, 99% or more or all of their nucleotides. Alternatively, "substantially complementary" means that two nucleic acid sequences can hybridize under high stringency conditions. As used herein, the term "substantially identical" or "corresponding to" means that two nucleic acid sequences have at least 80% sequence identity. Preferably, the two nucleic acid sequences have at least 85%, 90%, 95%, 96%, 97%, 98%, 99% or 100% of sequence identity.

Also as used herein, the terms "nucleic acid" and "polynucleotide" refer to RNA or DNA that is linear or branched, single or double stranded, or a hybrid thereof. The term also encompasses RNA/DNA hybrids. When dsRNA is produced synthetically, less common bases, such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, locked nucleic acid or the 2'-hydroxy in the ribose sugar group of the RNA can also be made.

Accordingly to the invention, the first strand and second strand may have identical sizes. Alternatively, the size of the first strand may be greater than that of the second strand. By way of example, the size of the first strand can be about 200 nucleotides greater than the size of the second strand. In another aspect of the invention, the size of second strand is greater than that of the first strand.

Accordingly to the invention, the dsRNA molecule comprises a first strand comprising a sequence substantially identical to at least 18 contiguous nucleotides of a fungus or oomycete saccharopine dehydrogenase gene.

In a particular embodiment, the invention provides a dsRNA molecule comprising 1) a first strand comprising a sequence substantially identical to at least 18 contiguous nucleotides of a fungus or oomycete gene and ii) a second strand comprising a sequence substantially complementary to the first strand, wherein said fungus or oomycete gene is selected from the list consisting of:
a) a polynucleotide comprising a sequence as set forth in SEQ ID N0: 1, 3, 5, 7, 9, 11, 13, 15, 17, 19, 21, 23, 25, 27, 29, 31, 33, 35, 37, 39, 41, 43;
b) a polynucleotide encoding a polypeptide having a sequence as set forth in SEQ ID N0: 2, 4, 6, 8, 10, 12, 14, 16, 18, 20, 22, 24, 26, 28, 30, 32, 34, 36, 38, 40, 42, 44;
c) a polynucleotide having at least 70% sequence identity, preferably at least 80%, more preferably at least 90%, even more preferably at least 95% to a polynucleotide having a sequence as set forth in SEQ ID N0: 1, 3, 5, 7, 9, 11, 13, 15, 17, 19, 21, 23, 25, 27, 29, 31, 33, 35, 37, 39, 41, 43;
d) a polynucleotide encoding a polypeptide having at least 70% sequence identity, preferably at least 80%, more preferably at least 90%, even more preferably at least 95%, to a polypeptide having a sequence as set forth in SEQ ID N0: 2, 4, 6, 8, 10, 12, 14, 16, 18, 20, 22, 24, 26, 28, 30, 32, 34, 36, 38, 40, 42, 44:
e) a polynucleotide hybridizing under stringent conditions to a polynucleotide having a sequence as set forth in SEQ ID N0: 1, 3, 5, 7, 9, 11, 13, 15, 17, 19, 21, 23, 25, 27, 29, 31, 33, 35, 37, 39, 41, 43; and
f) a polynucleotide hybridizing under stringent conditions to a polynucleotide encoding a polypeptide having at least 70% sequence identity to a polypeptide having a sequence as set forth in SEQ ID N0: 2, 4, 6, 8, 10, 12, 14, 16, 18, 20, 22, 24, 26, 28, 30, 32, 34, 36, 38, 40, 42, 44.

In accordance with the present invention, the term "identity" is to be understood to mean the number of amino acids/nucleotides corresponding with the amino acids/nucleotides of other protein/nucleic acid, expressed as a percentage. Identity is preferably determined by comparing the Seq. ID disclosed herein with other protein/nucleic acid with the help of computer programs. If sequences that are compared with one another have different lengths, the identity is to be determined in such a way that the number of amino acids, which have the shorter sequence in common with the longer sequence, determines the percentage quotient of the identity. Preferably, identity is determined by means of the computer program ClustalW, which is well known and available to the public (Thompson et al., 1994). ClustalW is made publicly available on
http://www.ebi.ac.uk/tools/clustalW2lindex.html.
Preferably, Version 2.1 of the ClustalW computer program is used to determine the identity between proteins according to the invention and other proteins. In doing so, the following parameters must be set: KTUPLE=1, TOPDIAG=5, WINDOW=5, PAIRGAP=3, GAPOPEN=10, GAPEXTEND=0.05,
GAPDIST=8, MAXDIV=40, MATRIX=GONNET, ENDGAPS(OFF), NOPGAP, NOHGAP.
Preferably, Version 2.1 of the ClustalW computer program is used to determine the identity between the nucleotide sequence of the nucleic acid molecules according to the invention, for example, and the nucleotide sequence of other nucleic acid molecules. In doing so, the following parameters must be set:
KTUPLE=2, TOPDIAGS=4, PAIRGAP=5, DNAMATRIX:IUB, GAPOPEN=10, GAPEXT=5, MAXDIV=40, TRANSITIONS: unweighted.

In accordance with the present invention, the term 'hybridizing under stringent conditions" refers to polynucleotides or nucleic acid sequences which hybridize with a reference nucleic acid sequence at a level significantly greater than the background noise. The background noise may be associated with the hybridization of other DNA sequences present, in particular of other cDNAs present in a cDNA library. The level of the signal generated by the interaction between the sequence capable of selectively hybridizing and the sequences defined by the sequence IDs above according to the invention is generally 10 times, preferably 100 times, greater than that of the interaction of the other DNA sequences generating the background noise. The level of interaction can be measured, for example, by labeling the probe with radioactive elements such as ³²P. The selective hybridization is generally obtained by using very severe conditions for the medium (for example 0.03 M NaCl and 0.03 M sodium citrate at approximately 50°C-60°C). The hybridization can of course be carried out according to the usual methods of the state of the art (in particular Sambrook et al., 2001, Molecular Cloning: A Laboratory Manual, third edition).

In a particular embodiment of the invention, the dsRNA molecule is applied on the plant pathogen, particularly fungi or oomycete, and/or on the plant or crop to be protected. The present invention therefore also relates to a composition comprising an effective and non-phytotoxic amount of a dsRNA molecule as defined herein.

dsRNA molecules accordingly to the invention may be made by classical chemical synthesis, by means of *in vitro* transcription or produced in organisms like animals cells, bacteria, yeasts, or plants by heterologous expression (Aalto A.P. et al, 2007 RNA. 13(3):422-9.)

The present invention therefore relates to a micro-organism producing a dsRNA molecule as herein defined.

The present invention also relates to a genetic construct which comprises at least one DNA sequence as well as heterologous regulatory element(s) in the 5' and optionally in the 3' positions, characterized in that the DNA sequence is able to form a dsRNA molecule as herein defined.
The present invention also relates to a cloning and/or expression vector, characterized in that it contains at least one genetic construct as herein defined.

The expression "effective and non-phytotoxic amount" means an amount of composition according to the invention which is sufficient to control or destroy the pathogen present or liable to appear on the crops and which does not entail any appreciable symptom of phytotoxicity for the said crops. Such an amount can vary within a wide range depending on the pathogen to be controlled, the type of crop, the climatic conditions and the compounds included in the composition according to the invention. This amount can be determined by systematic field trials, which are within the capabilities of a person skilled in the art.

Thus, according to the invention, there is provided a composition comprising, as an active ingredient, an effective amount of a dsRNA molecule as herein defined and an agriculturally acceptable support, carrier, filler and/or surfactant.

According to the invention, the term "support" denotes a natural or synthetic organic or inorganic compound with which the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol organic solvents, mineral and plant oils and derivatives thereof. Mixtures of such supports can also be used.

The composition according to the invention can also comprise additional components such as, but not limited to, surfactant, protective colloids, adhesives, thickeners, thixotropic agents, penetration agents, stabilisers, sequestering agents. More generally, the active compounds can be combined with any solid or liquid additive, which complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions which are ready to be applied to the plant or seed to be treated by means of a suitable device, such as a spraying or dusting device, but also concentrated commercial compositions which must be diluted before application to the crop.

The compounds according to the invention can also be mixed with one or more phytopharmaceutical or plant growth promoting compound, such as a fungicide, herbicide, insecticide, nematicide, acaricide, molluscicide, resistance inducer, safeners, signal compounds, biologicals, pheromone active substance or other compounds with biological activity. The mixtures thus obtained have a broadened spectrum of activity. The mixtures with other fungicide compounds are particularly advantageous.

In a particular embodiment of the invention, the dsRNA is introduced or produced into the plant to be protected. After introduction or production into the plant, the dsRNA may further be processed into relatively small fragments (siRNAs) and can subsequently become distributed throughout the plant. Alternatively, the dsRNA is introduced or produced into the plant using a regulatory element or promoter that results in expression of the dsRNA in a tissue, temporal, spatial or inducible manner and may further be processed into relatively small fragments by a plant cell containing the RNAi processing machinery.

The invention therefore relates to a genetic construct or chimeric gene which is able to produce the dsRNA of the invention inside a plant cell. Said genetic construct or chimeric gene comprises at least one DNA sequence as well as well as heterologous regulatory element(s) in the 5' and optionally in the 3' positions which are able to function in a plant, characterized in that the DNA sequence(s) is (are) able to form a dsRNA molecule as herein defined once expressed in the plant.

In a particular embodiment, the genetic construct or chimeric gene comprises:
- a promoter regulatory sequence that is functional in plant cells, operably linked to
- a DNA sequence which, when it is transcribed, generates an RNA molecule comprising at least a sense sequence and an antisense sequence which are at least partially complementary, said sense sequence comprising a sequence substantially identical to at least 18 contiguous nucleotides of a target gene (i.e in the meaning of the invention a saccharopine dehydrogenase gene), and said antisense sequence comprising a sequence substantially complementary to the sense sequence, and
- optionally a terminator regulatory sequence.

In said embodiment, the DNA sequence according to the invention may have more particularly two aspects; in the first, it comprises two nucleotide sequences, which are sense and antisense, separated by a spacer nucleotide sequence or an intron that does not exhibit any homology with the target gene. The sequence cloned in the sense and antisense orientation is that whose expression in the pathogen it is intended to inhibit. The transcription of this DNA sequence thus gives a large single-stranded RNA corresponding to the sense/spacer-intron/antisense construct. This long RNA transcript can be detected by RT-PCR. Since the sense and antisense sequences are homologous, they will pair, and the spacer or intron which separates them plays the role of a loop for folding. A dsRNA is then obtained over all the homologous regions. The dsRNA is subsequently specifically degraded by an enzymatic complex called "DICER". The degradation of the dsRNAs then forms siRNAs ("siRNA" in the figure), small double-stranded RNAs having a size of between 19 and 25 bases. These are then the siRNAs which, by pairing with the transcribed RNAs derived from the target gene will lead to their degradation via the RNA silencing machinery enzymatic machinery.
In the second aspect, the DNA sequence comprises two nucleotide sequences, which are sense and antisense, of different sizes, the loop structure corresponding to the part of the nucleotide sequence that does not exhibit any homology with the other nucleotide sequence. The nucleotide sequence cloned in the sense orientation is essentially homologous to the sequence of the target gene whose expression it is intended to inhibit. The antisense nucleotide sequence is essentially homologous to the complementary strand of the sequence of said target gene. The transcription of this DNA sequence thus gives a large single-stranded RNA corresponding to the sense/antisense construct. This long RNA transcript can be detected by RT-PCR. The homologous sense/antisense sequences are paired. A dsRNA is then obtained over all the homologous regions. The dsRNA is subsequently specifically degraded by an enzymatic complex called "DICER". The degradation of the dsRNAs then forms siRNAs ("siRNA" in the figure), small doubled-stranded RNAs having a size of between 18 and 25 bases. These are then the siRNAs which, by pairing with the target RNAs, will lead to their degradation via the RNA silencing machinery plant's enzymatic machinery.

In another particular embodiment, the genetic construct comprises:
- two promoter regulatory sequences that are functional in plant cells, wherein the first promoter regulatory sequence is operably linked to a DNA sequence which, when it is transcribed, generates an RNA molecule comprising at least a sense sequence, and the second promoter regulatory sequence is operably linked to a DNA sequence which, when it is transcribed, generates an RNA molecule comprising at least an antisense sequence partially complementary to the sense sequence, and wherein said sense sequence comprises a sequence substantially identical to at least 18 contiguous nucleotides of the target gene, and
- optionally terminator regulatory sequence(s).
In this particular embodiment, the genetic construct may be comprised as two chimeric genes, one comprising the first promoter regulatory sequence operably linked to the first DNA sequence which, when it is transcribed, generates an RNA molecule comprising at least a sense sequence substantially identical to at least 18 contiguous nucleotides of the target gene, and optionally a terminator regulatory sequence, and the second chimeric genes comprising the second promoter regulatory sequence operably linked to the second DNA sequence which, when it is transcribed, generates an RNA molecule comprising at least an antisense sequence partially complementary to the sense sequence, and optionally a terminator regulatory sequence.
These two chimeric genes are preferably introduced into the plant cell conjointly, but not neccessary, in order to favorize the hybridization of the two RNA single strands to form the dsRNA.

Alternatively, the genetic construct may be comprised as a construct comprising:
- a first promoter, operably linked to
- a double strand DNA sequence wherein one strand when it is transcribed under the control of the first promoter, generates an RNA molecule comprising at least a sense sequence substantially identical to at least 18 contiguous nucleotides of the target gene, and optionally a terminator regulatory sequence, and wherein the second strand, when it is transcribed under the control of the second promoter, generates an RNA molecule comprising at least an antisense sequence partially complementary to the sense sequence, and optionally a terminator regulatory sequence, and
- a second promoter, in the opposite direction that the first one.

First and second promoter regulatory sequences may be different or identical, preferably different.

The invention further relates to a cloning and/or expression vector for transforming a plant, characterized in that it contains at least one chimeric gene or genetic construct as defined herein.

The present invention further relates to a transgenic plant cell containing the dsRNA molecule of the invention and as herein defined.
The present invention therefore relates to a transgenic plant cell containing the genetic construct or chimeric gene of the invention as herein defined.

In a particular embodiment of the invention, the transgenic plant cell is a soybean, oilseed, rice or potato plant cell.

The present invention further relates to a transgenic plant, seed or part thereof, comprising a transgenic plant cell according to the invention.

In a particular embodiment of the invention, the transgenic plant, seed or part thereof, is a soybean, oilseed, rice or potato plant, seed or part thereof.

According to the invention, the expression "chimeric gene" or "expression cassette" is intended to mean a nucleotide sequence comprising, functionally linked to one another in the direction of transcription, a regulatory promoter sequence that is functional in plants, a sequence encoding a protein or an RNA chain, and, optionally, a terminator that is functional in plant cells. The term "chimeric gene" or "expression cassette" is generally intended to mean a gene for which certain elements are not present in the native gene, but have been substituted for elements present in the native gene or have been added.
According to the invention, the terms "chimeric gene" or "expression cassette" may also correspond to the case where all the elements of the gene are present in the native gene, and alternatively, the term "gene" may correspond to a chimeric gene.
The expression "chimeric gene" or "expression cassette" may also correspond to the case where the sequence encoding a protein or a RNA chain is not directly linked to a promoter, but is part, for example, of a polycistronic construct comprising several coding sequences under the control of the same promoter. In that case, each coding sequences under the control of the promoter is designed as a "chimeric gene" or "expression cassette".

According to the invention, the expression "functionally linked to one another" means that said elements of the elemental chimeric gene are linked to one another in such a way that their function is coordinated and allows the expression of the coding sequence. By way of example, a promoter is functionally linked to a coding sequence when it is capable of ensuring the expression of said coding sequence. The construction of the chimeric gene according to the invention and the assembly of its various elements can be carried out using techniques well known to those skilled in the art, in particular those described in Sambrook et al. (2001, Molecular Cloning : A Laboratory Manual (third edition), Nolan C. ed., New York: Cold Spring Harbor Laboratory Press). The choice of the regulatory elements constituting the chimeric gene depends essentially on the plant and on the type of cell in which they must function, and those skilled in the art are capable of selecting regulatory elements that are functional in a given plant.

According to the invention, the term "promoter regulatory sequence" is intended to mean any promoter regulatory sequence of a gene that is naturally expressed in plants, in particular a promoter that is expressed especially in the leaves of plants, for instance promoters referred to as constitutive of bacterial, viral or plant origin, or else promoters referred to as light-dependent, such as that of a plant ribulose-biscarboxylase/oxygenase (RuBisCO) small subunit gene, or any known suitable promoter that can be used. Among the promoters of plant origin, mention will be made of the histone promoters as described in application EP 0 507 698, or the rice actin promoter (US 5,641,876). Among the promoters of a plant virus gene, mention will be made of that of the cauliflower mosaic virus (CaMV 19S or 35S) or of the cassava vein mosaic virus (CsVMV: WO97/48819) or the circovirus promoter (AU 689 311). Use may also be made of a promoter regulatory sequence specific for particular regions or tissues of plants, and more particularly seed-specific promoters (Datla, R. et al., 1997, Biotechnology Ann. Rev., 3, 269-296), especially the napin (EP 255 378), phaseolin, glutenin, helianthinin (WO 92/17580), albumin (WO 98/45460) and oleosin (WO 98/45461) promoters. An inducible promoter can also be used, it can be advantageously chosen from the promoters of phenylalanine ammonia lyase (PAL), of HMG-CoA reductase (HMG), of chitinases, of glucanases, of proteinase inhibitors (PI), of genes of the PR1 family, of nopaline synthase (nos) or of the vspB gene (US 5 670 349), the HMG2 promoter (US 5 670 349), the apple beta-galactosidase (ABG1) promoter or the apple amino cyclopropane carboxylate synthase (ACC synthase) promoter (WO 98/45445).

The term "terminator regulatory sequence" is intended to mean any sequence that is functional in plant cells or plants, also comprising polyadenylation sequences, whether they are of bacterial origin, for instance the nos or ocs terminator of *Agrobacterium tumefaciens*, of viral origin, for instance the CaMV 35S terminator, or else of plant origin, for instance a histone terminator as described in application EP 0 633 317.

The selection step for identifying the transformed cells and/or plants having integrated the construct according to the invention can be carried out by virtue of the presence of a selectable gene present in the construct according to the invention or in the plasmid used for the transformation of the cells or of the plants and comprising said construct. The selectable gene may be in the form of a chimeric gene comprising the following elements, functionally linked in the direction of transcription: a promoter regulatory sequence that is functional in plant cells, a sequence encoding a selectable marker, and a terminator regulatory sequence that is functional in plant cells.

Among the selectable markers that can be used, mention may be made of markers containing genes for resistance to antibiotics, such as, for example, that of the hygromycin phosphotransferase gene (Gritz et al., 1983, Gene 25: 179-188), of the neomycin phosphotransferase II gene inducing resistance to kanamycin (Wirtz et al., 1987, DNA, 6(3): 245-253), or of the aminoglycoside 3"-adenyltransferase gene, but also markers containing genes for tolerance to herbicides, such as the *bar* gene (White et al., NAR 18: 1062, 1990) for tolerance to bialaphos, the EPSPS gene (US 5,188,642) for tolerance to glyphosate or else the HPPD gene (WO 96/38567) for tolerance to isoxazoles. Mention may also be made of genes encoding readily indentifiable enzymes, such as the GUS enzyme, GFP protein or genes encoding pigments or enzymes regulating pigment production in the transformed cells. Such selectable marker genes are in particular described in patent applications WO 91/02071, WO 95/06128, WO 96/38567, and WO 97/04103.

The present invention further relates to a method of making a transgenic plant cell or plant capable of expressing a dsRNA that inhibits a fungus or oomycete saccharopine dehydrogenase gene, wherein said method comprises the steps of transforming a plant cell with a chimeric gene or genetic construct according to the invention.
The method may further comprise the step of selecting the plant cell which has been transformed.

In a particular embodiment of the invention, the invention relates to a method of making a transgenic plant cell or plant capable of expressing a dsRNA that inhibits fungus or oomycete saccharopine dehydrogenase gene , wherein said method comprises the steps of transforming a plant cell with a chimeric gene or genetic construct according to the invention, and wherein said plant cell is a soybean, oilseed, rice or potato plant cell or said plant is a soybean, oilseed, rice or potato plant.

The present invention also relates to the transformed plants or part thereof, and to plants or part thereof which are derived by cultivating and/or crossing the above regenerated plants, and to the seeds of the transformed plants.
The present invention also relates to the end products such as the meal, oil, fiber which are obtained from the plants, part thereof, or seeds of the invention.

To obtain the cells or plants according to the invention, those skilled in the art can use one of the numerous known methods of transformation.
One of these methods consists in bringing the cells or tissues of the host organisms to be transformed into contact with polyethylene glycol (PEG) and the vectors of the invention (Chang and Cohen, 1979, Mol. Gen. Genet. 168(1), 111-115; Mercenier and Chassy, 1988, Biochimie 70(4), 503-517). Electroporation is another method, which consists in subjecting the cells or tissues to be transformed and the vectors of the invention to an electric field (Andreason and Evans, 1988, Biotechniques 6(7), 650-660; Shigekawa and Dower, 1989, Aust. J. Biotechnol. 3(1), 56-62). Another method consists in directly injecting the vectors into the cells or the tissues by microinjection (Gordon and Ruddle, 1985, Gene 33(2), 121-136). Advantageously, the "biolistic" method may be used. It consists in bombarding cells or tissues with particles onto which the vectors of the invention are adsorbed (Bruce et al., 1989, Proc. Natl. Acad. Sci. USA 86(24), 9692-9696; Klein et al., 1992, Biotechnology 10(3), 286-291; US Patent No. 4,945,050). Preferably, the transformation of plant cells or tissues can be carried out using bacteria of the *Agrobacterium* genus, preferably by infection of the cells or tissues of said plants with A. *tumefaciens* (Knopf, 1979, Subcell. Biochem. 6, 143-173; Shaw et a/., 1983, Gene 23(3): 315-330) or *A. rhizogenes* (Bevan and Chilton, 1982, Annu. Rev. Genet. 16: 357-384; Tepfer and Casse-Delbart, 1987, Microbiol. Sci. 4(1), 24-28). Preferably, the transformation of plant cells or tissues with *Agrabacterium tumefaciens* is carried out according to the protocol described by Hiei et al., (1994, Plant J. 6(2): 271-282). Those skilled in the art will choose the appropriate method according to the nature of the host organisms to be transformed.

The plants according to the invention contain transformed plant cells as defined above. In particular, the transformed plants can be obtained by regeneration of the transformed plant cells described above. The regeneration is obtained by any appropriate method, which depends on the nature of the species.

The invention also comprises parts of these plants, and the progeny of these plants. The term "part of these plants" is intended to mean any organ of these plants, whether above ground or below ground. The organs above ground are the stems, the leaves and the flowers comprising the male and female reproductive organs. The organs below ground are mainly the roots, but they may also be tubers. The term "progeny" is intended to mean mainly the seeds containing the embryos derived from the reproduction of these plants with one another. By extension, the term "progeny" applies to all the seeds formed at each new generation derived from crosses between the transformed plants according to the invention. Progeny and seeds can also be obtained by vegetative multiplication of said transformed plants. The seeds according to the invention can be coated with an agrochemical composition comprising at least one active product having an activity selected from fungicidal, herbicidal, insecticidal, nematicidal, bactericidal or virucidal activities.

The invention further relates to a method for controlling a plant pathogen, particularly a fungus or an oomycete, comprising providing to said pathogen a dsRNA molecule according to the invention and as herein defined.

In a particular embodiment of the invention, the method relates to a method for controlling a plant pathogen, particularly a fungus or an oomycete, comprising providing to said pathogen a dsRNAaccording to the invention and as herein defined , or a composition comprising said dsRNA, wherein said plant pathogen is *Magnaporthe grisea, Phytophthora infestans*, *Sclerotinia sclerotiomm* or *Phakopsora pachyrhizi.*

In a particular embodiment of the invention, the method relates to a method for controlling a plant pathogen, particularly a fungus or an oomycete, comprising providing to said pathogen a dsRNA molecule according to the invention and as herein defined , or a composition comprising said dsRNA, wherein said plant is a soybean, oilseed, rice or potato plant.

The invention further relates to a method for controlling a plant, crop or seed pathogen, particularly a fungus or an oomycete, characterized in that an agronomically effective and substantially non-phytotoxic quantity of dsRNA molecule according to the invention or composition according to the invention is applied as seed treatment, foliar application, stem application, drench or drip application (chemigation) to the seed, the plant or to the fruit of the plant or to soil or to inert substrate (e.g. inorganic substrates like sand, rockwool, glasswool; expanded minerals like perlite, vermiculite, zeolite or expanded clay), Pumice, Pyroclastic materials or stuff, synthetic organic substrates (e.g. polyurethane) organic substrates (e.g. peat, composts, tree waste products like coir, wood fibre or chips, tree bark) or to a liquid substrate (e.g. floating hydroponic systems, Nutrient Film Technique, Aeroponics) wherein the plant is growing or wherein it is desired to grow.

The invention therefore relates to a method for controlling a plant pathogen, particularly a fungus or oomycete, characterized in that an effective quantity of a dsRNA molecule according to the invention or a composition according to the invention is applied to the soil where plants grow or are capable of growing, to the leaves and/or the fruit of plants or to the seeds of plants.

In a particular embodiment of the invention, the invention relates to a method for controlling a plant pathogen, particularly a fungus or oomycete, characterized in that an effective quantity of a dsRNA molecule according to the invention or a composition according to the invention is applied to the soil where plants grow or are capable of growing, to the leaves and/or the fruit of plants or to the seeds of plants, wherein said plant pathogen is Magnaporthe grisea, Phytophthora infestans, Sclerotinia sclerotinium or Phakopsora pachyrhizi.

In a particular embodiment of the invention, the invention relates to a method for controlling a plant pathogen, particularly a fungus or oomycete, characterized in that an effective quantity of a dsRNA molecule according to the invention or a composition according to the invention is applied to the soil where plants grow or are capable of growing, to the leaves and/or the fruit of plants or to the seeds of plants, wherein said plant is a soybean, oilseed, rice or potato plant.

The expression "are applied to the plants to be treated" is understood to mean, for the purposes of the present invention, that the pesticide composition which is the subject of the invention can be applied by means of various methods of treatment such as:
- spraying onto the aerial parts of the said plants a liquid comprising one of the said compositions,
- dusting, the incorporation into the soil of granules or powders, spraying, around the said plants and in the case of trees injection or daubing,
- coating or film-coating the seeds of the said plants with the aid of a plant-protection mixture comprising one of the said compositions.
The method according to the invention can either be a curing, preventing or eradicating method.
In this method, a composition used can be prepared beforehand by mixing the two or more active compounds according to the invention.

According to an alternative of such a method, it is also possible to apply simultaneously, successively or separately compounds (A) and (B) so as to have the conjugated (A)/(B) effects, of distinct compositions each containing one of the two or three active ingredients (A) or (B).

The dose of active dsRNA compound usually applied in the method of treatment according to the invention is generally and advantageously
- for foliar treatments: from 0.0001 to 10,000 g/ha, preferably from 0,0001 to 1000 g/ha, more preferably from 0.001 to 300g/ha; in case of drench or drip application, the dose can even be reduced, especially while using inert substrates like rockwool or perlite;
- for seed treatment: from 0.0001 to 200 g per 100 kilogram of seed, preferably from 0.001 to 150 g per 100 kilogram of seed;
- for soil treatment: from 0.0001 to 10,000 g/ha, preferably from 0.001 to 5,000 g/ha.

When the dsRNA of the invention is mixed with another active phytopharmaceutical or plant growth promoting compound compound, said phytopharmaceutical or plant growth promoting compound is used in the dose usually applied.
Said phytopharmaceutical or plant growth promoting compound may be a fungicide, herbicide, insecticide, nematicide, acaricide, molluscicide, resistance inducer, safeners, or signal compounds. The dose of phytopharmaceutical active compound usually applied in the method of treatment according to the invention is generally and advantageously from 10 to 800 g/ha, preferably from 50 to 300 g/ha for applications in foliar treatment. The dose of active substance applied is generally and advantageously from 2 to 200 g per 100 kg of seed, preferably from 3 to 150 g per 100 kg of seed in the case of seed treatment.
The doses herein indicated are given as illustrative Examples of method according to the invention. A person skilled in the art will know how to adapt the application doses, notably according to the nature of the plant or crop to be treated.
Under specific conditions, for example according to the nature of the pathogen, phytopathogenic fungus or oomycete to be treated or controlled, a lower dose can offer adequate protection. Certain climatic conditions, resistance or other factors like the nature of the pathogen or the degree of infestation, for example, of the plants with these pathogens, can require higher doses of combined active ingredients. The optimum dose usually depends on several factors, for example on the type of pathogen to be treated, on the type or level of development of the infested plant or plant material, on the density of vegetation or alternatively on the method of application.
Without it being limiting, the crop treated with the pesticide composition or combination according to the invention is, for example, grapevine, cereals, vegetables, lucerne, soybean, market garden crops, turf, wood, tree or horticultural plants.
The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the over-ground parts of the plant such as trunks, stems or stalks, leaves, flowers and fruit of the concerned plant, and in general every material which is susceptiblel to fungal infection (e.g due to storage like hay)

The invention further relates to a method of controlling a plant pathogen, particularly a fungus or an oomycete, comprising providing in the host plant of said plant pathogen a transformed plant cell according to the invention.
The invention further relates to a method of controlling a plant pathogen, particularly a fungus or an oomycete, comprising providing in the host plant of said plant pathogen a transformed plant cell containing a dsRNA as herein defined.
The invention further relates to a method of controlling a plant pathogen, particularly a fungus or an oomycete, comprising transforming the plant with a genetic construct according to the invention.

The invention further relates to a method of controlling a plant pathogen, particularly a fungus or an oomycete, comprising the following steps:
i) transforming a plant cell with a chimeric gene according to the invention;
ii) placing the cells thus transformed under conditions that allow the transcription of said construct,
iii) having the cells into contact with the pathogen.

In a particular embodiment of the invention, methods according to the invention are controlling a plant pathogen selected from Magnaporthe grisea, Phytophthora infestans, Sclerotinia sclerotinium or Phakopsora pachyrhizi.

In a particular embodiment of the invention, methods according to the invention are controlling a plant pathogen wherein the plant is a soybean, oilseed, rice or potato plant.

The invention further relates to a method for inhibiting the expression of a plant pathogen, particularly fungus or oomycete, saccharopine dehydrogenase gene, comprising the following steps:
i) transforming a plant cell with a chimeric gene according to the invention;
ii) placing the cells thus transformed under conditions that allow the transcription of said construct,
iii) having the cells into contact with the pathogen.

In a particular embodiment of the invention, the method according to the invention is inhibiting a fungal or oomycete saccharopine dehydrogenase gene , wherein the fungus or oomycete is Magnaporthe grisea, Phytophthora infestans, Sclerotinia sclerotinium or Phakopsora pachyrhizi.

In a particular embodiment of the invention, the method according to the invention inhibites a fungal or oomycete saccharopine dehydrogenase gene , said method comprises the following steps:
i) transforming a plant cell with a chimeric gene according to the invention;
ii) placing the cells thus transformed under conditions that allow the transcription of said construct,
iii) having the cells into contact with the pathogen;
wherein the plant is a soybean, oilseed, rice or potato plant.

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.
Among the plants that can be protected by the method according to the invention, mention may be made of major field crops like corn, soybean, cotton, *Brassica* oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp.,*
*Musaceae sp.* (for instance banana trees and plantings), *Rubiaceae sp.* (for instance coffee), *Theaceae sp., Sterculiceae sp., Rutaceae sp.* (for instance lemons, oranges and grapefruit); *Solanaceae sp.* (for instance tomatoes, potatoes, peppers, eggplant), *Liliaceae sp.,* Compositiae sp. (for instance lettuce, artichoke and chicory - including root chicory, endive or common chicory), *Umbelliferae sp.* (for instance carrot, parsley, celery and celeriac), *Cucurbitaceae sp.* (for instance cucumber - including pickling cucumber, squash, watermelon, gourds and melons), *Alliaceae* sp. (for instance onions and leek), Cruciferae sp. (for instance white cabbage, red cabbage, broccoli, cauliflower, brussel sprouts, pak choi, kohlrabi, radish, horseradish, cress, Chinese cabbage), Leguminosae sp. (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology or RNA interference - RNAi - technology). A heterologous gene that is located in the genome is also called a transgene. A transgene that is defined by its particular location in the plant genome is called a transformation or transgenic event.
Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted microorganisms. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted microorganisms, the treated plants display a substantial degree of resistance to these microorganisms. In the present case, unwanted microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts" or "parts of plants" or "plant parts" have been explained above. More preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted microorganisms. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted microorganisms, the treated plants display a substantial degree of resistance to these microorganisms. In the present case, unwanted microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses). Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (WO 92/05251, WO 95/09910, WO 98/27806, WO 05/002324, WO 06/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 89/10396 in which, for example, a ribonuclease such as barnase is selectively expressed in the tapetum cells in the stamens. Fertility can then be restored by expression in the tapetum cells of a ribonuclease inhibitor such as barstar (e.g. WO 91/02069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium Salmonella typhimurium (Science 1983, 221, 370-371), the CP4 gene of the bacterium Agrobacterium sp. (Curr. Topics Plant Physiol. 1992, 7, 139-145), the genes encoding a Petunia EPSPS (Science 1986, 233, 478-481), a Tomato EPSPS (J. Biol. Chem. 1988, 263, 4280-4289), or an Eleusine EPSPS (WO 01/66704). It can also be a mutated EPSPS as described in for example EP 0837944, WO 00/66746, WO 00/66747 or WO 02/26995. Glyphosate-tolerant plants can also be obtained by expressing a gene that encodes a glyphosate oxido-reductase enzyme as described in US 5,776,760 and US 5,463,175. Glyphosate-tolerant plants can also be obtained by expressing a gene that encodes a glyphosate acetyl transferase enzyme as described in for example WO 02/036782, WO 03/092360, WO 2005/012515 and WO 2007/024782. Glyphosate-tolerant plants can also be obtained by selecting plants containing naturally-occurring mutations of the above-mentioned genes, as described in for example WO 01/024615 or WO 03/013226.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One such efficient detoxifying enzyme is an enzyme encoding a phosphinothricin acetyltransferase (such as the bar or pat protein from Streptomyces species). Plants expressing an exogenous phosphinothricin acetyltransferase are for example described in U.S. Patents 5,561,236; 5,648,477; 5,646,024; 5,273,894; 5,637,489; 5,276,268; 5,739,082; 5,908,810 and 7,112,665.

Further herbicide-tolerant plants are also plants that are made tolerant to the herbicides inhibiting the enzyme hydroxyphenylpyruvatedioxygenase (HPPD). HPPD is an enzyme that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated or chimeric HPPD enzyme as described in WO 96/38567, WO 99/24585, WO 99/24586, WO 09/144079, WO 02/046387, or US 6,768,044. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme having prephenate deshydrogenase (PDH) activity in addition to a gene encoding an HPPD-tolerant enzyme, as described in WO 04/024928. Further, plants can be made more tolerant to HPPD-inhibitor herbicides by adding into their genome a gene encoding an enzyme capable of metabolizing or degrading HPPD inhibitors, such as the CYP450 enzymes shown in WO 2007/103567 and WO 2008/150473.

Still further herbicide resistant plants are plants that are made tolerant to acetolactate synthase (ALS) inhibitors. Known ALS-inhibitors include, for example, sulfonylurea, imidazolinone, triazolopyrimidines, pryimidinyoxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright (Weed Science 2002, 50, 700-712), but also, in U.S. Patents 5,605,011, 5,378,824, 5,141,870, and 5,013,659. The production of sulfonylurea-tolerant plants and imidazolinone-tolerant plants is described in U.S. Patents 5,605,011; 5,013,659; 5,141,870; 5,767,361; 5,731,180; 5,304,732; 4,761,373; 5,331,107; 5,928,937; and 5,378,824; and WO 96/33270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 2007/024782 and U.S. Patent Application 61/288958.

Other plants tolerant to imidazolinone and/or sulfonylurea can be obtained by induced mutagenesis, selection in cell cultures in the presence of the herbicide or mutation breeding as described for example for soybeans in US 5,084,082, for rice in WO 97/41218, for sugar beet in US 5,773,702 and WO 99/057965, for lettuce in US 5,198,599, or for sunflower in WO 01/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:
1) an insecticidal crystal protein from Bacillus thuringiensis or an insecticidal portion thereof, such as the insecticidal crystal proteins listed by Crickmore et al. (1998, Microbiology and Molecular Biology Reviews, 62: 807-813), updated by Crickmore et al. (2005) at the Bacillus thuringiensis toxin nomenclature, online at: http://www.lifesci.sussex.ac.uk/Home/Neil_Crickmore/Bt/), or insecticidal portions thereof, e.g., proteins of the Cry protein classes Cry1Ab, Cry1Ac, Cry1B, Cry1C, Cry1D, Cry1F, Cry2Ab, Cry3Aa, or Cry3Bb or insecticidal portions thereof (e.g. EP-A 1 999 141 and WO 2007/107302), or such proteins encoded by synthetic genes as e.g. described in and U.S. Patent Application 12/249,016; or
2) a crystal protein from Bacillus thuringiensis or a portion thereof which is insecticidal in the presence of a second other crystal protein from Bacillus thuringiensis or a portion thereof, such as the binary toxin made up of the Cry34 and Cry35 crystal proteins (Nat. Biotechnol. 2001, 19, 668-72; Applied Environm. Microbiol. 2006, 71, 1765-1774) or the binary toxin made up of the Cry1A or Cry1F proteins and the Cry2Aa or Cry2Ab or Cry2Ae proteins (U.S. Patent Application 12/214,022 and EP-A 2 300 618); or
3) a hybrid insecticidal protein comprising parts of different insecticidal crystal proteins from Bacillus thuringiensis, such as a hybrid of the proteins of 1) above or a hybrid of the proteins of 2) above, e.g., the Cry1A.105 protein produced by corn event MON89034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or
5) an insecticidal secreted protein from Bacillus thuringiensis or Bacillus cereus, or an insecticidal portion thereof, such as the vegetative insecticidal (VIP) proteins listed at: http://www.lifesci.sussex.ac.uk/home/Neil_Crickmore/Bt/vip.html, e.g., proteins from the VIP3Aa protein class; or
6) a secreted protein from Bacillus thuringiensis or Bacillus cereus which is insecticidal in the presence of a second secreted protein from Bacillus thuringiensis or B. cereus, such as the binary toxin made up of the VIP1A and VIP2A proteins (WO 94/21795); or
7) a hybrid insecticidal protein comprising parts from different secreted proteins from Bacillus thuringiensis or Bacillus cereus, such as a hybrid of the proteins in 1) above or a hybrid of the proteins in 2) above; or
8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102; or
9) a secreted protein from Bacillus thuringiensis or Bacillus cereus which is insecticidal in the presence of a crystal protein from Bacillus thuringiensis, such as the binary toxin made up of VIP3 and Cry1A or Cry1F or the binary toxin made up of the VIP3 protein and the Cry2Aa or Cry2Ab or Cry2Ae proteins EP-A 2 300 618).
10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein)

Of course, an insect-resistant transgenic plant, as used herein, also includes any plant comprising a combination of genes encoding the proteins of any one of the above classes 1 to 10. In one embodiment, an insect-resistant plant contains more than one transgene encoding a protein of any one of the above classes 1 to 10, to expand the range of target insect species affected when using different proteins directed at different target insect species, or to delay insect resistance development to the plants by using different proteins insecticidal to the same target insect species but having a different mode of action, such as binding to different receptor binding sites in the insect.
An "insect-resistant transgenic plant", as used herein, further includes any plant containing at least one transgene comprising a sequence producing upon expression a double-stranded RNA which upon ingestion by a plant insect pest inhibits the growth of this insect pest, as described e.g. in WO 2007/080126, WO 2006/129204, WO 2007/074405, WO 2007/080127 and WO 2007/035650.
Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
1) plants which contain a transgene capable of reducing the expression and/or the activity of poly(ADP-ribose) polymerase (PARP) gene in the plant cells or plants as described in WO 00/04173, WO 2006/045633, EP-A 1 807 519, or EP-A 2 018 431.
2) plants which contain a stress tolerance enhancing transgene capable of reducing the expression and/or the activity of the PARG encoding genes of the plants or plants cells, as described e.g. in WO 2004/090140.
3) plants which contain a stress tolerance enhancing transgene coding for a plant-functional enzyme of the nicotineamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP-A 1 794 306, WO 2006/133827, WO 2007/107326, EP-A 1 999 263, or WO 2007/107326.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as:
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP-A 0 571 427, WO 95/04826, EP-A 0 719 338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 04/056999, WO 05/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, WO 2008/017518, WO 2008/080630, WO 2008/080631, EP 07090007.1, WO 2008/090008, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026, WO 97/20936, WO 2010/012796, WO 2010/003701,
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP-A 0 663 956, WO 96/01904, WO 96/21023, WO 98/39460, and WO 99/24593, plants producing alpha-1,4-glucans as disclosed in WO 95/31553, US 2002031826, US 6,284,479, US 5,712,107, WO 97/47806, WO 97/47807, WO 97/47808 and WO 00/14249, plants producing alpha-1,6 branched alpha-1,4-glucans, as disclosed in WO 00/73422, plants producing alternan, as disclosed in e.g. WO 00/47727, WO 00/73422, EP 06077301.7, US 5,908,975 and EP-A 0 728 213,
3) transgenic plants which produce hyaluronan, as for example disclosed in WO 2006/032538, WO 2007/039314, WO 2007/039315, WO 2007/039316, JP-A 2006-304779, and WO 2005/012529.
4) transgenic plants or hybrid plants, such as onions with characteristics such as 'high soluble solids content', 'low pungency' (LP) and/or 'long storage' (LS).

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes as described in WO 98/00549.
b) Plants, such as cotton plants, containing an altered form of rsw2 or rsw3 homologous nucleic acids as described in WO 2004/053219.
c) Plants, such as cotton plants, with increased expression of sucrose phosphate synthase as described in WO 01/17333.
d) Plants, such as cotton plants, with increased expression of sucrose synthase as described in WO 02/45485.
e) Plants, such as cotton plants, wherein the timing of the plasmodesmatal gating at the basis of the fiber cell is altered, e.g. through downregulation of fiber-selective β-1,3-glucanase as described in WO 2005/017157, or as described in WO 2009/143995.
f) Plants, such as cotton plants, having fibers with altered reactivity, e.g. through the expression of N-acetylglucosaminetransferase gene including nodC and chitin synthase genes as described in WO 2006/136351.

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics and include:
a) Plants, such as oilseed rape plants, producing oil having a high oleic acid content as described e.g. in US 5,969,169, US 5,840,946 or US 6,323,392 or US 6,063,947
b) Plants such as oilseed rape plants, producing oil having a low linolenic acid content as described in US 6,270,828, US 6,169,190, or US 5,965,755
c) Plant such as oilseed rape plants, producing oil having a low level of saturated fatty acids as described e.g. in US 5,434,283.

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering as described in WO 2009/068313 and WO 2010/006732.

Plants or plant cultivars (that can be obtained by plant biotechnology methods such as genetic engineering) which may also be treated according to the invention are plants, such as Tobacco plants, with altered post-translational protein modification patterns, for example as described in WO 2010/121818 and WO 2010/145846.

Particularly useful transgenic plants which may be treated according to the invention are plants containing transformation events, or combination of transformation events, that are the subject of petitions for non-regulated status, in the United States of America, to the Animal and Plant Health Inspection Service (APHIS) of the United States Department of Agriculture (USDA) whether such petitions are granted or are still pending. At any time this information is readily available from APHIS (4700 River Road, Riverdale, MD 20737, USA), for instance on its internet site (URL http://www.aphis.usda.gov/brs/not_reg.html). On the filing date of this application the petitions for nonregulated status that were pending with APHIS or granted by APHIS were those which contains the following information:
- Petition: the identification number of the petition. Technical descriptions of the transformation events can be found in the individual petition documents which are obtainable from APHIS, for example on the APHIS website, by reference to this petition number. These descriptions are herein incorporated by reference.
- Extension of Petition: reference to a previous petition for which an extension is requested.
- Institution: the name of the entity submitting the petition.
- Regulated article: the plant species concerned.
- Transgenic phenotype: the trait conferred to the plants by the transformation event.
- Transformation event or line: the name of the event or events (sometimes also designated as lines or lines) for which nonregulated status is requested.
- APHIS documents: various documents published by APHIS in relation to the Petition and which can be requested with APHIS.

Additional particularly useful plants containing single transformation events or combinations of transformation events are listed for example in the databases from various national or regional regulatory agencies (see for example http://gmoinfo.jrc.it/gmp_browse.aspx and http://www.agbios.com/dbase.php).
Particularly useful transgenic plants which may be treated according to the invention are plants containing transformation events, or a combination of transformation events, and that are listed for example in the databases for various national or regional regulatory agencies including Event 1143-14A (cotton, insect control, not deposited, described in WO 2006/128569); Event 1143-51 B (cotton, insect control, not deposited, described in WO 2006/128570); Event 1445 (cotton, herbicide tolerance, not deposited, described in US-A 2002-120964 or WO 02/034946); Event 17053 (rice, herbicide tolerance, deposited as PTA-9843, described in WO 2010/117737); Event 17314 (rice, herbicide tolerance, deposited as PTA-9844, described in WO 2010/117735); Event 281-24-236 (cotton, insect control - herbicide tolerance, deposited as PTA-6233, described in WO 2005/103266 or US-A 2005-216969); Event 3006-210-23 (cotton, insect control - herbicide tolerance, deposited as PTA-6233, described in US-A 2007-143876 or WO 2005/103266); Event 3272 (corn, quality trait, deposited as PTA-9972, described in WO 2006/098952 or US-A 2006-230473); Event 40416 (corn, insect control - herbicide tolerance, deposited as ATCC PTA-11508, described in WO 2011/075593); Event 43A47 (corn, insect control - herbicide tolerance, deposited as ATCC PTA-11509, described in WO 2011/075595); Event 5307 (corn, insect control, deposited as ATCC PTA-9561, described in WO 2010/077816); Event ASR-368 (bent grass, herbicide tolerance, deposited as ATCC PTA-4816, described in US-A 2006-162007 or WO 2004/053062); Event B16 (corn, herbicide tolerance, not deposited, described in US-A 2003-126634); Event BPS-CV127-9 (soybean, herbicide tolerance, deposited as NCIMB No. 41603, described in WO 2010/080829); Event CE43-67B (cotton, insect control, deposited as DSM ACC2724, described in US-A 2009-217423 or WO2006/128573); Event CE44-69D (cotton, insect control, not deposited, described in US-A 2010-0024077); Event CE44-69D (cotton, insect control, not deposited, described in WO 2006/128571); Event CE46-02A (cotton, insect control, not deposited, described in WO 2006/128572); Event COT102 (cotton, insect control, not deposited, described in US-A 2006-130175 or WO 2004/039986); Event COT202 (cotton, insect control, not deposited, described in US-A 2007-067868 or WO 2005/054479); Event COT203 (cotton, insect control, not deposited, described in WO 2005/054480); Event DAS40278 (corn, herbicide tolerance, deposited as ATCC PTA-10244, described in WO 2011/022469); Event DAS-59122-7 (corn, insect control - herbicide tolerance, deposited as ATCC PTA 11384 , described in US-A 2006-070139); Event DAS-59132 (corn, insect control - herbicide tolerance, not deposited, described in WO 2009/100188); Event DAS68416 (soybean, herbicide tolerance, deposited as ATCC PTA-10442, described in WO 2011/066384 or WO 2011/066360); Event DP-098140-6 (corn, herbicide tolerance, deposited as ATCC PTA-8296, described in US-A 2009-137395 or WO 2008/112019); Event DP-305423-1 (soybean, quality trait, not deposited, described in US-A 2008-312082 or WO 2008/054747); Event DP-32138-1 (corn, hybridization system, deposited as ATCC PTA-9158, described in US-A 2009-0210970 or WO 2009/103049); Event DP-356043-5 (soybean, herbicide tolerance, deposited as ATCC PTA-8287, described in US-A 2010-0184079 or WO 2008/002872); Event EE-1 (brinjal, insect control, not deposited, described in WO 2007/091277); Event FI117 (corn, herbicide tolerance, deposited as ATCC 209031, described in US-A 2006-059581 or WO 98/044140); Event GA21 (corn, herbicide tolerance, deposited as ATCC 209033, described in US-A 2005-086719 or WO 98/044140); Event GG25 (corn, herbicide tolerance, deposited as ATCC 209032, described in US-A 2005-188434 or WO 98/044140); Event GHB119 (cotton, insect control - herbicide tolerance, deposited as ATCC PTA-8398, described in WO 2008/151780); Event GHB614 (cotton, herbicide tolerance, deposited as ATCC PTA-6878, described in US-A 2010-050282 or WO 2007/017186); Event GJ11 (corn, herbicide tolerance, deposited as ATCC 209030, described in US-A 2005-188434 or WO 98/044140); Event GM RZ13 (sugar beet, virus resistance , deposited as NCIMB-41601, described in WO 2010/076212); Event H7-1 (sugar beet, herbicide tolerance, deposited as NCIMB 41158 or NCIMB 41159, described in US-A 2004-172669 or WO 2004/074492); Event JOPLIN1 (wheat, disease tolerance, not deposited, described in US-A 2008-064032); Event LL27 (soybean, herbicide tolerance, deposited as NCIMB41658, described in WO 2006/108674 or US-A 2008-320616); Event LL55 (soybean, herbicide tolerance, deposited as NCIMB 41660, described in WO 2006/108675 or US-A 2008-196127); Event LLcotton25 (cotton, herbicide tolerance, deposited as ATCC PTA-3343, described in WO 03/013224 or US-A 2003-097687); Event LLRICE06 (rice, herbicide tolerance, deposited as ATCC-23352, described in US 6,468,747 or WO 00/026345); Event LLRICE601 (rice, herbicide tolerance, deposited as ATCC PTA-2600, described in US-A 2008-2289060 or WO 00/026356); Event LY038 (corn, quality trait, deposited as ATCC PTA-5623, described in US-A 2007-028322 or WO 2005/061720); Event MIR162 (corn, insect control, deposited as PTA-8166, described in US-A 2009-300784 or WO 2007/142840); Event MIR604 (corn, insect control, not deposited, described in US-A 2008-167456 or WO 2005/103301); Event MON15985 (cotton, insect control, deposited as ATCC PTA-2516, described in US-A 2004-250317 or WO 02/100163); Event MON810 (corn, insect control, not deposited, described in US-A 2002-102582); Event MON863 (corn, insect control, deposited as ATCC PTA-2605, described in WO 2004/011601 or US-A 2006-095986); Event MON87427 (corn, pollination control, deposited as ATCC PTA-7899, described in WO 2011/062904); Event MON87460 (corn, stress tolerance, deposited as ATCC PTA-8910, described in WO 2009/111263 or US-A 2011-0138504); Event MON87701 (soybean, insect control, deposited as ATCC PTA-8194, described in US-A 2009-130071 or WO 2009/064652); Event MON87705 (soybean, quality trait - herbicide tolerance, deposited as ATCC PTA-9241, described in US-A 2010-0080887 or WO 2010/037016); Event MON87708 (soybean, herbicide tolerance, deposited as ATCC PTA9670, described in WO 2011/034704); Event MON87754 (soybean, quality trait, deposited as ATCC PTA-9385, described in WO 2010/024976); Event MON87769 (soybean, quality trait, deposited as ATCC PTA-8911, described in US-A 2011-0067141 or WO 2009/102873); Event MON88017 (corn, insect control - herbicide tolerance, deposited as ATCC PTA-5582, described in US-A 2008-028482 or WO 2005/059103); Event MON88913 (cotton, herbicide tolerance, deposited as ATCC PTA-4854, described in WO 2004/072235 or US-A 2006-059590); Event MON89034 (corn, insect control, deposited as ATCC PTA-7455, described in WO 2007/140256 or US-A 2008-260932); Event MON89788 (soybean, herbicide tolerance, deposited as ATCC PTA-6708, described in US-A 2006-282915 or WO 2006/130436); Event MS11 (oilseed rape, pollination control - herbicide tolerance, deposited as ATCC PTA-850 or PTA-2485, described in WO 01/031042); Event MS8 (oilseed rape, pollination control - herbicide tolerance, deposited as ATCC PTA-730, described in WO 01/041558 or US-A 2003-188347); Event NK603 (corn, herbicide tolerance, deposited as ATCC PTA-2478, described in US-A 2007-292854); Event PE-7 (rice, insect control, not deposited, described in WO 2008/114282); Event RF3 (oilseed rape, pollination control - herbicide tolerance, deposited as ATCC PTA-730, described in WO 01/041558 or US-A 2003-188347); Event RT73 (oilseed rape, herbicide tolerance, not deposited, described in WO 02/036831 or US-A 2008-070260); Event T227-1 (sugar beet, herbicide tolerance, not deposited, described in WO 02/44407 or US-A 2009-265817); Event T25 (corn, herbicide tolerance, not deposited, described in US-A 2001-029014 or WO 01/051654); Event T304-40 (cotton, insect control - herbicide tolerance, deposited as ATCC PTA-8171, described in US-A 2010-077501 or WO 2008/122406); Event T342-142 (cotton, insect control, not deposited, described in WO 2006/128568); Event TC1507 (corn, insect control - herbicide tolerance, not deposited, described in US-A 2005-039226 or WO 2004/099447); Event VIP1034 (corn, insect control - herbicide tolerance, deposited as ATCC PTA-3925., described in WO 03/052073), Event 32316 (corn,insect control-herbicide tolerance,deposited as PTA-11507, described in WO 2011/084632), Event 4114 (corn,insect control-herbicide tolerance,deposited as PTA-11506, described in WO 2011/084621).

The composition according to the invention can also be used against fungal diseases liable to grow on or inside timber. The term "timber" means all types of species of wood and all types of working of this wood intended for construction, for example solid wood, high-density wood, laminated wood and plywood. The method for treating timber according to the invention mainly consists in contacting one or more compounds according to the invention or a composition according to the invention; this includes for example direct application, spraying, dipping, injection or any other suitable means.

Among the diseases of plants or crops that can be controlled by the methods according to the invention, mention can be made of:
Powdery mildew diseases such as :
   Blumeria diseases, caused for example by *Blumeria graminis*;
   Podosphaera diseases, caused for example by *Podosphaera leucotricha*;
   Sphaerotheca diseases, caused for example by *Sphaerotheca fuliginea*;
   Uncinula diseases, caused for example by *Uncinula necator*;
Rust diseases such as :
   Gymnosporangium diseases, caused for example by *Gymnosporangium sabinae*;
   Hemileia diseases, caused for example by *Hemileia vastatrix*;
   Phakopsora diseases, caused for example by *Phakopsora pachyrhizi* or *Phakopsora meibomiae*;
   Puccinia diseases, caused for example by *Puccinia recondite*, *Puccinia graminis or Puccinia striiformis*;
   Uromyces diseases, caused for example by *Uromyces appendiculatus*;
Oomycete diseases such as :
   Albugo diseases caused for example by *Albugo candida;*
   Bremia diseases, caused for example by *Bremia lactucae*;
   Peronospora diseases, caused for example by *Peronospora pisi* or *P. brassicae*;
   Phytophthora diseases, caused for example by *Phytophthora infestans*;
   Plasmopara diseases, caused for example by *Plasmopara viticola*;
   Pseudoperonospora diseases, caused for example by *Pseudoperonospora humuli* or
*Pseudoperonospora cubensis ;*
   Pythium diseases, caused for example by *Pythium ultimum*;
Leafspot, leaf blotch and leaf blight diseases such as :
   Alternaria diseases, caused for example by *Alternaria solani*;
   Cercospora diseases, caused for example by *Cercospora beticola*;
   Cladiosporum diseases, caused for example by *Cladiosporium cucumerinum*;
   Cochliobolus diseases, caused for example by *Cochliobolus sativus* (Conidiaform:
      Drechslera, Syn: Helminthosporium) or *Cochliobolus miyabeanus*;
      Colletotrichum diseases, caused for example by *Colletotrichum lindemuthanium*;
      Cycloconium diseases, caused for example by *Cycloconium oleaginum*;
      Diaporthe diseases, caused for example by *Diaporthe citri*;
      Elsinoe diseases, caused for example by *Elsinoe fawcettii*;
      Gloeosporium diseases, caused for example by *Gloeosporium laeticolor*;
      Glomerella diseases, caused for example by *Glomerella cingulata*;
      Guignardia diseases, caused for example by *Guignardia bidwelli*;
      Leptosphaeria diseases, caused for example by *Leptosphaeria maculans*; *Leptosphaeria nodorum*;
      Magnaporthe diseases, caused for example by *Magnaporthe grisea*;
      Mycosphaerella diseases, caused for example by *Mycosphaerella graminicola*; Mycosphaerella arachidicola ; *Mycosphaerella fijiensis*;
      Phaeosphaeria diseases, caused for example by *Phaeosphaeria nodorum*;
      Pyrenophora diseases, caused for example by *Pyrenophora teres*, or *Pyrenophora tritici repentis*;
      Ramularia diseases, caused for example by *Ramularia collo-cygni*, or *Ramularia areola*;
      Rhynchosporium diseases, caused for example by *Rhynchosporium secalis*;
      Septoria diseases, caused for example by *Septoria apiior Septoria lycopercisi*;
      Typhula diseases, caused for example by *Typhula incarnata*;
      Venturia diseases, caused for example by *Venturia inaequalis*;
Root, Sheath and stem diseases such as :
   Corticium diseases, caused for example by *Corticium graminearum*;
   Fusarium diseases, caused for example by *Fusarium oxysporum*;
   Gaeumannomyces diseases, caused for example by *Gaeumannomyces graminis*;
   Rhizoctonia diseases, caused for example by *Rhizoctonia solani*;
   Sarocladium diseases caused for example by *Sarocladium oryzae*;
   Sclerotium diseases caused for example by *Sclerotium oryzae*;
   Tapesia diseases, caused for example by *Tapesia acuformis*;
   Thielaviopsis diseases, caused for example by *Thielaviopsis basicola*;
Ear and panicle diseases such as :
   Alternaria diseases, caused for example by *Alternaria spp.*;
   Aspergillus diseases, caused for example by *Aspergillus flavus*;
   Cladosporium diseases, caused for example by *Cladosporium spp.*;
   Claviceps diseases, caused for example by *Claviceps purpurea*;
   Fusarium diseases, caused for example by *Fusarium culmorum*;
   Gibberella diseases, caused for example by *Gibberella zeae*;
   Monographella diseases, caused for example by *Monographella nivalis*;
Smut and bunt diseases such as :
   Sphacelotheca diseases, caused for example by *Sphacelotheca reiliana*;
   Tilletia diseases, caused for example by *Tilletia caries*;
   Urocystis diseases, caused for example by *Urocystis occulta*;
   Ustilago diseases, caused for example by *Ustilago nuda*;
Fruit rot and mould diseases such as :
   Aspergillus diseases, caused for example by *Aspergillus flavus*;
   Botrytis diseases, caused for example by *Botrytis cinerea*;
   Penicillium diseases, caused for example by *Penicillium expansum*;
   Rhizopus diseases caused by example by *Rhizopus stolonifer*
   Sclerotinia diseases, caused for example by *Sclerotinia sclerotiorum*;
   Verticilium diseases, caused for example by *Verticilium alboatrum*;
Seed and soilborne decay, mould, wilt, rot and damping-off diseases :
   Alternaria diseases, caused for example by *Alternaria brassicicola*
   Aphanomyces diseases, caused for example by *Aphanomyces euteiches*
   Ascochyta diseases, caused for example by *Ascochyta lentis*
   Aspergillus diseases, caused for example by *Aspergillus flavus*
   Cladosporium diseases, caused for example by *Cladosporium herbarum*
   Cochliobolus diseases, caused for example by *Cochliobolus sativus*
   (Conidiaform: *Drechslera*, *Bipolaris Syn: Helminthosporium*);
   Colletotrichum diseases, caused for example by *Colletotrichum coccodes*;
   Fusarium diseases, caused for example by *Fusarium culmorum*;
   Gibberella diseases, caused for example by *Gibberella zeae*;
   Macrophomina diseases, caused for example by *Macrophomina* phaseolina
   Monographella diseases, caused for example by *Monographella nivalis*;
   Penicillium diseases, caused for example by *Penicillium expansum*
   Phoma diseases, caused for example by *Phoma lingam*
   Phomopsis diseases, caused for example by *Phomopsis sojae*;
   Phytophthora diseases, caused for example by Phytophthora cactorum;
   Pyrenophora diseases, caused for example by *Pyrenophora graminea*
   Pyricularia diseases, caused for example by *Pyricularia oryzae*;
   Pythium diseases, caused for example by *Pythium ultimum*;
   Rhizoctonia diseases, caused for example by *Rhizoctonia solani*;
   Rhizopus diseases, caused for example by *Rhizopus oryzae*
   Sclerotium diseases, caused for example by *Sclerotium rolfsii*;
   Septoria diseases, caused for example by *Septoria nodorum*;
   Typhula diseases, caused for example by *Typhula incarnata*;
   Verticillium diseases, caused for example by *Verticillium dahliae*;
Canker, broom and dieback diseases such as :
   Nectria diseases, caused for example by *Nectria galligena*;
Blight diseases such as :
   Monilinia diseases, caused for example by *Monilinia laxa*;
Leaf blister or leaf curl diseases such as :
   Exobasidium diseases caused for example by *Exobasidium vexans*
   Taphrina diseases, caused for example by *Taphrina deformans*;
Decline diseases of wooden plants such as :
   Esca diseases, caused for example by *Phaemoniella clamydospora*;
   Eutypa dyeback, caused for example by *Eutypa lata*;
   Ganoderma diseases caused for example by *Ganoderma boninense*;
   Rigidoporus diseases caused for example by *Rigidoporus lignosus*
Diseases of Flowers and Seeds such as
   Botrytis diseases caused for example by *Botrytis cinerea*;
Diseases of Tubers such as
   Rhizoctonia diseases caused for example by *Rhizoctonia solani*;
   Helminthosporium diseases caused for example by *Helminthosporium solani*;
Club root diseases such as
   Plasmodiophora diseases, cause for example by *Plamodiophora brassicae.*
Diseases caused by Bacterial Organisms such as
   Xanthomonas species for example *Xanthomonas campestris* pv. oryzae;
   Pseudomonas species for example *Pseudomonas syringae* pv. lachrymans;
   Erwinia species for example *Erwinia amylovora.*

### Legend of the figures

**Fig 1** **:** Measurement of *Phytophthora infestans* growth inhibition in the presence of dsRNA targeting the saccharopine dehydrogenase messenger RNA.
**Fig 2** **:** Analysis of saccharopine dehydrogenase mRNA level by qRT PCR.

### Sequence listing:

**SEQ ID N°1:** Saccharopine dehydrogenase (LYS1) from Aspergillus clavatus.
**SEQ ID N°2:** Protein encoded by the above nucleic acid sequence.
**SEQ ID N°3:** Saccharopine dehydrogenase (LYS1) from Aspergillus fumigatus.
**SEQ ID N°4:** Protein encoded by the above nucleic acid sequence.
**SEQ ID N°5:** Saccharopine dehydrogenase (LYS1) from Botrytis cinerea.
**SEQ ID N°6:** Protein encoded by the above nucleic acid sequence.
**SEQ ID N°7:** Saccharopine dehydrogenase (LYS1) from Fusarium graminearum.
**SEQ ID N°8:** Protein encoded by the above nucleic acid sequence.
**SEQ ID N°9:** Saccharopine dehydrogenase (LYS1) from Fusarium oxysporum.
**SEQ ID N°10:** Protein encoded by the above nucleic acid sequence.
**SEQ ID N°11:** Saccharopine dehydrogenase (LYS1) from Fusarium verticilloides.
**SEQ ID N°12:** Protein encoded by the above nucleic acid sequence.
**SEQ ID N°13:** Saccharopine dehydrogenase (LYS1) from Fusarium verticilloides.
**SEQ ID N°14:** Protein encoded by the above nucleic acid sequence.
**SEQ ID N°15:** Saccharopine dehydrogenase (LYS1) from Mycosphaerella fijiensis.
**SEQ ID N°16:** Polypeptide encoded by the above nucleic acid sequence.
**SEQ ID N°17:** Saccharopine dehydrogenase (LYS1) from Magnaporthe grisea.
**SEQ ID N°18:** Protein encoded by the above nucleic acid sequence.
**SEQ ID N°19:** Saccharopine dehydrogenase (LYS1) from Monoliophthora perniciosa.
**SEQ ID N°20:** Protein encoded by the above nucleic acid sequence.
**SEQ ID N21:** Saccharopine dehydrogenase (LYS1) from Puccinia graminis.
**SEQ ID N22:** Protein encoded by the above nucleic acid sequence.
**SEQ ID N23:** Saccharopine dehydrogenase (LYS1) from Phytophthora infestans.
**SEQ ID N24:** Protein encoded by the above nucleic acid sequence.
**SEQ ID N25:** Saccharopine dehydrogenase (LYS1) (from Phytophthora ramorum.
**SEQ ID N26:** Protein encoded by the above nucleic acid sequence.
**SEQ ID N27:** Saccharopine dehydrogenase (LYS1) from Phytophthora sojae.
**SEQ ID N°28:** Protein encoded by the above nucleic acid sequence.
**SEQ ID N29:** Saccharopine dehydrogenase (LYS1) from Pyrenophora tritici-repentis.
**SEQ ID N°30:** Protein encoded by the above nucleic acid sequence.
**SEQ ID N31:** Saccharopine dehydrogenase (LYS1) from Sclerotinia sclerotiorum.
**SEQ ID N32:** Protein encoded by the above nucleic acid sequence.
**SEQ ID N33:** Saccharopine dehydrogenase (LYS1) from Trichoderma reesei.
**SEQ ID N 34:** Protein encoded by the above nucleic acid sequence.
**SEQ ID N35:** Saccharopine dehydrogenase (LYS1) from Ustilago maydis.
**SEQ ID N36:** Protein encoded by the above nucleic acid sequence.
**SEQ ID N37:** Saccharopine dehydrogenase (LYS1) from Verticillium albo-atrum.
**SEQ ID N°38:** Protein encoded by the above nucleic acid sequence.
**SEQ ID N39:** Saccharopine dehydrogenase (LYS1) from Mycosphaerella graminicola.
**SEQ ID N°40:** Protein encoded by the above nucleic acid sequence.
**SEQ ID N°41:** Saccharopine dehydrogenase (LYS1) from Fusarium moniliform.
**SEQ ID N°42:** Protein encoded by the above nucleic acid sequence.
**SEQ ID N°43:** Saccharopine dehydrogenase (LYS1) from Claviceps purpurea.
**SEQ ID N°44:** Protein encoded by the above nucleic acid sequence.
**SEQ ID N°45:** Primer SACdh_Pi_T7_F
**SEQ ID N°46:** Primer SACdh_Pi_T7_R
**SEQ ID N°47:** Primer Actin forward
**SEQ ID N°48:** Primer Actin reverse
**SEQ ID N°49:** Primer β-Tub forward
**SEQ ID N°50:** Primer β-Tub reverse
**SEQ ID N°51:** Primer SACdh forward
**SEQ ID N°52:** Primer SACdh reverse
**SEQ ID N°53:** Primer pBINB33-1
**SEQ ID N°54:** Primer pBINB33-2
**SEQ ID N°55:** Primer SacdhPI R
**SEQ ID N°56:** Primer SacdhPI F
**SEQ ID N°57:** Primer LYS1 Pot 117-F
**SEQ ID N°58:** Primer LYS1 Pot 117-R

The various aspects of the invention will be understood more fully by means of the experimental examples below.

All the methods or operations described below are given by way of example and correspond to a choice, made among the various methods available for achieving the same result. This choice has no effect on the quality of the result, and, consequently, any appropriate method can be used by those skilled in the art to achieve the same result. In particular, and unless otherwise specified in the examples, all the recombinant DNA techniques employed are carried out according to the standard protocols described in Sambrook and Russel (2001, Molecular cloning: A laboratory manual, Third edition, Cold Spring Harbor Laboratory Press, NY) in Ausubel et al. (1994, Current Protocols in Molecular Biology, Current protocols, USA, Volumes 1 and 2), and in Brown (1998, Molecular Biology LabFax, Second edition, Academic Press, UK). Standard materials and methods for plant molecular biology are described in Croy R.D.D. (1993, Plant Molecular Biology LabFax, BIOS Scientific Publications Ltd (UK) and Blackwell Scientific Publications (UK)). Standard materials and methods for PCR (Polymerase Chain Reaction) are also described in Dieffenbach and Dveksler (1995, PCR Primer: A laboratory manual, Cold Spring Harbor Laboratory Press, NY) and in McPherson et al. (2000, PCR - Basics: From background to bench, First edition, Springer Verlag, Germany).

### Ref biblio:

- Bevan, 1984, Nucl Acids Res 12: 8711-8721
- Bhattacharjee, J. K., 1985, Crit. Rev.Microbiol. 12, 131-151
- Bhattacharjee, J. K., 1992, in Handbook of Evolution of Metabolic Function, Mortlock, R. P., ed., CRC Press, Boca Raton, FL, pp. 47-80
- Born T.L. and Blanchard J.S., 1999, Curr. Opin. Chem. Biol., 3:607-613
- Broquist, H. P., 1971, Methods Enzymol. 17, 112-129
- Chuang and Meyerowitz, 2000, PNAS, 97: 4985-4990
- Ehmann et al., 1999, biochemistry, 38,6171-6177
- Escobar et al., 2001, Proc. Natl. Acad. Sci. USA., 98(23): 13437-13442
- Fire et al. 1998, Nature 391: 806-811
- Garrad, R. C. and Bhattacharjee, J. K., 1992, J. Bacteriol. 174, 7379-7384
- Gielen et al, 1984, EMBO Journal 3, 835-846
- Hamilton and Baulcome, 1999, Science 286: 950-952
- Hamilton et al., 1998, Plant J, 15: 737-746
- Hammond et al., 2000, Nature 404: 293-296
- Hannon, 2002, Nature, 418 (6894): 244-51
- Herrera-Estrella et al, 1983 Nature, 303, 209-213
- Hofgen and Willmitzer, 1990, Plant Science 66, 221-230
- Houmard et al., 2007, Plant Biotechnology Journal 5, 605-614
- Montgomery et al., 1998, PNAS 95: 15502-15507
- Pandolfini et al., 2003, Biotechnol., 25; 3(1): 7
- Pietrzak et al, 1986 Nucleic Acids Res. 14, 5858
- Thompson et al., Nucleic Acids Research 22 (1994), 4673-4680
- Umbargar, H. E., 1978, Annu. Rev. Biochem. 47, 533-606
- Randall, T.A., 2005, MPMI, 18, 229-243
- Rocha-Sosa et al., 1989, EMBO J. 8, 23-29
- Ruiz Ferrer and Voinnet, Annu. Rev. Plant Biol. 2009. 60:485-510
- Sambrook et al., 2001, Molecular Cloning: A Laboratory Manual, third edition, ISBN978-087969577-4
- Silué et al. Physiol. Mol. Plant Pathol., 53 239-251, 1998
- Stärkel C., Ph. D. thesis "Host induced gene silencing - strategies for the improvement of resistance against Cercospora beticola in sugar beet (B. vulgaris L.) and against Fusarium graminearum in wheat (T. aestivum L.) and maize (Z. mays L.)", defended in June 2011, XP002668931, retrieved from internet (2012-02-07) on http://ediss.sub.uni-hamburg.de/vollteste/2011/5286/pdf/Dissertation.pdf
- Tang et al., 2003 Gene Dev., 17(1): 49-63
- Villalba et al. Fungal Genetics and Biology 45, 68-75, 2008
- Vogel, H. J., 1965, in Handbook of Evolving Genes and Proteins, Bryson, V., ed., Academic Press, New York, pp. 25-40
- Waterhouse et al., 1998, PNAS 95: 13959-13964,
- Wesley et al. The Plant Journal (2001); 27(6), 581-590
- Xiao et al., 2003, Plant Mol Biol., 52(5): 957-66
- Xu et al., 2006, Cell Bio chemistry and Biophysics, vol 46, 43-64
- Zamore et al., 2000, Cell, 101: 25-33,

### Examples

### Example 1: In vitro cultivation of Magnaporthe Grisea

Assays were carried out using the *Magnaporthe grisea* wild-type strain P1.2 originated from the collection of the phytopathology laboratory of the CIRAD (Centre de coopération internationale en recherche agronomique pour le developpement) in Montpellier. Conditions for culturing, the composition of the rice-agar medium, maintenance, and sporulation as well as protoplasts preparations are described by Silué et al. (1998).

### Example 2 : Magnaporthe grisea transfection with dsRNA targeting saccharopine dehydrogenase and measurement of growth inhibition

The Magnaporthe grisea saccharopine dehydrogenase (SACdh) gene sequence Lys-1 (MGG_01359.6: 1426 bp) was obtained from the Broad Institute (http://www.broadinstitute.org/). A region of about 325 bp was selected for dsRNA, comprizing the nucleotides 301 through 626, was synthesized by the Geneart company and cloned into the plasmid.

For transfection, ds RNA of saccharopine dehydrogenase from *Magnaporthe grisea* was produced using the MEGAscript RNAi Kit (Ambion) according to the manufacturers' instructions. Different amounts (200µg to 2µg of ds RNA) were treated with transfection agent Lipofectamin RNAi max (Invitrogen) following manufactures' instructions. Lipofectamin-ds RNA complexes were added to 2,5 x 10⁶ *Magnaporthe grisea* protoplast in a microtiterplate with TB3 media (Villalba et al., 2008), and growth was monitored for 5-7 days at a OD of 600 nm using a Infinite M1000 (Tecan) microplate reader..

Growth of *Magnaporthe grisea* protoplasts treated with ds RNA of saccharopine dehydrogenase comparing to untreated control.was monitored at several time points and showed a significant difference in growth.

### Example 3 : In vitro cultivation Phytophthora infestans and zoospores preparation.

*Phytophthora infestans* strain PT78 was cultivated *in vitro* in 9cm petri dishes on pea agar medium (125g/l boiled and crushed peas, 20 g/l agar agar, carbenicillin 100mg/l) at 21°C in the dark. Every 15 days, new medium was inoculated with four 5mm cubic plugs of mycelium.

To release the zoospores, 12 ml of ice cold water were put on a 10 days old culture and the culture was placed at 4°C for 2 hours. The supernatant was then collected without dwasturbing the mycelium and filtered through a 100 µm sieve to remove hyphal fragments. The zoospores were placed on ice and counted with a haemocytometer.

### Example 4: Phytophthora infestans transfection with dsRNA targeting saccharopine dehydrogenase and measurement of growth inhibition

The *Phytophthora infestans* saccharopine dehydrogenase gene sequence Lys-1 (PITG_03530 : 3020 bp) was obtained from the *P. infestans* database of the Broad Institute. A region about 500 bp offering the best siRNA according to the BLOCKit RNAi designer software (Invitrogen) and comprizing the nucleotides 2251 through 2750, was synthesized by the Geneart company and cloned into the plasmid 0920357_SacDH_Pi_pMA..

Synthesis of dsRNA was carried out using the Megascript RNAi kit (Ambion) following the manufacturer's protocol and using as a template a PCR product amplified from the plasmid 0920357_SacDH_Pi_pMA. The forward primer used was SACdh_Pi_T7_F: 5' TAATACGACTCACTATAGGGTTGCAGGAGAGCGCAGAAAGC and the reverse primer was SACdh_Pi_T7_R: TAATACGACTCACTATAGGGTCAGTTGGAGTCCGCGTGGTGT.

dsRNA were then precipitated with 100% ethanol and sodium acetate 3M, pH5.2, washed 2 times with 70 % ethanol and the pellets were resuspended in RNase free water.

The transfection mixes were prepared in a 48 well plate by adding sequentially V8 medium (5% of V8 juice (Campbell Foods Belgium), pH5), the appropriate amount of dsRNA and 10 µl of lipofectamine RNAi max (Invitrogen) in a final volume of 200 µl. The transfection mixture was incubated during 15 min at room temperature..

Zoospores were diluted in V8 medium to a concentration of 5x10⁴ zoospores/ml. Then, 800 µL of the zoospores solution were added in each well of the plate. The final concentration of zoospores was 4x10⁵ zoospores / ml.

Three controls were added on each plate: V8 medium, V8 medium + zoospores, V8 medium + zoospores + lipofectamine. The plates were incubated at 21°C, in the dark.

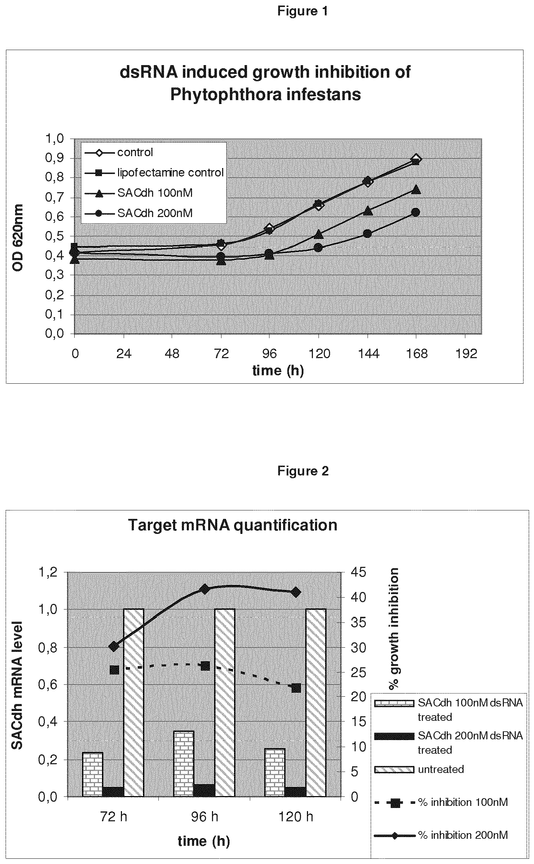

The growth of the fungus was followed by measuring the absorbance at 620 nm in a plate reader (Infinite 1000, Tecan) over 8 days The percentage of growth inhibition was calculated using the following formula: 100-(OD_{dsRNA} x 100 / OD_{control lipofectamine}). The growth of the fungi was reduced in the presence of dsRNA directed against saccharopine dehydrogenase in a concentration dependant manner (100nM and 200nM respectively) as shown in Figure 1

### Example 5 : Quantitative PCR analysis of P. infestans saccharopine dehydrogenase messenger RNA:

To yield sufficient RNA for cDNA synthesis and real-time RT-PCR, several wells of the 48 wells plate were pooled for one concentration of dsRNA tested : 10 wells for 72h time point, 6 wells for 96h time point, 3 wells for 120h time point.

After 72h, 96h and 120h of treatment with the dsRNA, the mycelia were collected. The samples were centrifuged to remove the medium. The samples were frozen in liquid nitrogen and then lyophilized overnight.

Before RNA extraction, the mycelium was grinded. Total RNA was extracted using the RNeasy Plant mini kit (Qiagen) following the manufacturer's protocol. DNA contamination of the RNA samples was removed by DNase digestion (DNA free, Ambion). Integrity of the RNA was tested on the 2100 Bioanalyzer (RNA 6000 nano kit, Agilent) following the protocol supplied by the manufacturer. The cDNA were synthesized from 2 µg of total RNA by oligo dT priming using the kit Thermoscript RT-PCR system (Invitrogen) following the manufacturer's protocol. The cDNA were precipitated with 100 %EtOH and sodium acetate 3M, pH5.2, washed 2 times with 70 % EtOH and the pellets were resuspended in 10 µL of RNase free water. The cDNA were diluted a hundred fold for the qPCR test. Primer pairs were designed for each gene sequence by using the Primer Express 3 software (Applied Biosystems). Real time RT-PCR was performed on a 7900 Real Time PCR system (Applied Biosystems) with Power SYBR green PCR master mix (Applied Biosystems) following the manufacturer's protocol. Q-PCR was performed as follows : 95°C for 10 min, 45 cycles at 95°C for 15s and 60°C 1 min, followed by a dissociation stage at 95°C for 15s, 60°C for 1 min and 95°C for 15s.

The actin and β-tubuline genes were used as endogenous controls. The relative expression of genes was calculated with the 2ΔΔCt method. The figure 2 shows a significant reduction of the level of the saccharopine dehydrogenase messenger RNA and this reduction correlates with growth inhibition.

**Table 1: Sequence of qPCR primers :**

| Primer name | Forward primer sequence | Reverse primer sequence |
|---|---|---|
| actin | CGACTCTGGTGACGGTGTGT | GCGTGAGGAAGAGCGTAACC |
| β-Tub | CCGCCCAGACAATTTCGT | CCTTGGCCCAGTTGTTACCA |
| SACdh | TGGGTGGTTTCCAAGGTCTTC | AAAGGCACCAAGCCACTGAA |

### Example 6 : Construction of transformation vectors containing the Phytophthora infestans Saccharopine Dehydrogenase gene.

### a) Preparation of the plant expression vector IR 47-71

The plasmid pBinAR is a derivative of the binary vector plasmid pBin19 (Bevan, 1984) which was constructed as follows: A fragment of a length of 529 bp which comprised the nucleotides 6909-7437 of the 35S promoter of the cauliflower mosaic virus was isolated as EcoR \IKpn I fragment from the plasmid pDH51 (Pietrzak et al, 1986) and ligated between the EcoR I and Kpn I restriction sites of the polylinker of pUC18. In this manner, the plasmid pUC18-35S was formed. Using the restriction endonucleases Hind III and Pvu II, a fragment of a length of 192 bp which included the polyadenylation signal (3' terminus) of the Octopin Synthase gene (gene 3) of the T-DNA of the Ti plasmid pTiACHδ (Gielen et al, 1984) (nucleotides 11 749-11 939) was isolated from the plasmid pAGV40 (Herrera-Estrella et al, 1983). Following addition of Sph I linkers to the Pvu II restriction site, the fragment was ligated between the Sph I and Hind III restriction sites of pUC18-35S. This gave the plasmid pA7. Here, the entire polylinker comprising the 35S promoter and Ocs terminator was removed using EcoR I and Hind III and ligated into the appropriately cleaved vector pBin19. This gave the plant expression vector pBinAR (Hofgen and Willmitzer, 1990).

The promoter of the patatin gene B33 from Solanum tuberosum (Rocha-Sosa et al., 1989) was, as Dra I fragment (nucleotides -1512 - +14), ligated into the Ssf I-cleaved vector pUC19 whose ends had been blunted using T4-DNA polymerase. This gave the plasmid pUC19-B33. From this plasmid, the B33 promoter was removed using EcoR I and Sma I and ligated into the appropriately restricted vector pBinAR. This gave the plant expression vector pBinB33. To facilitate further cloning steps, the MCS (Multiple Cloning Site) was extended. To this end, two complementary oligonucleotides were synthesized, heated at 95°C for 5 minutes, slowly cooled to room temperature to allow good fixation (annealing) and cloned into the Sal I and Kpn I restriction sites of pBinB33. The oligonucleotides used for this purpose had the following sequence:
pBINB33-1: 5'-TCG ACA GGC CTG GAT CCT TAA TTA AAC TAG TCT CGA GGA GCT CGG TAC-3 pBINB33-2: 5'-CGA GCT CCT CGA GAC TAG TTT AAT TAA GGA TCC AGG CCT G-3'

The plasmid obtained was named IR 47-71.

### b) Preparation of the plant expression vectors pEPA248 and pEPA262 comprising a nucleic acid sequences for the Phytophthora infestans Saccharopine Dehydrogenase gene.

The saccharopine dehydrogenase sequence (PITG_03530: 3020 bp) was obtained from the P. infestans ORF Prot V1 database. A region about 500 bp offering the best siRNA according to the BLOCKit RNAi designer software (Invitrogen), was synthesized by the Geneart company.
A 300 bp fragment was amplified by PCR from this sequence DNA with the primers SacdhPI R (5'-agaggtaccaagcttgcgtagctgg-3') and SacdhPI F (5'-tatctcgagtctagacaacgccattggttac-3'). The amplified fragment was cloned into pCRII-Topo (Invitrogen) to obtain the plasmid pEPA250. According to Wesley *et al.* (2001), the sequence of interest was cloned in pHannibal vector to give plasmid pEPA241. Then the dsRNA expression cassette was sub-cloned into different binary (plant expression) vectors pART27 (Gleave AP, PMB 20, (1992), 1203-1207) and IR 47 to produce the plant expression vectors pEPA248 and pEPA262, respectively.
Vector pEPA248 and pEPA262 were introduced into respectively GV3101 and C58C1RIF (pGV2260) agrobacteria cells by electroporation (Rocha-Sosa et al.(1989)), in order to further transform potato plants.

### Example 7: Construction of transformation vectors targeting the Sclerotinia sclerotiorum Saccharopine Dehydrogenase gene lys1.

The 351 bp of a region of the *S*. *sclerotiorum Lys1* coding sequence (saccharopine dehydrogenase SS1G_06166.1) was synthesized by the Geneart company (pEPA293), and flanked by internal (*Xba*I, *Hind*III) and external (*Xho*I*, Kpn*I) restriction sites designed to perform a two-step cloning into the pHannibal vector (Wesley et al., 2001). The intermediate plasmid harbored two inverted copies of the *Lys1* gene fragment spaced by the pHannibal *PdK* intron and regulated by the cauliflower mosaic virus (CaMV) 35S promoter and the OCS terminator.

The entire DNA cassette was then excised with *Not*I and inserted into the pART27 binary vector (Gleave, 1992), giving the final plasmid pEPA307 with a plant selection cassette based on kanamycin resistance (nptII gene regulated by the *Nos* promoter and terminator).

The same *Not*I cassette was also inserted in a binary vector (pFCO31) with a plant selection marker based on an HPPD inhibitors resistance, to be used in Soybean transformation. The final plasmid can then transform plants with a T-DNA comprising in between the Right and Left borders, our cassette of interest and an HPPD gene regulated by a CsVMV promoter, a chloroplast transit peptide sequence and a 3'Nos terminator.

### Example 8 : Construction of transformation vectors targeting the Phakopsora pachirizi Saccharopine Dehydrogenase gene lys1.

The 364bp of a region of a *Phakopsora pachirizi Lys1* E.S.T. (saccharopine dehydrogenase PHAPC_EH247326.1) was synthesized by the Geneart company (pCED42), and flanked by internal (*Xba*I, *Hind*III) and external (*Xho*I, *Kpn*I), restriction sites designed to perform a two-step cloning into the pHannibal vector (Wesley et al., 2001). The intermediate plasmid harbored two inverted copies of the *Lys1* gene fragment spaced by the pHannibal *PdK intron* and regulated by the cauliflower mosaic virus (CaMV) 35S promoter and the OCS terminator.

The entire DNA cassette was then excised with *Not*I and inserted into the pART27 binary vector (Gleave, 1992), giving the final plasmid pCED45 with a plant selection cassette based on kanamycin resistance (nptII gene regulated by by the *Nos* promoter and terminator).

The same *Not*I cassette was also inserted in a binary vector (pFCO31) with a plant selection marker based on an HPPD inhibitors resistance, to be used in Soybean transformation. The final plasmid (pCED87) can then transform plants with a T-DNA comprising inbetween the Right and Left borders, our cassette of interest and an HPPD gene regulated by a CsVMV promoter, a chloroplast transit peptide sequence and a 3'Nos terminator.

### Example 9: Transformation of potato plants with plant expression vectors comprising nucleic acid molecules coding for hairpin saccharopine dehydrogenase construct pEPA262

Potato plants were transformed via Agrobacterium using the plant expression vector pEPA262, which comprises a coding nucleic acid sequence for saccharopine dehydrogenase under the control of the promoter of the patatin gene B33 from Solanum tuberosum as described by Rocha-Sosa et al. (1989). The transgenic potato plants transformed with the plasmid pEPA262, were named "537 ES ". Molecular analysis of the events of "537 ES" was performed using standart PCR methods (Sambrook et al.) to detect the presence of the nucleic acid sequence for saccharopine dehydrogenase using the following primers SacDH PI F: 5'-TATCTCGAGTCTAGACAACGCCATTGGTTAC-3' and SacDH PI R: 5'-AGAGGTACCAAGCTTGCGTAGCTGG-3'. Further selection was accomplished either by Northen blotting or by expression analysis of the the nucleic acid sequence for saccharopine dehydrogenase via RT-Q PCR leading to a selection of different events. The oligonucleotides used for this purpose had the following sequence: LYS1_Pot 117-F: 5'-TCA ATA GAA GCG AAC GCG TAA A-3' and LYS1_Pot 117-R: 5'-GTT CGG GAT CTG CTC GAT GT-3'

### Example 10: Agrobacterium-mediated transformation of Arabidopsis thaliana.

The pART27 derived plasmids was introduced into *Agrobacterium tumefaciens* strain LBA4404 (Invitrogen Electromax) by electroporation. The obtained bacterial strains were then used for the floral dip infiltration of the A. *thaliana* Col-0 or Wassileskija plants as described by Clough & Bent (PlantJ 1998).

### Example 11: Agrobacterium-mediated transformation of Glycine max.

The pFCO31 derived plasmids were introduced into *Agrobacterium tumefaciens* strain LBA4404 (Invitrogen Electromax) by electroporation. The obtained bacterial strains were then used for Soybean transformation as described below.

Soybean seeds are sterilized for 24h with Chlorine gas (Cl2). Seeds are then placed in Petri dishes and soaked in sterile deionized water for 20 hours prior to inoculation, in the dark, at room temperature. An overnight culture grown at 28 °C a nd 200rpm agitation of *Agrobacterium tumefaciens* in 200ml of YEP (5 g/L Yeast extract, 10 g/L Peptone, 5 g/L NaCl2. pH to 7.0) containing the appropriate antibiotic is centrifugated at 4000rpm, 4 °C, 15min. The pellet is resuspended in 40 to 50mL of infection medium to a final OD600nm between 0.6 and 1 and stored on ice. Soaked seeds are dissected, under sterile conditions, using a #15 scalpel blade to separate the cotyledons and remove the primary leaves attached to them. Each cotyledon is kept as explant for inoculation. About 100 explants are prepared and subsequently inoculated together, for 30 minutes in the *Agrobacterium* inoculum, with occasional agitation. Cocultivation is performed in classical Petri dishes containing 4 papers filter (Whatman ® grade 1) and 4 mL of Cocultivation medium (1/10X B5 major salts, 1/10X B5 minor salts, 2.8 mg/L Ferrous, 3.8 mg/L NaEDTA, 30 g/L Sucrose, 3.9 g/L MES (pH 5.4). Filter sterilized 1X B5 vitamins, GA3 (0.25 mg/L), BAP (1.67 mg/L), Cysteine (400 mg/L), Dithiothrietol (154.2 mg/L), and 200µM acetosyringone). Explants are placed on co-cultivation plates (9 per plate), adaxial (flat) side down and sealed with a single vertical string of tape (Leucopore ®) and further incubated for 5 days, at 24°C, in a 18:6 photoperio d.At the end of cocultivation, the explants are placed (6 per plate) on the Shoot Initiation Medium (1X B5 major salts, 1X B5 minor salts, 28 mg/L Ferrous, 38 mg/L NaEDTA, 30 g/L Sucrose, 0.56 g/L MES, and 8 g/L agar (pH 5.6). Filter sterilized 1X B5 vitamins, BAP (1.67 mg/L), Timentin (50 mg/L), Cefotaxime (50 mg/L), Vancomycin (50 mg/L) and Tembotrione (0.1 mg/L)), inclined at 45 °, with the cotyledonary node area imbedded in the medium and upwards. The Shoot Initiation step lasts1 month (24°C 16/8 photoperiod). After one more month, explants with green shoots are transferred on Shoot Elongation Medium (1X MS/B5 medium amended with 1 mg/l zeatin riboside (ZR), 0.1 mg/l IAA, 0.5 mg/l GA3, 3% sucrose, 100 mg/l pyroglutamic acid, 50 mg/l asparagine, 0.56 g/L MES, pH 5.6, solidified with 0.8% agar, ticarcillin (50 mg/l), cefotaxime (50 mg/l) and vancomycin (50 mg/L)), with fresh transfer every 2 weeks. Plantlets that are more than 2cm high are transferred on Rooting medium. Plantlets are cut and placed on rooting medium (1/2 MS major salts, minor salts and vitamins B5, 15 g/L Sucrose, 1 mg/L IBA 8 g/L Noble agar, pH 5.7). in an 180mL vertical plastic container.

Once the roots are well formed and the apex is strong, plants are placed into soil in the greenhouse and covered with a green plastic box for acclimatization for 5 days on a 36°C heating bed. After 10 days of acclimatization, the plants are transferred into big pots, without heating bed.

### Example 12: Asian Soybean Rust (Phakopsora pachyrhizi) assay.

Soybean plants expressing dsRNA directed against *Phakopsora pachirizi Lys1* were grown in the greenhouse in 7,5 cm pots (28,5 °C, 50% humidity, 1 4h light). In an incubator, plants were sprayed with a conidia suspension (50ml at 10-15X10⁴ spores/ml obtained from artificially infected soybean plants serving as a source of inoculum, for one tray of dimensions 55X34X5cm containing 15 pots). Suspension includes Tween20 at 0.033%. To ensure even inoculation multidirectional spraying is necessary. Plants are then incubated for 4 days at ca. 25 °C (daytime) and ca. 20 °C (night) with very high humidity (90% to saturation). After this period plants are transferred back to normal growing conditions. Asian soybean rust development is evaluated at regular intervals to follow kinetics of disease development and severity of symptoms. All experiments with Asian soybean rust are performed in L2 safety level culture chambers or incubators according to HCB requirements.

### Example 13: Sclerotinia sclerotiorum assay

Development of the Wild-type S. sclerotiorum isolate 1980 as well as the pac 1 mutant (Rollins, 2003) fungus was studied on a whole plant assay. S. sclerotiorum was stored at 4 °C on potato dextrose agar (PDA, potato 200 g/l, glucose 20 g/l, agar 18 g/l). The fungus was cultured in a Petri dish containing PDA by placing a mycelial plug in the centre and was maintained under static conditions at 21°C for 4 days. 4 weeks old Arabidopsis wild-type and transgenic plants were inoculated with 12-mm diameter agar-mycelium plugs excised from the actively growing margin of the fungal colony in the centre of the plant. Inoculated plants were kept in a growth chamber at 21°C with 100% relative humidity under a 12-h light photoperiod with a light intensity of 34 mmol m-2 s-1 using fluorescent white lights and were monitored every 12 h to observe fungal development. Disease symptoms were monitored by number of infected leaves as well as lengths and widths of lesions.

## Claims

1. A dsRNA molecule comprising i) a first strand comprising a sequence identical to at least 18 contiguous nucleotides of a fungus or oomycete saccharopine dehydrogenase gene and ii) a second strand comprising a sequence substantially complementary to the first strand, wherein the fungus or oomycete gene is selected from the group consisting of:
a) a polynucleotide comprising a sequence as set forth in SEQ ID N0: 1, 3, 5, 7, 9, 11, 13, 15, 17, 19, 21, 23, 25, 27, 29, 31, 33, 35, 37, 39, 41, 43;
b) a polynucleotide encoding a polypeptide having a sequence as set forth in SEQ ID N0: 2, 4, 6, 8, 10, 12, 14, 16, 18, 20, 22, 24, 25, 28, 30, 32, 34, 36, 38, 40, 42, 44;
c) a polynucleotide having at least 70% sequence identity, preferably at least 80%, more preferably at least 90%, even more preferably at least 95% to a polynucleotide having a sequence as set forth in SEQ ID N0: 1, 3, 5, 7, 9, 11, 13, 15, 17, 19, 21, 23, 25, 27, 29, 31, 33, 35, 37, 39, 41, 43;
d) a polynucleotide encoding a polypeptide having at least 70% sequence identity, preferably at least 80%, more preferably at least 90%, even more preferably at least 95%, to a polypeptide having a sequence as set forth in SEQ ID N0: 2, 4, 6, 8, 10, 12, 14, 16, 18, 20, 22, 24, 26, 28, 30, 32, 34, 36, 38, 40, 42, 44;
e) a polynucleotide hybridizing under stringent conditions to a polynucleotide having a sequence as set forth in SEQ ID N0: 1, 3, 5, 7, 9, 11, 13, 15, 17, 19, 21, 23, 25, 27, 29, 31, 33, 35, 37, 39, 41, 43; and
f) a polynucleotide hybridizing under stringent conditions to a polynucleotide encoding a polypeptide having at least 70% sequence identity to a polypeptide having a sequence as set forth in SEQ ID N0: 2, 4, 6, 8, 10, 12, 14, 16, 18, 20, 22, 24, 26, 28, 30, 32, 34, 36, 38, 40, 42, 44.

2. A composition comprising at least a dsRNA molecule according to claim 1.

3. A composition according to claim 2, **characterised in that** it further comprises an agriculturally acceptable support, carrier, filler and/or surfactant.

4. A composition according to claim 2 or 3 comprising further a phytopharmaceutical or plant growth promoting compound.

5. A micro-organism producing a dsRNA molecule according to claim 1.

6. A genetic construct which comprises at least one DNA sequence as well as heterologous regulatory element(s) in the 5' and optionally in the 3' positions, **characterized in that** the DNA sequence(s) encodes the dsRNA molecule according to claim 1.

7. A cloning and/or expression vector, **characterized in that** it contains at least one genetic construct according to claim 6.

8. A transgenic plant cell which comprises a dsRNA molecule according to claim 1 or a construct according to claim 6.

9. A transgenic plant, seed or part thereof, comprising a transgenic plant cell according to claim 8.

10. The transgenic plant cell according to claim 8 or transgenic plant, seed or part thereof, according to claim 9 wherein said plant is a soybean, oilseed, rice or potato plants.

11. A method of making a transgenic plant cell capable of expressing a dsRNA that inhibits a fungus or oomycete saccharopine dehydrogenase gene, said method comprises the steps of transforming a plant cell with a genetic construct according to claim 6.

12. A method of controlling a plant pathogen, particularly a fungus or oomycete, comprising providing to said pathogen a dsRNA molecule according to claim 1, or a composition according to anyone of claims 2 to 4.

13. A method according to claim 12, **characterized in that** an effective quantity of a dsRNA molecule according to claim 1 or a composition according to anyone of claims 2 to 4 is applied to the soil where plants grow or are capable of growing, to the leaves and/or the fruit of plants or to the seeds of plants.

14. A method according to claim 12, comprising providing in the host plant of said plant pathogen a transformed plant cell according to claim 8.

15. A method for inhibiting the expression of a plant pathogen gene, comprising the following steps:
i) transforming a plant cell with a genetic construct according to claim 6.
ii) placing the cells thus transformed under conditions that allow the transcription of said construct,
iii) bringing the cells into contact with the plant pathogen.

16. The method according to any one of claims 11 to 15 wherein said plant pathogen is *Magnaporthe grisea, Phytophthora infestans, Sclerotinia sclerotinium* or *Phakopsora pachyrhizi.*

17. The method according to any one of claims 11 to 15 wherein said plant is soybean, oilseed, rice or potato.

## Patentansprüche

1. dsRNA-Molekül, umfassend i) einen ersten Strang, umfassend eine Sequenz, die zu mindestens 18 aufeinanderfolgenden Nukleotiden eines Pilz- oder Oomyceten-Saccharopindehydrogenase-Gens identisch ist und ii) einen zweiten Strang, umfassend eine Sequenz, die zu dem ersten Strang im Wesentlichen komplementär ist, wobei das Pilz- oder Oomycetengen aus der Gruppe bestehend aus den Folgenden ausgewählt ist:
a) einem Polynukleotid, umfassend eine Sequenz gemäß SEQ ID NO: 1, 3, 5, 7, 9, 11, 13, 15, 17, 19, 21, 23, 25, 27, 29, 31, 33, 35, 37, 39, 41, 43;
b) einem Polynukleotid, das für ein Polypeptid mit einer Sequenz gemäß SEQ ID NO: 2, 4, 6, 8, 10, 12, 14, 16, 18, 20, 22, 24, 26, 28, 30, 32, 34, 36, 38, 40, 42, 44 codiert;
c) einem Polynukleotid mit mindestens 70%, vorzugsweise mindestens 80%, stärker bevorzugt mindestens 90%, noch stärker bevorzugt mindestens 95% Sequenzidentität zu einem Polynukleotid mit einer Sequenz gemäß SEQ ID NO: 1, 3, 5, 7, 9, 11, 13, 15, 17, 19, 21, 23, 25, 27, 29, 31, 33, 35, 37, 39, 41, 43;
d) einem Polynukleotid, das für ein Polypeptid mit mindestens 70%, vorzugsweise mindestens 80%, stärker bevorzugt mindestens 90%, noch stärker bevorzugt mindestens 95% Sequenzidentität zu einem Polypeptid mit einer Sequenz gemäß SEQ ID NO: 2, 4, 6, 8, 10, 12, 14, 16, 18, 20, 22, 24, 26, 28, 30, 32, 34, 36, 38, 40, 42, 44 codiert;
e) einem Polynukleotid, das unter strengenten Bedingungen mit einem Polynukleotid mit einer Sequenz gemäß SEQ ID NO: 1, 3, 5, 7, 9, 11, 13, 15, 17, 19, 21, 23, 25, 27, 29, 31, 33, 35, 37, 39, 41, 43 hybridisiert; und
f) einem Polynukleotid, das unter strengenten Bedingungen mit einem Polynukleotid hybridisiert, das für ein Polypeptid mit mindestens 70% Sequenzidentität zu einem Polypeptid mit einer Sequenz gemäß SEQ ID NO: 2, 4, 6, 8, 10, 12, 14, 16, 18, 20, 22, 24, 26, 28, 30, 32, 34, 36, 38, 40, 42, 44 codiert.

2. Zusammensetzung umfassend mindestens ein dsRNA-Molekül nach Anspruch 1.

3. Zusammensetzung nach Anspruch 2, **dadurch gekennzeichnet, dass** sie weiterhin einen landwirtschaftlich annehmbaren Träger, Carrier, Füllstoff und/oder ein landwirtschaftlich annehmbares Tensid umfasst.

4. Zusammensetzung nach Anspruch 2 oder 3, weiterhin umfassend ein Phytopharmazeutikum oder eine pflanzenwachstumsfördernde Verbindung.

5. Mikroorganismus, der ein dsRNA-Molekül nach Anspruch 1 produziert.

6. Genkonstrukt, das mindestens eine DNA-Sequenz sowie (ein) heterologe(s) Regulationselement(e) in der 5`-Position und gegebenenfalls in der 3`-Position umfasst, **dadurch gekennzeichnet, dass** die DNA-Sequenz(en) für das dsRNA-Molekül nach Anspruch 1 codiert/codieren.

7. Klonierungs- und/oder Expressionsvektor, **dadurch gekennzeichnet, dass** er mindestens ein Genkonstrukt nach Anspruch 6 enthält.

8. Transgene Pflanzenzelle, die ein dsRNA-Molekül nach Anspruch 1 oder ein Konstrukt nach Anspruch 6 umfasst.

9. Transgene Pflanze, transgener Samen oder Teil davon, umfassend eine transgene Pflanzenzelle nach Anspruch 8.

10. Transgene Pflanzenzelle nach Anspruch 8 oder transgene Pflanze, transgener Samen oder Teil davon nach Anspruch 9, wobei es sich bei der Pflanze um eine Sojabohnen-, Ölsaat-, Reis- oder Kartoffelpflanze handelt.

11. Verfahren zur Herstellung einer transgenen Pflanzenzelle, die fähig ist, eine dsRNA zu exprimieren, die ein Pilz- oder Oomyceten-Saccharopindehydrogenase-Gen hemmt, wobei das Verfahren die Schritte des Transformierens einer Pflanzenzelle mit einem Genkonstrukt nach Anspruch 6 umfasst.

12. Verfahren zum Bekämpfen eines Pflanzenpathogens, insbesondere eines Pilzes oder Oomyceten, bei dem man dem Pathogen ein dsRNA-Molekül nach Anspruch 1 oder eine Zusammensetzung nach einem der Ansprüche 2 bis 4 bereitstellt.

13. Verfahren nach Anspruch 12, **dadurch gekennzeichnet, dass** eine wirksame Menge eines dsRNA-Moleküls nach Anspruch 1 oder eine Zusammensetzung nach einem der Ansprüche 2 bis 4 auf dem Boden, wo die Pflanzen wachsen oder wachsen können, auf die Blätter und/oder die Früchte von Pflanzen oder auf die Samen von Pflanzen ausgebracht wird.

14. Verfahren nach Anspruch 12, bei dem man in der Wirtspflanze des Pflanzenpathogens eine transformierte Pflanzenzelle nach Anspruch 8 bereitstellt.

15. Verfahren zum Hemmen der Expression eines Pflanzenpathogen-Gens, umfassend die folgenden Schritte:
i) Transformieren einer Pflanzenzelle mit einem Genkonstrukt nach Anspruch 6,
ii) Platzieren der so transformierten Zellen unter Bedingungen, die die Transkription des Konstrukts gestatten,
iii) Inkontaktbringen der Zellen mit dem Pflanzenpathogen.

16. Verfahren nach einem der Ansprüche 11 bis 15, wobei es sich bei dem Pflanzenpathogen um *Magnaporthe grisea, Phytophthora infestans, Sclerotinia sklerotinium* oder *Phakopsora pachyrhizi* handelt.

17. Verfahren nach einem der Ansprüche 11 bis 15, wobei es sich bei der Pflanze um Sojabohne, Ölsaat, Reis oder Kartoffel handelt.

## Revendications

1. Molécule d'ARNdb comprenant : i) un premier brin comprenant une séquence identique à au moins 18 nucléotides contigus du gène d'une saccharopine déshydrogénase de champignon ou d'oomycète et ii) un second brin comprenant une séquence essentiellement complémentaire du premier brin, dans laquelle le gène de champignon ou d'oomycète est choisi dans le groupe constitué :
a) d'un polynucléotide comprenant une séquence telle que définie dans SEQ ID NO : 1, 3, 5, 7, 9, 11, 13, 15, 17, 19, 21, 23, 25, 27, 29, 31, 33, 35, 37, 39, 41, 43 ;
b) d'un polynucléotide codant pour un polypeptide présentant une séquence telle que définie dans SEQ ID NO : 2, 4, 6, 8, 10, 12, 14, 16, 18, 20, 22, 24, 26, 28, 30, 32, 34, 36, 38, 40, 42, 44 ;
c) d'un polynucléotide présentant au moins 70 % d'identité de séquence, de préférence au moins 80 %, de façon encore préférée au moins 90 % et, de façon encore davantage préférée, au moins 95 %, avec un polynucléotide présentant une séquence telle que définie dans SEQ ID NO : 1, 3, 5, 7, 9, 11, 13, 15, 17, 19, 21, 23, 25, 27, 29, 31, 33, 35, 37, 39, 41, 43 ;
d) d'un polynucléotide codant pour un polypeptide présentant au moins 70 % d'identité de séquence, de préférence au moins 80 %, de façon encore préférée au moins 90 % et, de façon encore davantage préférée, au moins 95 %, avec un polypeptide présentant une séquence telle que définie dans SEQ ID NO : 2, 4, 6, 8, 10, 12, 14, 16, 18, 20, 22, 24, 26, 28, 30, 32, 34, 36, 38, 40, 42, 44 ;
e) d'un polynucléotide s'hybridant dans des conditions stringentes avec un polynucléotide présentant une séquence telle que définie dans SEQ ID NO : 1, 3, 5, 7, 9, 11, 13, 15, 17, 19, 21, 23, 25, 27, 29, 31, 33, 35, 37, 39, 41, 43 ; et
f) d'un polynucléotide s'hybridant dans des conditions stringentes avec un polynucléotide codant pour un polypeptide présentant au moins 70 % d'identité de séquence avec un polypeptide présentant une séquence telle que définie dans SEQ ID NO : 2, 4, 6, 8, 10, 12, 14, 16, 18, 20, 22, 24, 26, 28, 30, 32, 34, 36, 38, 40, 42, 44.

2. Composition comprenant au moins une molécule d'ARNdb selon la revendication 1.

3. Composition selon la revendication 2, **caractérisée en ce qu'**elle comprend, en outre, un support, un vecteur, une charge et/ou un tensioactif de qualité agricole.

4. Composition selon la revendication 2 ou 3 comprenant, en outre, un produit phytopharmaceutique ou un composé favorisant la croissance des plantes.

5. Microorganisme produisant une molécule d'ARNdb selon la revendication 1.

6. Produit de recombinaison génétique comprenant au moins une séquence d'ADN, ainsi qu'un ou plusieurs éléments régulateurs hétérologues en position 5' et, éventuellement, 3', **caractérisé en ce que** cette ou ces séquences d'ADN codent pour la molécule d'ARNdb selon la revendication 1.

7. Vecteur de clonage et/ou d'expression, **caractérisé en ce qu'**il contient au moins un produit de recombinaison génétique selon la revendication 6.

8. Cellule de plante transgénique qui comprend une molécule d'ARNdb selon la revendication 1 ou un produit de recombinaison selon la revendication 6.

9. Plante, semence ou partie de plante transgénique, comprenant une cellule de plante transgénique selon la revendication 8.

10. Cellule de plante transgénique selon la revendication 8 ou plante, semence ou partie de plante transgénique selon la revendication 9, dans laquelle ladite plante correspond au soja, à une oléagineuse, au riz ou à la pomme de terre.

11. Procédé de production d'une cellule de plante transgénique capable d'exprimer un ARNdb qui inhibe le gène de la saccharopine déshydrogénase d'un champignon ou d'un oomycète, ledit procédé comprenant les étapes de transformation d'une cellule de plante à l'aide d'un produit de recombinaison génétique selon la revendication 6.

12. Procédé de lutte contre un organisme pathogène des plantes, en particulier un champignon ou un oomycète, comprenant la fourniture audit organisme pathogène d'une molécule d'ARNdb selon la revendication 1, ou d'une composition selon l'une quelconque des revendications 2 à 4.

13. Procédé selon la revendication 12, **caractérisé en ce qu'**une quantité efficace d'une molécule d'ARNdb selon la revendication 1 ou d'une composition selon l'une quelconque des revendications 2 à 4 est appliquée sur le sol où poussent ou peuvent pousser des plantes, sur les feuilles et/ou les fruits de plantes ou sur les semences de plantes.

14. Procédé selon la revendication 12, comprenant la fourniture, dans ladite plante hôte dudit organisme pathogène des plantes, d'une cellule de plante transformée selon la revendication 8.

15. Procédé d'inhibition de l'expression d'un gène d'un organisme pathogène des plantes, comprenant les étapes suivantes :
i) transformation d'une cellule végétale à l'aide d'un produit de recombinaison génétique selon la revendication 6 ;
ii) placement des cellules ainsi transformées dans des conditions permettant la transcription dudit produit de recombinaison ;
iii) mise en contact des cellules avec l'organisme pathogène des plantes.

16. Procédé selon l'une quelconque des revendications 11 à 15, dans lequel ledit organisme pathogène des plantes est *Magnaporthe grisea, Phytophthora infestans, Sclerotinia sclerotinium* ou *Phakopsora pachyrhizi.*

17. Procédé selon l'une quelconque des revendications 11 à 15, dans lequel ladite plante est le soja, une oléagineuse, le riz ou la pomme de terre.
